(19) Europäisches Patentamt
European Patent Office
Office européen des brevets

(11) EP 3 025 712 A1

(12) EUROPEAN PATENT APPLICATION

(43) Date of publication:
**01.06.2016 Bulletin 2016/22**

(21) Application number: **15189348.4**

(22) Date of filing: **02.08.2007**

(51) Int Cl.:
***A61K 31/4375*** (2006.01) ***A61K 31/513*** (2006.01)
***A61P 35/02*** (2006.01)

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **02.08.2006 US 835239 P**
**08.12.2006 US 873760 P**

(62) Document number(s) of the earlier application(s) in accordance with Art. 76 EPC:
**07811049.1 / 2 049 109**

(71) Applicant: **Sunesis Pharmaceuticals, Inc.**
**South San Francisco, CA 94080 (US)**

(72) Inventors:
- **ADELMAN, Daniel, C.**
  **Redwood City, CA California 94061 (US)**
- **SILVERMAN, Jeffrey, A.**
  **Burlingame, CA California 94010 (US)**
- **MICHELSON, Glenn**
  **Fairfax CA 94930 (US)**
- **SCATENA, Caroline Darne**
  **Alameda, CA California 94502 (US)**

(74) Representative: **Jones Day**
**Rechtsanwälte, Attorneys-at-Law, Patentanwälte**
**Prinzregentenstrasse 11**
**80538 München (DE)**

Remarks:
•Claims filed after the date of filing of the application / after the date of receipt of the divisional application (Rule 68(4) EPC)
•This application was filed on 12-10-2015 as a divisional application to the application mentioned under INID code 62.

(54) **COMBINED USE OF (+)-1,4-DIHYDRO-7-[(3S,4S)-3-METHOXY-4-(METHYLAMINO)-1-PYRROLIDINYL]-4-OXO-1-(2-THIAZOLYL)-1,8-NAPHTHYRIDINE-3-CARBOXYLIC ACID AND CYTARABINE (ARA-C) FOR THE TREATMENT OF LEUKEMIA**

(57) Methods of treating, preventing or managing hematologic disorders, such as leukemia are disclosed. The methods encompass the administration of SNS-595. Also provided are methods of treatment using this compound with chemotherapy, radiation therapy, hormonal therapy, biological therapy or immunotherapy. In certain embodiments, the method of treatment comprise administering SNS-595 in combination with Ara-C.

EP 3 025 712 A1

## Description

### 1. CROSS REFERENCE TO RELATED APPLICATIONS

This application claims priority to U.S. provisional application nos. 60/835,239, filed August 2,2006, and 60/873,760, filed December 8,2006. Both of these applications are incorporated in their entireties by reference.

### 2. FIELD OF THE INVENTION

Provided herein are methods of treating, preventing or managing hematologic disorders with enantiomerically pure (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid, which is also known as SNS-595 or AG-7352. In certain embodiments, the methods encompass treating, preventing or managing leukemias, including but not limited to, chronic lymphocytic leukemia, chronic myelocytic leukemia, acute lymphoblastic leukemia, acute myelogenous leukemia and acute myeloblastic leukemia, using (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid alone or, in particular, in combination with other therapeutic agents.

Further provided are combinations or "cocktails" of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid and other therapy, *e.g.*, radiation or other chemotherapeutics, including but not limited to, anti-cancer agents, immunosuppressive agents, and anti-inflammatories such as steroids for use in the methods of treating, preventing or managing hematologic disorders. It should be noted that the combinations or cocktails encompasses simultaneous as well as sequential administration.

In one embodiment, the combination therapy comprises administering a combination of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid and cytarabine (Ara-C). Also provided are pharmaceutical compositions and dosing regimens, in particular, for the combination thereof.

### 3. BACKGROUND OF THE INVENTION

Hematologic disorders affect the body's blood-forming and immune systems-the bone marrow and lymphatic tissues. They include hematologic malignancies such as leukemias, lymphomas (Non-Hodgkin's Lymphoma), Hodgkin's disease (also called Hodgkin's Lymphoma) and myeloma. New cases of leukemia, lymphoma, and myeloma account for 9 percent of cancer cases diagnosed in the United States, and about 59,200 persons are killed by the diseases each year.

Leukemia is classified by the rate at which it progresses. Acute leukemia is fast-growing and can overrun the body within weeks or months. By contrast, chronic leukemia is slow-growing and progressively worsens over a span of years.

#### Acute versus Chronic Leukemia

The blood-forming (hematopoietic) cells of acute leukemia remain in an immature state, so they reproduce and accumulate very rapidly. Therefore, acute leukemia needs to be treated immediately, otherwise the disease may be fatal within a few months. Fortunately, some subtypes of acute leukemia respond very well to available therapies and are curable. Children often develop acute forms of leukemia, which are managed differently from leukemia in adults.

In chronic leukemia, the blood-forming cells eventually mature, or differentiate, but are not "normal." They remain in the bloodstream much longer than normal white blood cells, and are unable to combat infection well.

#### Myelogenous versus Lymphocytic Leukemia

Leukemia may also be classified according to the type of white blood cell that is undergoing multiplication, *e.g.*, lymphocytes (immune system cells), granulocytes (bacteria-destroying cells) or monocytes (macrophage-forming cells). If the abnormal white blood cells are primarily granulocytes or monocytes, the leukemia is categorized as myelogenous, or myeloid, leukemia. If the abnormal blood cells are bone marrow lymphocytes, the cancer is called lymphocytic leukemia.

Other cancers, known as lymphomas, develop from lymphocytes within the lymph nodes, spleen, and other organs. Such cancers do not originate in the bone marrow and have a biological behavior different from lymphocytic leukemia.

Four types of leukemia are seen most frequently. They are:

Acute Myelogenous (granulocytic) Leukemia (AML)

Chronic Myelogenous (granulocytic) Leukemia (CML)
Acute Lymphocytic (lymphoblastic) Leukemia (ALL)
Chronic Lymphocytic Leukemia (CLL)

Acute myelogenous leukemia (AML), also known as acute nonlymphocytic leukemia (ANLL), is the most common form of adult leukemia. AML begins with abnormalities in the bone marrow blast cells that develop to form granulocytes, the white blood cells that contain small particles, or granules. AML blasts do not mature and quickly accumulate in the blood and bone marrow. As the cells build up, they hamper the body's ability to fight infection and prevent bleeding. AML, particularly in the monocytic M5 form, may spread to the gums and cause swelling, bleeding and pain. AML also may metastasize (spread) to the skin, causing small colored spots that mimic a rash.

Acute leukemia, such as AML, can be categorized into eight subtypes according to a system known as French-American-British (FAB) classification:

1. Undifferentiated AML (M0). In this form of leukemia, the bone marrow cells show no significant signs of differentiation (maturation to obtain distinguishing cell characteristics).
2. Myeloblastic leukemia (M1; with/without minimal cell maturation). The bone marrow cells show some signs of granulocytic differentiation.
3. Myeloblastic leukemia (M2; with cell maturation). The maturation of bone marrow cells is at or beyond the promyelocyte (early granulocyte) stage; varying amounts of maturing granulocytes may be seen. This subtype often is associated with a specific genetic change involving translocation of chromosomes 8 and 21.
4. Promyelocytic leukemia (M3 or M3 variant [M3V]). Most cells are abnormal early granulocytes that are between myeloblasts and myelocytes in their stage of development and contain many small particles. The cell nucleus may vary in size and shape. Bleeding and blood clotting problems, such as disseminated intravascular coagulation (DIC), are commonly seen with this form of leukemia. Good responses are observed after treatment with retinoids, which are drugs chemically related to vitamin A.
5. Myelomonocytic leukemia (M4 or M4 variant with eosinophilia [M4E]). The bone marrow and circulating blood have variable amounts of differentiated granulocytes and monocytes. The proportion of monocytes and promonocytes (early monocyte form) in the bone marrow is greater than 20% of all nucleated (nucleus-containing) cells. The M4E variant also contains a number of abnormal eosinophils (granular leukocyte with a two-lobed nucleus) in the bone marrow.
6. Monocytic leukemia (M5). There are two forms of this subtype. The first form is characterized by poorly differentiated monoblasts (immature monocytes) with lacy-appearing genetic material. The second, differentiated form is characterized by a large population of monoblasts, promonocytes, and monocytes. The proportion of monocytes in the bloodstream may be higher than that in the bone marrow. M5 leukemia may infiltrate the skin and gums, and has a worse prognosis than other subtypes.
7. Erythroleukemia (M6). This form of leukemia is characterized by abnormal red blood cell-forming cells, which make up over half of the nucleated cells in the bone marrow.
8. Megakaryoblastic leukemia (M7). The blast cells in this form of leukemia look like immature megakaryocytes (giant cells of the bone marrow) or lymphoblasts (lymphocyte-forming cells). M7 leukemia may be distinguished by extensive fibrous tissue deposits (fibrosis) in the bone marrow.

In addition, patients may develop isolated tumors of the myeloblasts (early granulocytes). An example of this is isolated granulocytic sarcoma, or chloroma - a malignant tumor of the connective tissue. Individuals with chloroma frequently develop AML, and thus are treated with an aggressive, AML-specific chemotherapy program.

Chronic myelogenous leukemia (CML) is known as a myeloproliferative disorder, *i.e.* a disease in which bone marrow cells proliferate outside of the bone marrow tissue. CML is easy to diagnose since it has a genetic marker that is readily identifiable under a microscope. About 95% of CML patients have a genetic translocation between chromosomes 9 and 22 in their leukemic cells. This abnormality, which is known as the Philadelphia chromosome (Ph1), causes uncontrolled reproduction and proliferation of all types of white blood cells and platelets (blood clotting factors).

CML tends to occur in middle- and retirement-aged people (the median age is 67 years). It occasionally affects people in their 20s, but it is rare in the very young; only 2% to 3% of childhood leukemias are CML. Early disease is often asymptomatic and discovered accidentally. Individuals with more advanced cases of CML may appear sickly and experience fevers, easy bruising, and bone pain. Laboratory and physical findings include enlarged spleen (splenomegaly), a high white blood cell count, and absent or low amounts of the white blood cell enzyme alkaline phosphatase.

CML is categorized according to the three phases of its development:

Chronic phase - Patients in this initial phase have fewer than 5% blast cells and promyelocytes (immature granulocytes) in their blood and bone marrow. This phase is marked by increasing overproduction of granulocytes.

Accelerated phase - Patients in this progressive phase have more than 5%, but fewer than 30% blast cells. Their leukemic cells exhibit more chromosomal abnormalities besides the Philadelphia chromosome, and so more abnormal cells are produced.
Blast phase (acute phase, blast crisis) - Patients in this final phase have more than 30% blast cells in their blood and bone marrow samples. The blast cells frequently invade other tissues and organs outside of the bone marrow. During this phase, the disease transforms into an aggressive, acute leukemia (70% acute myelogenous leukemia, 30% acute lymphocytic leukemia). If untreated, CML is fatal in roughly 20% of all patients each year.

Acute lymphocytic leukemia (ALL) - also known as acute lymphoblastic leukemia - is a malignant disease caused by the abnormal growth and development of early nongranular white blood cells, or lymphocytes. The leukemia originates in the blast cells of the bone marrow (B-cells), thymus (T-cells), and lymph nodes. ALL occurs predominantly in children, peaking at 4 years of age. ALL is seen more frequently in industrialized nations, is slightly more common among Caucasian children, and is more common in males than in females.

If ALL is T-cell in type, the thymus is involved. Leukemia-related enlargement of the thymus may lead to coughing, shortness of breath, or compression of the superior vena cava (SVC), the large vein that carries blood from the head and arms back to the heart. Such venous blockage may induce head and arm swelling and may cause a life-threatening condition known as SVC syndrome.

ALL can be categorized according to a system known as the French-American-British (FAB) Morphological Classification Scheme:

L1 - Mature-appearing lymphoblasts (T-cells or pre-B-cells). Cells are small with uniform genetic material, regular nuclear shape, nonvisible nucleoli (round bodies within the nucleus, the site of RNA synthesis), and little cytoplasm (substance of a cell, excluding the nucleus).
L2 - Immature and pleomorphic (variously shaped) lymphoblasts (T-cells or pre-B-cells). Cells are large and variable in size, with variable genetic material, irregular nuclear shape, one or more large nucleoli, and variable cytoplasm.
L3 - Lymphoblasts (B-cells; Burkitt's cells) are large and uniform; genetic material is finely stippled and uniform; nuclear shape is regular (oval to round); there are one or more prominent nucleoli; and cytoplasm is abundant.

Chronic lymphocytic leukemia is the most common leukemia in North America and in Europe. It is a disease of older adults and is very rare among people who are younger than 50 years of age. Men with CLL outnumber women by a 2-to-1 average.

CLL is thought to result from the gradual accumulation of mature, long-lived lymphocytes. Therefore, this cancer is caused not so much by overgrowth as it is by the extreme longevity and build-up of malignant cells. Although the rate of accumulation varies among individuals, the extensive tumor burden eventually causes complications in all CLL patients.

The incidence of hematological disorders, including leukemias, continues to climb as the general population ages, as new cancers develop, and as susceptible populations (e.g., people infected with AIDS or excessively exposed to sunlight) grow. In particular, chronic lymphocytic leukemia is an incurable leukemia with limited therapeutic options for patients with relapsed or refractory disease. A tremendous demand therefore exists for new methods, treatment regimens and pharmaceutical compositions that can be used to treat patients with hematological disorders including certain leukemias.

## 4. SUMMARY OF THE INVENTION

Provided herein are methods of treating, preventing or managing hematologic disorders, including, but not limited to leukemias, lymphomas (Non-Hodgkin's Lymphoma), Hodgkin's disease (also called Hodgkin's Lymphoma) and myeloma. In some embodiments, methods provided herein encompass methods of treating, preventing or managing various forms of leukemias such as chronic lymphocytic leukemia, chronic myeloid leukemia, acute lymphocytic leukemia, acute myelogenous leukemia and acute myeloblastic leukemia. The methods provided herein include treatment, prevention or management of leukemias that are relapsed, refractory or resistant. In certain embodiments, methods provided herein encompass methods of treating, preventing or managing promyelocytic leukemia.

The methods comprise administering to a subject in need of such treatment, prevention or management a therapeutically or prophylactically effective amount of (+)-1,4-dihydro-7-[(3S,4S)-3-methoxy-4-(methylamino)-1-pyrrolidinyl]-4-oxo-l-(2-thiazolyl)-1,8-naphthyridine-3-carboxylic acid. In some embodiments, (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid is used alone e.g., without other chemotherapeutics.

In another embodiment, (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid is administered in combination with a therapy e.g., another pharmaceutical agent with activity against cancer or its symptoms. Examples of therapies within the scope of the methods include, but

are not limited to, surgery, chemotherapy, radiation therapy, hormonal therapy, biological therapy, immunotherapy, and combinations thereof.

In a particular embodiment, the combination therapy comprises administering (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid and cytarabine (Ara-C). Also provided are dosing regimens, dosing schedules and methods of using SNS-595 in combination with Ara-C.

The methods provided include the administration of SNS-595 in combination with 5 to 1500 mg/$m^2$ of Ara-C. For example, one embodiment includes continuous daily administration of Ara-C at a dose of 200 to 400 mg/$m^2$. The administration of Ara-C can be made by intravenous infusion, intravenous push, bolus injection or subcutaneous injection. Significantly, the administration of Ara-C is daily, e.g., for 5 days, while the administration of SNS-595 occurs once or twice per week. As discussed herein, the administration of SNS-595 and Ara-C as set forth above in a week is considered a weekly cycle. The methods contemplate performing one weekly cycle, waiting a period of one week to several weeks where neither Ara-C nor SNS-595 is given then repeating a weekly cycle. The methods also contemplate repeating the weekly cycles continuously, for example, for 4 weeks or 28 days. In addition, the methods contemplate repeating the cycle for several cycles, waiting a period of a week to several weeks where neither Ara-C or SNS-595 is given then repeating one or more cycles. Finally, the methods provide administration of a SNS-595/Ara-C weekly cycle followed by a cycle of only Ara-C or SNS-595.

Also provided are methods where the daily Ara-C is administration is at a dose of 5-50 mg/$m^2$ and where the SNS-595 is administered once a week or twice a week. For example, the Ara-C may be administered daily for 10 days, and the SNS-595 may be administered on a schedule of once a week for three weeks, or twice a week for two weeks.

Also provided are pharmaceutical compositions, single unit dosage forms, and dosing regimens which comprise (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid, and a second, or additional, active agent. Second active agents include specific drugs or therapy, or combinations thereof, *i.e.* "cocktails."

## 5. BRIEF DESCRIPTION OF DRAWINGS

**FIG.1:** provides a comparison of anti-tumor activities of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid (SNS-595), etoposide, doxorubicin and irinotecan in human T-lymphoblastoid leukaemia cell lines (CCRF-CEM) xenograft model;

**FIG. 2:** provides a comparison of anti-tumor activities of (+)-1,4-dihydro-7-[(3S,4S)-3-methoxy-4-(methylamino)-l-pyrrolidinyl]-4-oxo-l-(2-thiazolyl)-1,8-naphthyridine-3-carboxylic acid (at 20 mg/kg and 25 mg/kg), etoposide, doxorubicin and irinotecan in a LM3-Jck xenograft model;

**FIG. 3:** shows cellularity in bone marrow 6 days post injection of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid. (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid was administered on day 0 and day 4. All images shown at 10x magnification;

**FIG. 4:** provides neutrophil response to increasing doses of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid;

**FIG. 5:** provides neutrophil count at various doses of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid at day 8;

**FIG. 6:** provides WBC count in response to various doses of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid at day 8;

**FIG. 7:** provides minor platelet count at various (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid doses by day 8;

**FIG. 8:** provides percent change in body weight at various time intervals after administering (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid;

**FIG. 9:** shows bone marrow rebound at day 12 after administering 20 mg/kg (+)-1,4-dihydro-7- [(3 S,4S)-3-methoxy-4-(methylamino)-1-pyrrolidinyl]-4-oxo-1-(2-thiazolyl)-1,8-naphthyridine-3-carboxylic acid;

**FIG. 10:** illustrates body weight changes in nu/nu mice after q4d x2 intravenous (IV) administration of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid ("q4d x2 IV" denotes a dosing cycle of one intravenous dose every four days, repeated one time);

**FIG. 11:** illustrates body weight changes in nu/nu mice after tid q4d x2 subcutaneous (SC) administration of Ara-C;

**FIG. 12:** illustrates body weight changes in nu/nu mice after combination administration of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid (q4d x2 IV) and Ara-C (tid q4d x2 IP, *i.e.* a dosing cycle of a subcutaneous dose of Ara-C administered thrice a day every four days, repeated twice);

**FIG. 13:** shows peripheral neutrophil levels in nu/nu mice after q4d x2 IV administration of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid;
**FIG. 14:** shows peripheral neutrophil levels in nu/nu mice after tid q4d x2 IP administration of Ara-C;
**FIG. 15:** shows peripheral neutrophil levels in nu/nu mice after combination administration of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid q4d x2 IV and Ara-C tid q4d x2 IP;
**FIG. 16:** shows peripheral neutrophil levels in nu/nu mice after combination administration of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid q4d x2 IV and Ara-C tid q4d x2 IP;
**FIG. 17:** shows peripheral white blood cell levels in nu/nu mice after q4d x2 IV administration of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid;
**FIG. 18:** shows peripheral white blood cell levels in nu/nu mice after tid q4d x2 IP administration of Ara-C;
**FIG. 19:** shows peripheral white blood cell levels in nu/nu mice after combination administration of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid q4d x2 IV and Ara-C. tid q4d x2 IP;
**FIG. 20:** shows peripheral white blood cell levels in nu/nu mice after combination administration of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid and Ara-C tid q4d x2 IP;
**FIG. 21:** shows peripheral platelet levels in nu/nu mice after q4d x2 IV administration of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid;
**FIG. 22:** shows peripheral platelet levels in nu/nu mice after q4d x2 IV administration of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid;
**FIG. 23:** shows peripheral platelet levels in nu/nu mice after combination administration of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid q4d x2 IV and Ara-C tid q4d x2 IP;
**FIG. 24:** shows peripheral platelets levels in nu/nu mice after combination administration of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid q4d x2 IV and Ara-C tid q4d x2 IP;
**FIG. 25:** shows cross sections of mouse femurs following various dosing regimens. Femurs were harvested on Day 6, two days post last dose; H&E, magnification 10X.

a) Animal #2 after co-administration of q4d x 2 IV (+)-1,4-dihydro-7-[(3S,4S)-3-methoxy-4-(methylamino)--pyrrolidinyl]-4-oxo-1-(2-thiazolyl)-1,8-naphthyridine-3-carboxylic acid vehicle and tid q4d x 2 Ara-C vehicle demonstrating 100% total cellularity,
b) Animal #23 after administration of 5 mg/kg q4d x 2 IV of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid demonstrating 60% total cellularity,
c) Animal #44 after administration of 10 mg/kg q4d x 2 IV of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid demonstrating 50% total cellularity,
d) Animal #63 after administration of 15 mg/kg q4d x 2 IV of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid demonstrating 20% total cellularity,
e) Animal #83 after administration of 20mg/kg tid q4d x 2 SC of Ara-C demonstrating 90% total cellularity,
f) Animal # 103 after administration of 40 mg/kg tid q4d x 2 SC of Ara-C demonstrating 90% total cellularity) Animal #122 after administration of 60 mg/kg tid q4d x 2 SC Ara-C demonstrating 50% total cellularity,
h) Animal #142 after co-administration of 5 mg/kg q4dx2 IV (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid and 40 mg/kg tid q4d x 2 SC Ara-C demonstrating 30% total cellularity and
i) Animal #163 after co-administration of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid 10 mg/kg q4d x 2 IV and Ara-C 20 mg/kg tid q4d x2 SC demonstrating 5% total cellularity;

**FIG. 26:** provides mouse femur cross sections at various dosing regimens. Femurs were harvested on Day 12, eight days post last dose. H&E, magnification 10X.

a) Animal #73 after administration of 15 mg/kg q4d x 2 IV of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid demonstrating 100% total cellularity,

b) Animal #134 after administration of 60 mg/kg tid q4d x 2 SC of Ara-C demonstrating 50% total cellularity,

c) Animal #154 after co-administration of 5 mg/kg q4d x 2 IV (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid and 40 mg/kg tid q4d x2 SC Ara-C demonstrating 100% total cellularity, and

d) Animal #174 after co-administration of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid 10 mg/kg q4dx2 IV and Ara-C 20 mg/kg tid q4d x 2 SC demonstrating 100% total cellularity; and

**FIG. 27** demonstrates a decrease in white blood cells (panel A), neutrophils (panel B) and platelets (panel C) following combination and single agent treatments.

**FIG. 28** illustrates the changes in bone marrow smears following treatment with SNS-595, Ara-C or SNS-595 in combination with Ara-C. Panel A demonstrates a numeric decrease in mature neutrophils two days after completion of the SNS-595/Ara-C combination treatment with recovery occurring six days later. Panel B demonstrates increase in immature neutrophils on Day 6 in the SNS-595 treated animals which returned to control levels by Day 12. Panel C demonstrates that two days after treatment blast counts in SNS-595/Ara-C treated animals were slightly elevated relative to the vehicle control.

**FIG. 29** demonstrates increase in neutrophils two weeks post treatment with SNS-595 and Ara-C.

**FIG. 30** illustrates body weight changes in nu/nu mice after q4d x2 intravenous (IV) administration of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid (SNS-595) alone (Grp 1); subcutaneous administration of Ara-c alone (Grp 2); or SNS-595 combined with Ara-C at the first, second or third daily subcutaneous administration of Ara-C (Grp 3, Grp 5 and Grp 4, respectively).

**FIG. 31** provides tolerability data, expressed as % body weight change for SNS-595 administered on day 0 or day 4 alone or in combination with Ara-C (cytarabine). The tolerability of Ara-c alone and the vehicle treated animals are also represented. Administration of Ara-C doses indicated with gray arrows, administration of SNS-595 dose indicated with black arrow.

**FIG. 32** shows the percent cellularity of the bone marrow in femurs following administration of SNS-595 (10, 15 or 20 mg/kg) on day 0 in combination with Ara-C (cytarabine, 20 mg/kg). The cellularity of Ara-C alone and vehicle-treated animals is also presented. Administration of Ara-C doses indicated with gray arrows, administration of SNS-595 dose indicated with black arrow.

**FIG. 33** shows the peripheral neutrophil counts following administration of SNS-595 (10, 15 or 20 mg/kg) on day 0 in combination with Ara-C (cytarabine, 20 mg/kg). The peripheral neutrophil counts of Ara-C alone and vehicle-treated animals is also presented. Administration of Ara-C doses indicated with gray arrows, administration of SNS-595 dose indicated with black arrow.

**FIG. 34** shows the percent cellularity of the bone marrow in femurs following administration of SNS-595 (10, 15 or 20 mg/kg) on day 4 (approximately 96 hours after the first injection) in combination with Ara-C (cytarabine, 20 mg/kg). The cellularity of Ara-C alone and vehicle-treated animals is also presented. Administration of Ara-C doses indicated with gray arrows, administration of SNS-595 dose indicated with black arrow.

**FIG. 35** shows the peripheral neutrophil counts following administration of SNS-595 (10, 15 or 20 mg/kg) on day 4 (approximately 96 hours after the first injection) in combination with Ara-C (cytarabine, 20 mg/kg). The peripheral neutrophil counts of Ara-C alone and vehicle-treated animals is also presented. Administration of Ara-C doses indicated with gray arrows, administration of SNS-595 dose indicated with black arrow.

## 6. DETAILED DESCRIPTION OF THE INVENTION

Provided herein are methods of treating, managing, or preventing hematologic disorders comprising administering to a mammal in need of such treatment, management or prevention a therapeutically or prophylactically effective amount of (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid alone, or in particular, in combination with another chemotherapeutic agent such as Ara-C. In one embodiment, the methods encompass treating, preventing or managing various forms of leukemias, including but not limited to, chronic lymphocytic leukemia, chronic myelocytic leukemia, acute lymphoblastic leukemia, acute myelogenous leukemia and acute myeloblastic leukemia. In one embodiment, the leukemia is acute lymphocytic leukemia (ALL). In one embodiment, the leukemia is acute myelogenous leukemia (AML). In one embodiment, the leukemia is chronic lymphocytic leukemia (CLL). In one embodiment, the leukemia is chronic myelogenous leukemia (CML). In one embodiment, the leukemia is refractory leukemia, relapsed leukemia or a leukemia that is resistant to other chemotherapeutic agents.

In other embodiments, (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid is administered in combination with another drug (*i.e.* a "second active agent") or another therapy for treating, managing, or preventing cancer. Second active agents include small molecules and large

molecules (e.g., proteins and antibodies), examples of which are provided herein, as well as stem cells or cord blood. Methods or therapies that can be used in combination with the administration of an (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid include, but are not limited to, surgery, blood transfusions, immunotherapy, biological therapy, radiation therapy, and other non-drug based therapies presently used to treat, prevent or manage cancer.

In one embodiment, the combination therapy comprises administering (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid and Ara-C. Specific doses and dosing regimens for this and other combinations is provided below.

Also provided are pharmaceutical compositions (e.g., single unit dosage forms) that can be used in methods disclosed herein. In one embodiment, pharmaceutical compositions comprise (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid and a second active agent.

## 6.1 DEFINITIONS

Unless defined otherwise, all technical and scientific terms used herein have the same meaning as is commonly understood by one of ordinary skill in the art. All patents, applications, published applications and other publications are incorporated by reference in their entirety. In the event that there are a plurality of definitions for a term herein, those in this section prevail unless stated otherwise.

As used herein, enantiomerically pure (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid is substantially free from (-)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid (*i.e.,* in enantiomeric excess). In other words, the "(+)" form of 1,4-dihydro-7-[(3S,4S)-3-methoxy-4-(methylamino)-1-pyrrolidinyl]-4-oxo-1-(2-thiazolyl)-1,8-naphthyridine-3-carboxylic acid is substantially free from the "(-)" form of the compound and is, thus, in enantiomeric excess of the "(-)" form. The term "enantiomerically pure" or "pure enantiomer" denotes that the compound comprises more than 75% by weight, more than 80% by weight, more than 85% by weight, more than 90% by weight, more than 91% by weight, more than 92% by weight, more than 93% by weight, more than 94% by weight, more than 95% by weight, more than 96% by weight, or more than 97% by weight of the enantiomer.

As used herein and unless otherwise indicated, the term "enantiomerically pure (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid" refers to at least about 80% by weight (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid and at most about 20% by weight (-)-1,4-dihydro-7-[(3 S,4S)-3-methoxy-4-(methylamino)-1-pyrrolidinyl]-4-oxo-1-(2-thiazolyl)-1,8-naphthyridine-3-carboxylic acid, at least about 90% by weight (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid and at most about 10% by weight the (-)-enantiomer, at least about 95% by weight (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid and at most about 5% by weight the (-)-enantiomer, at least about 97% by weight (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid and at most about 3% by weight (-)-enantiomer.

As used herein and unless otherwise indicated, the terms "treat," "treating" and "treatment" refer to alleviating or reducing the severity of a symptom associated with the disease or condition being treated.

The term "prevention" includes the inhibition of a symptom of the particular disease or disorder. In some embodiments, patients with familial history of cancer or leukemia are candidates for preventive regimens. Generally, the term "preventing" refers to administration of the drug prior to the onset of symptoms, particularly to patients at risk of cancer, and in particular leukemia.

As used herein and unless otherwise indicated, the term "managing" encompasses preventing the recurrence of the particular disease or disorder in a patient who had suffered from it, lengthening the time a patient who had suffered from the disease or disorder remains in remission, reducing mortality rates of the patients, and/or maintaining a reduction in severity or avoidance of a symptom associated with the disease or condition being managed.

As used herein, "subject" is an animal, typically a mammal, including a human, such as a human patient.

As used herein, "hematologic malignancy" refers to cancer of the body's blood-forming and immune system-the bone marrow and lymphatic tissue. Such cancers include leukemias, lymphomas (Non-Hodgkin's Lymphoma), Hodgkin's disease (also called Hodgkin's Lymphoma) and myeloma.

The term "leukemia" refers to malignant neoplasms of the blood-forming tissues. The leukemia includes, but is not limited to, chronic lymphocytic leukemia, chronic myelocytic leukemia, acute lymphoblastic leukemia, acute myelogenous leukemia, and acute myeloblastic leukemia. The leukemia can be relapsed, refractory or resistant to conventional therapy.

The term "relapsed" refers to a situation where patients who have had a remission of leukemia after therapy have a return of leukemia cells in the marrow and a decrease in normal blood cells.

The term "refractory or resistant" refers to a circumstance where patients, even after intensive treatment, have

residual leukemia cells in their marrow.

As used herein, "promyelocytic leukemia" or "acute promyelocytic leukemia" refers to a malignancy of the bone marrow in which there is a deficiency of mature blood cells in the myeloid line of cells and an excess of immature cells called promyelocytes. It is usually marked by an exchange of regions of chromosomes 15 and 17.

As used herein, "acute lymphocytic leukemia (ALL)", also known as "acute lymphoblastic leukemia" refers to a malignant disease caused by the abnormal growth and development of early nongranular white blood cells, or lymphocytes.

As used herein, "T- cell leukemia" refers to a disease in which certain cells of the lymphoid system called T lymphocytes or T cells are malignant. T cells are white blood cells that normally can attack virus-infected cells, foreign cells, and cancer cells and produce substances that regulate the immune response.

As used herein, the $IC_{50}$ refers to an amount, concentration or dosage of a particular test compound that achieves a 50% inhibition of a maximal response in an assay that measures such response.

As used herein and unless otherwise indicated, the term "pharmaceutically acceptable salt" includes, but is not limited to, salts of acidic or basic groups that can be present in the compounds provided herein. Under certain acidic conditions, the compound can form a wide variety of salts with various inorganic and organic acids. The acids that can be used to prepare pharmaceutically acceptable salts of such basic compounds are those that form salts comprising pharmacologically acceptable anions including, but not limited to, acetate, benzenesulfonate, benzoate, bicarbonate, bitartrate, bromide, calcium edetate, camsylate, carbonate, chloride, bromide, iodide, citrate, dihydrochloride, edetate, edisylate, estolate, esylate, fumarate, gluceptate, gluconate, glutamate, glycollylarsanilate, hexylresorcinate, hydrabamine, hydroxynaphthoate, isethionate, lactate, lactobionate, malate, maleate, mandelate, mesylate, methylsulfate, muscate, napsylate, nitrate, panthothenate, phosphate/diphosphate, polygalacturonate, salicylate, stearate, succinate, sulfate, tannate, tartrate, teoclate, triethiodide and pamoate. Under certain basic conditions, the compound can form base salts with various pharmacologically acceptable cations. Non-limiting examples of such salts include alkali metal or alkaline earth metal salts and, particularly, calcium, magnesium, sodium, lithium, zinc, potassium and iron salts.

As used herein and unless otherwise indicated, the term "hydrate" means a compound provided herein or a salt thereof, that further includes a stoichiometric or non-stoichiometeric amount of water bound by non-covalent intermolecular forces.

As used herein and unless otherwise indicated, the term "solvate" means a solvate formed from the association of one or more solvent molecules to a compound provided herein. The term "solvate" includes hydrates (e.g., monohydrate, dihydrate, trihydrate, tetrahydrate and the like).

As used herein, and unless otherwise specified, the terms "therapeutically effective amount" and "effective amount" of a compound refer to an amount sufficient to provide a therapeutic benefit in the treatment, prevention and/or management of a disease, to delay or minimize one or more symptoms associated with the disease or disorder to be treated. The terms "therapeutically effective amount" and "effective amount" can encompass an amount that improves overall therapy, reduces or avoids symptoms or causes of disease or disorder, or enhances the therapeutic efficacy of another therapeutic agent.

The terms "co-administration" and "in combination with" include the administration of two therapeutic agents (for example, SNS-595 and another anti-cancer agent) either simultaneously, concurrently or sequentially with no specific time limits. In one embodiment, both agents are present in the cell or in the patient's body at the same time or exert their biological or therapeutic effect at the same time. In one embodiment, the two therapeutic agents are in the same composition or unit dosage form. In another embodiment, the two therapeutic agents are in separate compositions or unit dosage forms.

The term "the supportive care agent" refers to any substance that treats, prevents or manages an adverse effect from SNS-595 treatment.

The term "biological therapy" refers to administration of biological therapeutics such as cord blood, stem cells, growth factors and the like.

The term "about," as used herein, unless otherwise indicated, refers to a value that is no more than 10% above or below the value being modified by the term. For example, the term "about 10 $mg/m^2$" means a range of from 9 $mg/m^2$ to 11 $mg/m^2$.

## 6.2 SNS-595

The compound for use in the methods, including the combination therapy, and compositions provided herein is enantiomerically pure (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid, which is also known as SNS-595 or AG-7352. SNS-595 has the following chemical structure:

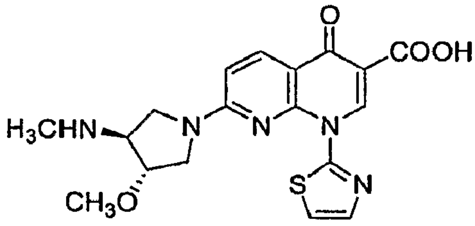

.

In certain embodiments, pharmaceutically acceptable salts, solvates, hydrates or prodrugs of SNS-595 are used in the methods and compositions provided herein.

SNS-595 can be prepared by methods known to one of skill in the art, for example, according to the preparation procedure for Example C-1 of U.S. Patent No. 5,817,669, entitled "Compounds, processes for the preparation thereof and anti-tumor agents," issued October 6, 1998, and in Japanese Patent Application No. Hei 10-173986, to Chikugi et al., which are incorporated herein by reference in their entireties. Certain exemplary pharmaceutical compositions comprising SNS-595 and methods of using the same are described in U.S. Patent Application Pub. Nos. 2005/0203120; 2005/0215583, 2006/0025437,2006/0063795 and 2006/0247267 which are incorporated herein by reference in their entireties.

## 6.3 SECOND ACTIVE AGENTS

In the methods and compositions provided herein, SNS-595 can be used with or combined with other pharmacologically active compounds ("second active agents"). Without being limited by any theory, it is believed that certain combinations work synergistically in the treatment of particular types of leukemias. The methods also encompass the use of SNS-595 in a manner to alleviate, reduce or avoid adverse effects associated with certain second active agents. Also provided are methods, wherein the second active agents are used in the manner to alleviate, reduce or avoid adverse or unwanted effects associated with SNS-595 including dose limiting toxicity.

One or more second active ingredients or agents can be used together with SNS-595 in the methods and compositions provided herein. Second active agents can be large molecules (*e.g.,* proteins) or small molecules (*e.g.,* synthetic inorganic, organometallic, or organic molecules).

Examples of large molecule active agents include, but are not limited to, hematopoietic growth factors, cytokines, and monoclonal and polyclonal antibodies, particularly, therapeutic antibodies to cancer antigens. Typical large molecule active agents are biological molecules, such as naturally occurring or synthetic or recombinant proteins. Proteins that are particularly useful in the methods and compositions provided herein include proteins that stimulate the survival and/or proliferation of hematopoietic precursor cells and immunologically active poietic cells *in vitro* or *in vivo.* Other useful proteins stimulate the division and differentiation of committed erythroid progenitors in cells *in vitro* or *in vivo.* Particular proteins include, but are not limited to: interleukins, such as IL-2 (including recombinant IL-II ("rIL2") and canarypox IL-2), IL-10, IL-12; and IL-18; interferons, such as interferon alfa-2a, interferon alfa-2b, interferon alfa-n1, interferon alfa-n3, interferon beta-I a, and interferon gamma-I b; GM-CF and GM-CSF; and EPO.

Particular proteins that can be used in the methods and compositions include, but are not limited to: filgrastim, which is sold in the United States under the trade name Neupogen® (Amgen, Thousand Oaks, CA); sargramostim, which is sold in the United States under the trade name Leukine® (Immunex, Seattle, WA); and recombinant EPO, which is sold in the United States under the trade name Epogen® (Amgen, Thousand Oaks, CA).

Recombinant and mutated forms of GM-CSF can be prepared as described in U.S. patent nos. 5,391,485; 5,393,870; and 5,229,496; all of which are incorporated herein by reference. Recombinant and mutated forms of G-CSF can be prepared as described in U.S. patent nos. 4,810,643; 4,999,291; 5,528,823; and 5,580,755; the entireties of which are incorporated herein by reference.

Also provided for use in combination with SNS-595 are native, naturally occurring, and recombinant proteins. Further encompassed are mutants and derivatives (*e.g.*, modified forms) of naturally occurring proteins that exhibit, *in vivo,* at least some of the pharmacological activity of the proteins upon which they are based. Examples of mutants include, but are not limited to, proteins that have one or more amino acid residues that differ from the corresponding residues in the naturally occurring forms of the proteins. Also encompassed by the term "mutants" are proteins that lack carbohydrate moieties normally present in their naturally occurring forms (*e.g.*, nonglycosylated forms). Examples of derivatives include, but are not limited to, pegylated derivatives and fusion proteins, such as proteins formed by fusing IgG1 or IgG3 to the protein or active portion of the protein of interest. *See, e.g.,* Penichet, M.L. and Morrison, S.L., J. Immunol. Methods 248:91-101 (2001).

Antibodies that can be used in combination with SNS-595 include monoclonal and polyclonal antibodies. Examples of antibodies include, but are not limited to, trastuzumab (Herceptin®), rituximab (Rituxan®), bevacizumab (Avastin™), pertuzumab (Omnitarg™), tositumomab (Bexxar®), edrecolomab (Panorex®), and G250. SNS-595 can also be combined with, or used in combination with, anti-TNF-$\alpha$ antibodies, and/or anti-EGFR antibodies, such as, for example, Erbitux® or panitumumab.

Large molecule active agents may be administered in the form of anti-cancer vaccines. For example, vaccines that secrete, or cause the secretion of, cytokines such as IL-2, G-CSF, and GM-CSF can be used in the methods and pharmaceutical compositions provided. *See, e.g.,* Emens, L.A., et al., Curr. Opinion Mol. Ther. 3(1):77-84 (2001).

Second active agents that are small molecules can also be used to alleviate adverse effects associated with the administration of SNS-595. However, like some large molecules, many are believed to be capable of providing a synergistic effect when administered with (*e.g.*, before, after or simultaneously) SNS-595. Examples of small molecule second active agents include, but are not limited to, anti-cancer agents, antibiotics, immunosuppressive agents, and steroids.

Examples of anti-cancer agents to be used within the methods or compositions described herein include, but are not limited to: acivicin; aclarubicin; acodazole hydrochloride; acronine; adozelesin; aldesleukin; altretamine; ambomycin; ametantrone acetate; amsacrine; anastrozole; anthramycin; asparaginase; asperlin; azacitidine; azetepa; azotomycin; batimastat; benzodepa; bicalutamide; bisantrene hydrochloride; bisnafide dimesylate; bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; celecoxib (COX-2 inhibitor); chlorambucil; cirolemycin; cisplatin; cladribine; crisnatol mesylate; cyclophosphamide; Ara-C; dacarbazine; dactinomycin; daunorubicin hydrochloride; decitabine; dexormaplatin; dezaguanine; dezaguanine mesylate; diaziquone; docetaxel; doxorubicin; doxorubicin hydrochloride; droloxifene; droloxifene citrate; dromostanolone propionate; duazomycin; edatrexate; eflomithine hydrochloride; elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; flurocitabine; fosquidone; fostriecin sodium; gemcitabine; gemcitabine hydrochloride; hydroxyurea; idarubicin hydrochloride; ifosfamide; ilmofosine; iproplatin; irinotecan; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine hydrochloride; megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxisuran; paclitaxel; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; taxotere; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; and zorubicin hydrochloride.

Other anti-cancer drugs to be included within the methods or comprising include, but are not limited to: 20-epi-1,25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorlns; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; Ara-C ocfosfate; cytolytic factor; cytostatin; dacliximab; decitabine; dehydrodidemnin B; deslorelin; dexamethasone; dexifosfamide; dexrazoxane; dexverapamil; diaziquone; didemnin B; didox; diethylnorspermine; dihydro-5-azacytidine; dihydrotaxol, 9-; dioxamycin; diphenyl spiromustine; docetaxel; docosanol; dolasetron; doxifluridine; doxorubicin; droloxifene; dronabinol; duocarmycin SA; ebselen; ecomustine; edelfosine; edrecolomab; eflornithine; elemene; emitefur; epirubicin; epristeride; estramustine analogue; estrogen agonists; estrogen antagonists; etanidazole; etoposide phosphate; exemestane; fadrozole;

fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; hypericin; ibandronic acid; idarubicin; idoxifene; idramantone; ilmofosine; ilomastat; imatinib (*e.g.*, Gleevec®); imiquimod; immunostimulant peptides; insulin-like growth factor-1 receptor inhibitor; interferon agonists; interferons; interleukins; iobenguane; iododoxorubicin; ipomeanol, 4-; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mitoguazone; mitolactol; mitomycin analogues; mitonafide; mitotoxin fibroblast growth factor-saporin; mitoxantrone; mofarotene; molgramostim;Erbitux, human chorionic gonadotrophin; monophosphoryl lipid A+myobacterium cell wall sk; mopidamol; mustard anticancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; nilutamide; nisamycin; nitric oxide modulators; nitroxide antioxidant; nitrullyn; oblimersen (Genasense®); $O^6$-benzylguanine; octreotide; okicenone; oligonucleotides; onapristone; ondansetron; ondansetron; oracin; oral cytokine inducer; ormaplatin; osaterone; oxaliplatin; oxaunomycin; paclitaxel; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rohitukine; romurtide; roquinimex; rubiginone B1; ruboxyl; safingol; saintopin; SarCNU; sarcophytol A; sargramostim; Sdi 1 mimetics; semustine; senescence derived inhibitor 1; sense oligonucleotides; signal transduction inhibitors; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stipiamide; stromelysin inhibitors; sulfinosine; superactive vasoactive intestinal peptide antagonist; suradista; suramin; swainsonine; tallimustine; tamoxifen methiodide; tauromustine; tazarotene; tecogalan sodium; tegafur; tellurapyrylium; telomerase inhibitors; temoporfin; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; velaresol; veramine; verdins; verteporfin; vinorelbine; vinxaltine; vitaxin; vorozole; zanoterone; zeniplatin; zilascorb; and zinostatin stimalamer.

Specific second active agents particularly useful in the methods or compositions include, but are not limited to, rituximab, oblimersen (Genasense®), remicade, docetaxel, celecoxib, melphalan, dexamethasone (Decadron®), steroids, gemcitabine, cisplatinum, temozolomide, etoposide, cyclophosphamide, temodar, carboplatin, procarbazine, gliadel, tamoxifen, topotecan, methotrexate, Arisa®, taxol, taxotere, fluorouracil, leucovorin, irinotecan, xeloda, CPT-11, interferon alpha, pegylated interferon alpha (e.g., PEG INTRON-A), capecitabine, cisplatin, thiotepa, fludarabine, carboplatin, liposomal daunorubicin, Ara-C, doxetaxol, pacilitaxel, vinblastine, IL-2, GM-CSF, dacarbazine, vinorelbine, zoledronic acid, palmitronate, biaxin, busulphan, prednisone, bisphosphonate, arsenic trioxide, vincristine, doxorubicin (Doxil®), paclitaxel, ganciclovir, adriamycin, estramustine sodium phosphate (Emcyt®), sulindac, and etoposide.

In certain embodiments, the first active agent is SNS-595 and the second active agent is etoposide, daunomycin, actinomycin D, mitomycin C, cisplatin, carboplatin, premetrexed, methotrexate, Ara-C (Ara-C), 5-Fu, wortmannin, geldanamycin, gemcitabin or a combination thereof.

In certain embodiments, the second active agent is an antileukemic nucleoside, such as Ara-C and/or decitabine and/or troxacytabine. In one embodiment, the nucleoside is Ara-C. Ara- C can be administered simultaneously or sequentially with SNS-595. In certain embodiments, SNS-595 and Ara-C are used in combination methods that may also include the use of one or more other therapies including, but not limited to, other anti-cancer agents, anti-emetics and the like.

In certain embodiments of the methods provided herein, use of a second active agent in combination with SNS-595 may be modified or delayed during or shortly following administration of SNS-595 as deemed appropriate by the

practitioner of skill in the art. In certain embodiments, subjects being administered SNS-595 alone or in combination with other therapies may receive supportive care including antiemetics, myeloid growth factors, and transfusions of platelets, when appropriate. In some embodiments, subjects being administered SNS-595 may be administered a growth factor as a second active agent according to the judgment of the practitioner of skill in the art. In some embodiments, provided is administration of SNS-595 in combination with erythropoietin or darbepoetin (Aranesp). Again, the method includes the use of these two agents with the addition of others such as Ara-C. In certain embodiments, administration of erythropoietin or darbepoetin is delayed duiring administration of SNS-595, Ara-C or both. In certain embodiments, erythropoietin or darbepoetin is administered during administration of SNS-595, for instance when the subject presents anemia or severe anemia. In some embodiments, administration of prophylactic granulocyte-macrophage colony-stimulating factor (GM-CSF); sargramostim (Leukine®), molgramostim, (Leukomax) or granulocyte colony-stimulating factor (G-CSF); filgrastim (Neupogen®), pegfilgrastim (Neulasta®) is delated during one or more administrations of SNS-595. In certain emodiments, provided are adminstrations of prophylactic granulocyte-macrophage colony-stimulating factor (GM-CSF); sargramostim (Leukine®), molgramostim, (Leukomax) or granulocyte colony-stimulating factor (G-CSF); filgrastim (Neupogen®), pegfilgrastim (Neulasta®) permitted after administration of SNS-595, for instance in a subject experiencing neutropenia or recurrent neutropenia. In certain embodiments, provided is administration of a myeloid growth factors in combination with SNS-595, for instance in a subject with a serious neutropenic complications, such as tissue infection, sepsis syndrome, or fungal infection, or at the discretion of the practitioner of skill.

In certain embodiments, provided is administration of SNS-595 in combination with one or more of the following: oral allopurinol, Rasburicase, Leukapheresis (for istance, administered up to 72 hours after the first treatment with SNS-595 Injection), and any other medication deemed appropriate by the practitioner of skill in the art.

### 6.4 METHODS OF TREATMENT AND PREVENTION

The methods provided herein encompass treating, preventing or managing various types of leukemias in a subject, such as chronic lymphocytic leukemia (CLL), chronic myelocytic leukemia (CML), acute lymphoblastic leukemia (ALL), acute myelogenous leukemia (AML), and acute myeloblastic leukemia (AML). In certain embodiments, the methods comprise the step of administering to the subject a therapeutically effective amount of an enantiomerically pure (+)-1,4-dihydro-7-[(3S,4S)-3-methoxy-4-(methylamino)-1-pyrrolidinyl]-4-oxo-1-(2-thiazolyi)-1,8-naphthyridine-3-carboxylic acid (SNS-595). In some embodiments, the methods comprise the step of administering to the subject a therapeutically effective amount of SNS-595 in combination with a therapeutically effective amount of a second active agent. In some embodiments, the second active agent is a therapeutic antibody to a cancer antigen, a hematopoietic growth factor, a cytokine, an anti-cancer agent, an antibiotic, a cox-2 inhibitor, an immunomodulatory agent, an immunosuppressive agent, a corticosteroid or a pharmacologically active mutant or derivative thereof. In other embodiments, the second active agent is an alkylating agent, an antineoplastic antibiotic, an anti-metabolite, a platinum coordination complex, a topoisomerase II inhibitor or radiation. In other embodiments, the second active agent is etoposide, daunomycin, actinomycin D, mitomycin C, cisplatin, carboplatin, premetrexed, methotrexate, Ara-C, 5-Fu, wortmannin, geldanamycin, gemcitabin or a combination thereof. In a particular embodiment, the second active agent is Ara-C.

In some embodiments, the methods provided herein encompass treating, preventing or managing acute leukemia in a subject. In some embodiments, the acute leukemia is acute myelogenous leukemia (AML), which includes, but is not limited to, undifferentiated AML (M0), myeloblastic leukemia (M1), myeloblastic leukemia (M2), promyelocytic leukemia (M3 or M3 variant [M3V]), myelomonocytic leukemia (M4 or M4 variant with eosinophilia [M4E]), monocytic leukemia (M5), erythroleukemia (M6), and megakaryoblastic leukemia (M7). In one embodiment, the acute myelogenous leukemia is undifferentiated AML (M0). In one embodiment, the acute myelogenous leukemia is myeloblastic leukemia (M1). In one embodiment, the acute myelogenous leukemia is myeloblastic leukemia (M2). In one embodiment, the acute myelogenous leukemia is promyelocytic leukemia (M3 or M3 variant [M3V]). In one embodiment, the acute myelogenous leukemia is myelomonocytic leukemia (M4 or M4 variant with eosinophilia [M4E]). In one embodiment, the acute myelogenous leukemia is monocytic leukemia (M5). In one embodiment, the acute myelogenous leukemia is erythroleukemia (M6). In one embodiment, the acute myelogenous leukemia is megakaryoblastic leukemia (M7). Thus, the methods of treating, preventing or managing acute myelogenous leukemia in a subject comprise the step of administering to the subject an amount of SNS-595 effective to treat, prevent or manage acute myelogenous leukemia alone or in combination. In some embodiments, the methods comprise the step of administering to the subject SNS-595 in combination with a second active agent in amounts effective to treat, prevent or manage acute myelogenous leukemia. In a particular embodiment, the second active agent is cytarabine (Ara-C).

In some embodiments, the methods provided herein encompass treating, preventing or managing acute lymphocytic leukemia (ALL) in a subject. In some embodiments, acute lymphocytic leukemia includes leukemia that originates in the blast cells of the bone marrow (B-cells), thymus (T-cells), and lymph nodes. The acute lymphocytic leukemia can be categorized according to the French-American-British (FAB) Morphological Classification Scheme as L1 - Mature-appearing lymphoblasts (T-cells or pre-B-cells), L2-Immature and pleomorphic (variously shaped) lymphoblasts (T-cells

or pre-B-cells), and L3 - Lymphoblasts (B-cells; Burkitt's cells). In one embodiment, the acute lymphocytic leukemia originates in the blast cells of the bone marrow (B-cells). In one embodiment, the acute lymphocytic leukemia originates in the thymus (T-cells). In one embodiment, the acute lymphocytic leukemia originates in the lymph nodes. In one embodiment, the acute lymphocytic leukemia is L1 type characterized by mature-appearing lymphoblasts (T-cells or pre-B-cells). In one embodiment, the acute lymphocytic leukemia is L2 type characterized by immature and pleomorphic (variously shaped) lymphoblasts (T-cells or pre-B-cells). In one embodiment, the acute lymphocytic leukemia is L3 type characterized by lymphoblasts (B-cells; Burkitt's cells). In certain embodiments, the acute lymphocytic leukemia is T-cell leukemia. In one embodiment, the T-cell leukemia is peripheral T-cell leukemia. In another embodiment, the T-cell leukemia is T-cell lymphoblastic leukemia. In another embodiment, the T-cell leukemia is cutaneous T-cell leukemia. In another embodiment, the T-cell leukemia is adult T-cell leukemia.Thus, the methods of treating, preventing or managing acute lymphocytic leukemia in a subject comprise the step of administering to the subject an amount of SNS-595 effective to treat, prevent or manage acute lymphocytic leukemia alone or in combination with a second active agent. In some embodiments, the methods comprise the step of administering to the subject SNS-595 in combination with a second active agent in amounts effective to treat, prevent or manage acute lymphocytic leukemia. In a particular embodiment, the second active agent is cytarabine (Ara-C).

In some embodiments, the methods provided herein encompass treating, preventing or managing chronic myelogenous leukemia (CML) in a subject. The methods comprise the step of administering to the subject an amount of SNS-595 effective to treat, prevent or manage chronic myelogenous leukemia. In some embodiments, the methods comprise the step of administering to the subject SNS-595 in combination with a second active agent in amounts effective to treat, prevent or manage chronic myelogenous leukemia. In a particular embodiment, the second active agent is cytarabine (Ara-C).

In some embodiments, the methods provided herein encompass treating, preventing or managing chronic lymphocytic leukemia (CLL) in a subject. The methods comprise the step of administering to the subject an amount of SNS-595 effective to treat, prevent or manage chronic lymphocytic leukemia. In some embodiments, the methods comprise the step of administering to the subject SNS-595 in combination with a second active agent in amounts effective to treat, prevent or manage chronic lymphocytic leukemia. In a particular embodiment, the second active agent is cytarabine (Ara-C).

### 6.4.1 SUBJECTS

In certain embodiments of the methods provided herein, the subject is an animal, preferably a mammal, more preferably a non-human primate. In particular embodiments, the subject is a human. The subject can be a male or female subject.

Particularly useful subjects for the methods provided herein include human cancer patients; for example, those who have been diagnosed with leukemia, including acute myelogenous leukemia, acute lymphocytic leukemia, chronic myelogenous leukemia, and chronic myelogenous leukemia. In certain embodiments, the subject has not been diagnosed with acute promyelocytic leukemia.

In some embodiments, the subject has a higher than normal blast population. In some embodiments, the subject has a blast population of at least 10%. In some embodiments, the subject has a blast population of between 10 and 15%. In some embodiments, the subject has a blast population of at least 15%. In some embodiments, the subject has a blast population of between 15 and 20%. In some embodiments, the subject has a blast population of at least 20%. In some embodiments, the subject has a blast population of about 10-15%, about 15-20%, or about 20-25%. In other embodiments, the subject has a blast population of less than 10%. In the context of the methods described herein, useful subjects having a blast population of less than 10% includes those subjects that, for any reason according to the judgment of the skilled practitioner in the art, are in need of treatment with SNS-595, alone or in combination with a second active agent.

In some embodiments, the subject is treated based on the Eastern Cooperative Oncology Group (ECOG) performance status score of the subject for leukemia. ECOG performance status can be scored on a scale of 0 to 5, with 0 denoting asymptomatic; 1 denoting symptomatic but completely ambulant; 2 denoting symptomatic and <50% in bed during the day; 3 denoting symptomatic and >50% in bed, but not bed bound; 4 denoting bed bound; and 5 denoting death. In some embodiments, the subject has an ECOG performance status score of 0 or 1. In some embodiments, the subject has an ECOG performance status score of 0. In some embodiments, the subject has an ECOG performance status score of 1. In other embodiments, the subject has an ECOG performance status score of 2.

In certain embodiments, the methods provided herein encompass the treatment of subjects who have not been previously treated for leukemia. In some embodiments, the subject has not undergone allogeneic bone marrow transplantation. In some embodiments, the subject has not undergone a stem cell transplantation. In some embodiments, the subject has not received hydroxyurea treatment. In some embodiments, the subject has not been treated with any investigational products for leukemia. In some embodiments, the subject has not been treated with systemic glucocorticoids.

In other embodiments, the methods encompass treating subjects who have been previously treated or are currently being treated for leukemia. For example, the subject may have been previously treated or are currently being treated with a standard treatment regimen for leukemia. The subject may have been treated with any standard leukemia treatment regimen known to the practitioner of skill in the art. In certain embodiments, the subject has been previously treated with at least one induction/reinduction or consolidation AML regimen. In some embodiments, the subject has undergone autologous bone marrow transplantation or stem cell transplantation as part of a consolidation regimen. In some embodiments, the bone marrow or stem cell transplantation occurred at least 3 months prior to treatment according to the methods provided herein. In some embodiments, the subject has undergone hydroxyurea treatment. In some embodiments, the hydroxyurea treatment occurred no later than 24 hours prior to treatment according to the methods provided herein. In some embodiments, the subject has undergone prior induction or consolidation therapy with cytarabine (Ara-C). In some embodiments, the subject has undergone treatment with systemic glucocorticosteroids. In some embodiments, the glucocorticosteroid treatment occurred no later 24 hours prior to treatment according to the methods described herein. In other embodiments, the methods encompass treating subjects who have been previously treated for cancer, but are non-responsive to standard therapies.

In some embodiments, the subject has not previously undergone treatment with SNS-595. In some embodiments, the subject has not previously undergone treatment with Ara-C. In some embodiments, the subject has not previously undergone treatment with SNS-595 in combination with a second active agent. In some embodiments, the subject has not previously undergone treatment with SNS-595 in combination with Ara-C. In other embodiments, the subject has previously undergone treatment with SNS-595. In some embodiments, the subject has previously undergone treatment with Ara-C. In some embodiments, the subject has previously undergone treatment with SNS-595 in combination with a second active agent. In some embodiments, the subject has previously undergone treatment with SNS-595 in combination with Ara-C.

Also encompassed are methods of treating subjects having relapsed or refractory leukemia. In some embodiments, the subject has been diagnosed with a relapsed or refractory AML subtype, as defined by the World Health Organization (WHO). Relapsed or refractory disease may be de novo AML or secondary AML, *e.g.*, therapy-related AML (t-AML).

In some embodiments, the methods provided herein are used to treat drug resistant leukemias, such as chronic myelogenous leukemia (CML). Thus, treatment with SNS-595 could provide an alternative for patients who do not respond to other methods of treatment. In some embodiments, such other methods of treatment encompass treatment with Gleevec® (imatinib mesylate). In some embodiments, provided herein are methods of treatment of Philadelphia chromosome positive chronic myelogenous leukemia (Ph+CML). In some embodiments, provided herein are methods of treatment of Gleevec® (imatinib mesylate) resistant Philadelphia chromosome positive chronic myelogenous leukemia (Ph+CML).

Also encompassed are methods of treating a subject regardless of the subject's age, although some diseases or disorders are more common in certain age groups. In some embodiments, the subject is at least 18 years old. In some embodiments, the subject is more than 18, 25, 35, 40, 45, 50, 55, 60, 65, or 70 years old. In other embodiments, the subject is less than 65 years old. In some embodiments, the subject is less than 18 years old. In some embodiments, the subject is less than 18, 15, 12, 10, 9, 8 or 7 years old.

In some embodiments, the methods may find use in subjects at least 50 years of age, although younger subjects could benefit from the method as well. In other embodiments, the subjects are at least 55, at least 60, at least 65, and at least 70 years of age. In another embodiment, the subjects have adverse cytogenetics. "Adverse cytogenetics" is defined as any nondiploid karyotype, or greater than or equal to 3 chromosomal abnormalities. In another embodiment, the subjects are at least 60 years of age and have adverse cytogenetics. In another embodiment, the subjects are 60-65 years of age and have adverse cytogenetics. In another embodiment, the subjects are 65-70 years of age and have adverse cytogenetics. In some embodiments, subjects in this paragraph are administered a low dose of ara-C as described herein.

In certain embodiments, the subject treated has no history of myocardial infarction within three months of treatment according to the methods provided herein. In some embodiments, the subject has no history of cerebrovascular accident or transient ischemic attack within three months of treatment according to the methods provided herein. In some embodiments, the subject has no suffered no thromboembelic event, including deep vein thrombosis or pulmonary embolus, within 28 days of treatment according to the methods provided herein. In other embodiments, the subject has not experienced or is not experiencing uncontrolled disseminated intravascular coagulation.

Because subjects with cancer have heterogeneous clinical manifestations and varying clinical outcomes, the treatment given to a patient may vary, depending on his/her prognosis. The skilled clinician will be able to readily determine without undue experimentation specific secondary agents, types of surgery, and types of non-drug based standard therapy that can be effectively used to treat an individual subject with cancer.

## 6.4.2 COMBINATION THERAPY WITH A SECOND ACTIVE AGENT

In certain embodiments, the methods provided herein comprise administering SNS-595 in combination with one or more second active agents, and/or in combination with radiation therapy, blood transfusions, or surgery. The administration of SNS-595 and the second active agents to a patient can occur simultaneously or sequentially by the same or different routes of administration. The suitability of a particular route of administration employed for a particular active agent will depend on the active agent itself (e.g., whether it can be administered orally without decomposing prior to entering the blood stream) and the disease being treated. Recommended routes of administration for the second active agents are known to those of ordinary skill in the art. *See, e.g.,* Physicians' Desk Reference, 1755-1760 (56th ed., 2002).

In one embodiment, the second active agent is administered intravenously or subcutaneously and once or twice daily in an amount of from about 1 to about 1,500 mg/m2, from about 5 to about 1,500 mg/m2, from about 1 to about 1,000 mg, from about 5 to about 500 mg, from about 10 to about 375 mg, or from about 50 to about 200 mg. In one embodiment, the second active agent is rituximab, oblimersen (Genasense®), GM-CSF, G-CSF, EPO, taxotere, irinotecan, dacarbazine, transretinoic acid, topotecan, pentoxifylline, ciprofloxacin, dexamethasone, vincristine, doxorubicin, COX-2 inhibitor, IL2, IL8, IL18, IFN, Ara-C, vinorelbine, or a combination thereof. In certain embodiments, the second active agent is etoposide, daunomycin, actinomycin D, mitomycin C, cisplatin, carboplatin, premetrexed, methotrexate, Ara-C, 5-Fu, wortmannin, geldanamycin, daunorubicin, or a combination thereof. In a particular embodiment, the second active agent is Ara-C.

The second active agent may be administered simultaneously, at essentially the same time, or sequentially with SNS-595. If administration takes place sequentially, second active agent may be administered before or after administration of SNS-595. In some embodiments, the second active agent is administered before administration of SNS-595. In some embodiments, the second active agent is administered simultaneously with administration of SNS-595. In some embodiments, the second active agent is administered after the administration of SNS-595. SNS-595 and the second active agent need not be administered by means of the same vehicle. In some embodiments, the second active agent and SNS-595 are administered in different vehicles. In embodiments of the methods described herein where delivery of SNS-595 and the second active agent are both by an intravenous route of administration, administration of each component of the combination need not be administered in the same IV line. In some embodiments, SNS-595 is administered in a different IV line than the second active agent. The second active agent may be administered one or more times, and the number of administrations of each component of the combination may be the same or different. In addition, SNS-595 and the second active agent need not be administered at the same site.

In other embodiments, provided herein are methods of treating, preventing and/or managing hematologic disorders, which comprise administering SNS-595 in conjunction with (*e.g.,* before, during, or after) conventional therapy including, but not limited to, surgery, immunotherapy, biological therapy, radiation therapy, or other non-drug based therapy presently used to treat, prevent or manage cancer. Without being limited by theory, it is believed that SNS-595 may provide additive or synergistic effects when given concurrently with other anti-cancer therapy.

In one embodiment, SNS-595 can be administered in an amount of from about 1 to about 150 $mg/m^2$, about 1 to about 120 $mg/m^2$, about 1 to about 100 $mg/m^2$, about 1 to about 75 $mg/m^2$, about 1 to about 60 $mg/m^2$, about 1 to about 50 $mg/m^2$, about 3 to about 30 $mg/m^2$, about 3 to about 24 $mg/m^2$ alone, or in combination with a second active agent disclosed herein (*see, e.g.,* section 5.3), prior to, during, or after the use of conventional therapy. In another specific embodiment, SNS-595 is administered at a dose of about 5 to about 50 $mg/m^2$ or about 10 to about 40 $mg/m^2$, or about 10 to about 90 $mg/m^2$.

In another embodiment, the methods provided herein comprise: a) administering to a patient in need thereof, a dose of about 1 $mg/m^2$-150 $mg/m^2$ of SNS-595 and b) administering a therapeutically effective amount of a supportive care agent. Such supportive care agents are known in the art, for example, *see,* U.S. Application Publication No. 2006/0025437, which is incorporated by reference in its entirety.

In certain embodiments, the combination dosing of SNS-595 and Ara-C is used together as well with supportive care agents or other auxillary therapies. While not intending to be bound by any particular theory of operation, it is believed that SNS-595 and Ara-C can act synergistically in the methods provided herein. Exemplary dosing schedules for the combination dosing of SNS-595 and Ara-C are provided below.

## 6.5 PHARMACEUTICAL COMPOSITIONS AND DOSAGE FORMS

The methods provided herein use pharmaceutical compositions containing SNS-595 and pharmaceutically acceptable carriers, such as diluents or adjuvants, or in combination with other active ingredient, such as another anti-cancer agent. In clinical practice SNS-595 may be administered by any conventional route, including but not limited to orally, parenterally, rectally or by inhalation (*e.g.* in the form of aerosols). In one embodiment, SNS-595 is administered by an IV injection.

The compositions for parenteral administration can be emulsions or sterile solutions. Use may be made, as solvent or vehicle, of propylene glycol, a polyethylene glycol, vegetable oils, in particular olive oil, or injectable organic esters, for example ethyl oleate. These compositions can also contain adjuvants, in particular wetting, isotonizing, emulsifying, dispersing and stabilizing agents. Sterilization can be carried out in several ways, for example using a bacteriological filter, by radiation or by heating. They can also be prepared in the form of sterile solid compositions which can be dissolved at the time of use in sterile water or any other injectable sterile medium.

The compositions can also be aerosols. For use in the form of liquid aerosols, the compositions can be stable sterile solutions or solid compositions dissolved at the time of use in apyrogenic sterile water, in saline or any other pharmaceutically acceptable vehicle. For use in the form of dry aerosols intended to be directly inhaled, the active principle is finely divided and combined with a water-soluble solid diluent or.vehicle, for example dextran, mannitol or lactose.

Pharmaceutical compositions can be used in the preparation of individual, single unit dosage forms. Pharmaceutical compositions and dosage forms comprise SNS-595 and one or more excipients.

Pharmaceutical compositions and dosage forms can also comprise one or more additional active ingredients. Examples of optional second, or additional, active ingredients are disclosed herein.

In certain embodiments, a composition provided herein is a pharmaceutical composition or a single unit dosage form. Pharmaceutical compositions and single unit dosage forms provided herein comprise a prophylactically or therapeutically effective amount of SNS-595, and typically one or more pharmaceutically acceptable carriers or excipients. The term "carrier" refers to a diluent, adjuvant (*e.g.*, Freund's adjuvant (complete and incomplete)), excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. In certain embodiments, water is a carrier when the pharmaceutical composition is administered intravenously. Saline solutions and aqueous dextrose and glycerol solutions can also be employed as liquid carriers, particularly for injectable solutions. Examples of suitable pharmaceutical carriers are described in Remington: The Science and Practice of Pharmacy, 21st edition, Lippincott, Williams and Wilkins, Baltimore, MD (2005), the contents of which are hereby incorporated by reference in their entirety.

Typical pharmaceutical compositions and dosage forms comprise one or more excipients. Suitable excipients are well-known to those skilled in the art of pharmacy, and non limiting examples of suitable excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. Whether a particular excipient is suitable for incorporation into a pharmaceutical composition or dosage form depends on a variety of factors well known in the art including, but not limited to, the way in which the dosage form will be administered to a subject and the specific active ingredients in the dosage form. The composition or single unit dosage form, if desired, can also contain minor amounts of wetting or emulsifying agents, or pH buffering agents.

Further provided herein are pharmaceutical compositions and dosage forms that comprise one or more compounds that reduce the rate by which an active ingredient will decompose. Such compounds, which are referred to herein as "stabilizers," include, but are not limited to, antioxidants such as ascorbic acid, pH buffers, or salt buffers.

The pharmaceutical compositions and single unit dosage forms can take the form of solutions, suspensions, emulsion, powders and the like. Such compositions and dosage forms will contain a prophylactically or therapeutically effective amount of a prophylactic or therapeutic agent, in certain embodiments, in purified form, together with a suitable amount of carrier so as to provide the form for proper administration to the subject. The formulation should suit the mode of administration. In one embodiment, the pharmaceutical compositions or single unit dosage forms are sterile and in suitable form for administration to a subject, such as a mammalian subject, such an animal subject, or in particular a human subject.

A pharmaceutical composition provided herein is formulated to be compatible with its intended route of administration. Examples of routes of administration include, but are not limited to, parenteral, *e.g.,* intravenous, intradermal, subcutaneous, intramuscular, subcutaneous, inhalation, intranasal, transdermal, topical, transmucosal, intra-tumoral, intra-synovial and rectal administration. In a specific embodiment, the composition is formulated in accordance with routine procedures as a pharmaceutical composition adapted for intravenous, subcutaneous, intramuscular, intranasal or topical administration to human beings. In one embodiment, a pharmaceutical composition is formulated in accordance with routine procedures for subcutaneous administration to human beings. Typically, compositions for intravenous administration are solutions in sterile isotonic aqueous buffer. Where necessary, the composition may also include a solubilizing agent and a local anesthetic such as lignocaine to ease pain at the site of the injection.

Examples of dosage forms include, but are not limited to: liquid dosage forms suitable for parenteral administration to a subject; and sterile solids (*e.g.*, crystalline or amorphous solids) that can be reconstituted to provide liquid dosage forms suitable for parenteral administration to a subject.

The composition, shape, and type of dosage forms provided herein will typically vary depending on their use. For example, a dosage form used in the initial treatment of disease may contain larger amounts of one or more of the

active ingredients it comprises than a dosage form used in the maintenance treatment of the same infection. Similarly, a parenteral dosage form may contain smaller amounts of one or more of the active ingredients it comprises than an oral dosage form used to treat the same disease or disorder. These and other ways in which specific dosage forms encompassed herein will vary from one another will be readily apparent to those skilled in the art. See, *e.g.,* Remington: The Science and Practice of Pharmacy, 21st edition, Lippincott, Williams and Wilkins, Baltimore, MD (2005), the contents of which are hereby incorporated by reference in their entirety.

Generally, the ingredients of compositions provided herein are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

Typical dosage forms provided herein comprise SNS-595 within the range of about 1 mg to about 150 mg per vial. Particular dosage forms provided herein have about 1, 3, 6, 9, 10, 12, 13.5, 15, 18, 19, 21, 24, 25, 27, 30, 38, 45, 50, 60, 63, 70, 75, 80, 85, 90, 95, 100, 105, 110, 115, 120, 125, 130, 135, 140, 145 or 150 mg of SNS-595 per vial.

### 6.5.1 PARENTERAL DOSAGE FORMS

Parenteral dosage forms can be administered to patients by various routes including, but not limited to, subcutaneous, intravenous (including bolus injection), intramuscular, and intraarterial. Because their administration typically bypasses patients' natural defenses against contaminants, parenteral dosage forms are preferably sterile or capable of being sterilized prior to administration to a patient. Examples of parenteral dosage forms include, but are not limited to, solutions ready for injection, dry products ready to be dissolved or suspended in a pharmaceutically acceptable vehicle for injection, suspensions ready for injection, and emulsions.

Suitable vehicles that can be used to provide parenteral dosage forms are well known to those skilled in the art. Examples include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water-miscible vehicles such as, but not limited to, ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate.

Compounds that increase the solubility of one or more of the active ingredients disclosed herein can also be incorporated into the parenteral dosage forms. For example, cyclodextrin and its derivatives can be used to increase the solubility of active ingredients. *See, e.g.,* U.S. Patent No. 5,134,127, which is incorporated herein by reference.

### 6.6 EXEMPLARY DOSAGES

In one embodiment, the methods of treating, preventing or managing cancers provided herein comprise administering to a patient SNS-595, alone or in combination with a second active agent, on the basis of body surface area. Body surface area calculations can be calculated for example, with the Mosteller formula wherein:

$$BSA(m^2)=\text{square root of } [(\text{height}(cm) \times \text{weight}(kg)/3600].$$

In one embodiment, SNS-595 can be administered orally or intravenously and in single or divided daily doses in an amount of about 1 to about 150 mg/m$^2$. Certain exemplary doses per day include about 1, 3, 6, 9, 10, 12, 13.5, 15, 18, 19, 21, 24, 25, 27, 30, 38, 45, 50, 60, 63, 75, 80, 85, 90, 95, 100, 105, 110, 115, 120, 125, 130, 135, 140, 145 or 150 mg/m$^2$.

In another embodiment, the methods of comprise administering a dose of about 3 mg/m$^2$- 120 mg/m$^2$ of SNS-595. In another embodiment, the dose is about 10 mg/m$^2$ -100 mg/m$^2$. In another embodiment, the dose is about 30 mg/m$^2$- 75 mg/m$^2$. In another embodiment, the dose is about 40 mg/m$^2$- 80 mg/m$^2$. In another embodiment, the dose is about 50 mg/m$^2$ - 90 mg/m$^2$. In another embodiment, the dose is about 15 mg/m$^2$ - 80 mg/m$^2$.

In another embodiment the dose of SNS-595 is about 20 mg/m$^2$ -30 mg/m$^2$. In another embodiment the dose is about 25 mg/m$^2$-35 mg/m$^2$. In another embodiment the dose is about 40 mg/m$^2$-50 mg/m$^2$. In another embodiment the dose is about 45 mg/m$^2$-55 mg/m$^2$. In another embodiment the dose is about 50 mg/m$^2$-60 mg/m$^2$. In another embodiment the dose is about 55 mg/m$^2$-65 mg/m$^2$. In another embodiment the dose is about 60 mg/m$^2$-70 mg/m$^2$. In another embodiment the dose is about 65 mg/m$^2$-75 mg/m$^2$. In another embodiment the dose is about 70 mg/m$^2$-80 mg/m$^2$. In another embodiment the dose is about 75 mg/m$^2$-85 mg/m$^2$. In another embodiment the dose is about 80

mg/$m^2$-90 mg/$m^2$. In another embodiment the dose is about 85 mg/$m^2$-95 mg/$m^2$. In another embodiment the dose is about 90 mg/$m^2$-100 mg/$m^2$. In another embodiment the dose is about 100 mg/$m^2$-110 mg/$m^2$. In another embodiment the dose is about 110 mg/$m^2$-120 mg/$m^2$. In another embodiment the dose is about 120 mg/$m^2$-130 mg/$m^2$. In another embodiment the dose is about 130 mg/$m^2$-140 mg/$m^2$. In another embodiment the dose is about 140 mg/$m^2$-150 mg/$m^2$.

In another embodiment, the dose of SNS-595 is about 1 mg/$m^2$ - 75 mg/$m^2$. In another embodiment, the dose is about 1 mg/$m^2$ - 60 mg/$m^2$. In another embodiment, the dose is about 1 mg/$m^2$ - 48 mg/$m^2$. In another embodiment, the dose is about 3 mg/$m^2$ - 24 mg/$m^2$. In another embodiment, the dose is about 3 mg/$m^2$ - 18 mg/$m^2$. In another embodiment, the dose is about 3 mg/$m^2$ - 15 mg/$m^2$.

In another embodiment, the dose is 1 mg/$m^2$, 2 mg/$m^2$, 3 mg/$m^2$, 4 mg/$m^2$, 5 mg/$m^2$, 6 mg/$m^2$, 7 mg/$m^2$, 8 mg/$m^2$, 9 mg/$m^2$, 10 mg/$m^2$, 11 mg/$m^2$, 12 mg/$m^2$, 13 mg/$m^2$, 14 mg/$m^2$, 15 mg/$m^2$, 16 mg/$m^2$, 17 mg/$m^2$, 18 mg/$m^2$, 19 mg/$m^2$, 20 mg/$m^2$, 21 mg/$m^2$, 22 mg/$m^2$, 23 mg/$m^2$, 24 mg/$m^2$, 25 mg/$m^2$, 26 mg/$m^2$, 27 mg/$m^2$ , 28 mg/$m^2$, 29 mg/$m^2$, 30 mg/$m^2$, 31 mg/$m^2$, 32 mg/$m^2$, 33 mg/$m^2$, 34 mg/$m^2$, 35 mg/$m^2$, 36 mg/$m^2$, 37 mg/$m^2$, 38 mg/$m^2$, 39 mg/$m^2$, 40 mg/$m^2$, 41 mg/$m^2$, 42 mg/$m^2$, 43 mg/$m^2$, 44 mg/$m^2$, 45 mg/$m^2$, 46 mg/$m^2$, 47 mg/$m^2$, 48 mg/$m^2$, 49 mg/$m^2$, 50 mg/$m^2$.

The administered dose of SNS-595 can be delivered as a single dose (*e.g.* a single bolus IV injection) or over a 24-hour period (*e.g.*, continuous infusion over time or divided bolus doses over time) and is repeated until the patient experiences stable disease or regression, or until the patient experiences disease progression or unacceptable toxicity. Stable disease or lack thereof is determined by methods known in the art, such as evaluation of patient symptoms, physical examination and other commonly accepted evaluation modalities.

The administered dose of SNS-595 can be expressed in units other than as mg/$m^2$. For example, doses can be expressed as mg/kg. One of ordinary skill in the art would readily know how to convert doses from mg/$m^2$ to mg/kg to given either the height or weight of a subject or both (*see, e.g,* www.fda.gov/cder/cancer/animalframe.htm). For example, a dose of 10 mg/$m^2$-150 mg/$m^2$ for a 65 kg human is approximately equal to 0.26 mg/kg-3.95 mg/kg. In another example, a dose of 15 mg/$m^2$-80 mg/$m^2$ for a 65 kg human is approximately equal to 0.39 mg/kg-2.11 mg/kg.

In certain embodiments, SNS-595 is cyclically administered to a patient. Cycling therapy involves the administration of an active agent for a period of time, followed by a rest for a period of time, and repeating this sequential administration. Cycling therapy can reduce the development of resistance to one or more of the therapies, avoid or reduce the side effects of one of the therapies, and/or improves the efficacy of the treatment.

In one embodiment, the methods provided herein comprise: i) administering a dose of about 10 mg/$m^2$-120 mg/$m^2$ of SNS-595 to a mammal; ii) waiting a period of at least one day where the mammal is not administered any SNS-595; iii) administering another dose of about 10 mg/$m^2$-120 mg/$m^2$ of SNS-595 to the mammal; and, iv) repeating steps ii)-iii) a plurality of times.

In one embodiment, the methods provided herein comprise: i) administering a dose of about 10 mg/$m^2$-90 mg/$m^2$ of SNS-595 to a mammal; ii) waiting a period of at least one day where the mammal is not administered any SNS-595; iii) administering another dose of about 10 mg/$m^2$-90 mg/$m^2$ of SNS-595 to the mammal; and, iv) repeating steps ii)-iii) a plurality of times.

In one embodiment, the methods provided herein comprise: i) administering a dose of about 10 mg/$m^2$-40 mg/$m^2$ of SNS-595 to a mammal; ii) waiting a period of at least one day where the mammal is not administered any SNS-595; iii) administering another dose of about 10 mg/$m^2$-40 mg/$m^2$ of SNS-595 to the mammal; and, iv) repeating steps ii)-iii) a plurality of times.

### 6.6.1 EXEMPLARY DOSAGES: COMBINATION DOSING OF SNS-595 AND ARA-C

In certain embodiments, the methods provided herein comprise administering SNS-595 in combination with one or more second active agents, wherein the second active agent is Ara-C. Ara- C can be administered either prior to, concurrently with, or subsequent to administration of SNS-595. In some embodiments, Ara-C can be administered subcutaneously or intravenously. In certain embodiments, Ara-C is administered subcutaneously. In certain embodiments, Ara-C is administered intravenously. In one embodiment, the dose of Ara-C is about 5 mg/$m^2$ to about 1500 mg/$m^2$, about 5 mg/$m^2$ to about 50 mg/$m^2$, about 25 mg/$m^2$ to 1000 mg/$m^2$, 50 mg/$m^2$ to 600 mg/$m^2$ and 200 to 400 mg/$m^2$. In another embodiment, the dose of Ara-C is about 100 mg/$m^2$ to 500 mg/$m^2$. In another embodiment, the dose of Ara-C is about 200 mg/$m^2$,300 mg/$m^2$ or 400 mg/$m^2$. In another embodiment, the dose of Ara-C is about 400 mg/$m^2$. Ara-C can be administered continuously, by bolus injection, or by divided bolus injections over a particular time period such as, for example, one day.

In another embodiment the dose of Ara-C is about 50 mg/$m^2$ -100 mg/$m^2$. In another embodiment the dose of Ara-C is about 100 mg/$m^2$ -150 mg/$m^2$. In another embodiment the dose of Ara-C is about 150 mg/$m^2$-200 mg/$m^2$. In another embodiment the dose of Ara-C is about 200 mg/$m^2$-250 mg/$m^2$. In another embodiment the dose of Ara-C is about 250 mg/$m^2$- 300 mg/$m^2$. In another embodiment the dose of Ara-C is about 350 mg/$m^2$-400 mg/$m^2$. In another embodiment the dose of Ara-C is about 400 mg/$m^2$-450 mg/$m^2$. In another embodiment the dose of Ara-C is about 450 mg/$m^2$-500 mg/$m^2$. In another embodiment the dose of Ara-C is about 500 mg/$m^2$- 550 mg/$m^2$. In another embodiment

the dose of Ara-C is about 550 mg/m$^2$-600 mg/m$^2$.

In some embodiments, treatment of leukemia in a subject in need thereof with a combination of SNS-595 and Ara-C comprises dosing the subject with about 1 mg/m$^2$-150 mg/m$^2$ of SNS-595 and about 100 mg/m$^2$-500 mg/m$^2$ of Ara-C. In certain embodiments, the subject is dosed with 10 mg/m$^2$ SNS-595 and 400 mg/m$^2$ of Ara-C; 18 mg/m$^2$ SNS-595 and 400 mg/m$^2$ of Ara-C; 30 mg/m$^2$ SNS-595 and 400 mg/m$^2$ of Ara-C; 45 mg/m$^2$ SNS-595 and 400 mg/m$^2$ of Ara-C; 63 mg/m$^2$ SNS-595 and 400 mg/m$^2$ of Ara-C; 70 mg/m$^2$ SNS-595 and 400 mg/m$^2$ of Ara-C; 80 mg/m$^2$ SNS-595 and 400 mg/m$^2$ of Ara-C; 90 mg/m$^2$ SNS-595 and 400 mg/m$^2$ of Ara-C; 100 mg/m$^2$ SNS-595 and 400 mg/m$^2$ of Ara-C; 110 mg/m$^2$ SNS-595 and 400 mg/m$^2$ of Ara-C; 120 mg/m$^2$ SNS-595 and 400 mg/m$^2$ of Ara-C; 130 mg/m$^2$ SNS-595 and 400 mg/m$^2$ of Ara-C; 140 mg/m$^2$ SNS-595 and 400 mg/m$^2$ of Ara-C; or 150 mg/m$^2$ SNS-595 and 400 mg/m$^2$ of Ara-C. In some embodiments, the subject is dosed with about 10 mg/m$^2$ of SNS-595 and about 400 mg/m$^2$ of Ara-C. In some embodiments, the subject is dosed with about 20 mg/m$^2$ of SNS-595 and about 400 mg/m$^2$ of Ara-C. In some embodiments, the subject is dosed with about 30 mg/m$^2$ of SNS-595 and about 400 mg/m$^2$ of Ara-C. In some embodiments, the subject is dosed with about 45 mg/m$^2$ of SNS-595 and about 400 mg/m$^2$ of Ara-C. In some embodiments, the subject is dosed with about 60 mg/m$^2$ of SNS-595 and about 400 mg/m$^2$ of Ara-C. In some embodiments, the subject is dosed with about 70 mg/m$^2$ of SNS-595 and about 400 mg/m$^2$ of Ara-C. In some embodiments, the subject is dosed with about 80 mg/m$^2$ of SNS-595 and about 400 mg/m$^2$ of Ara-C. In some embodiments, the subject is dosed with about 90 mg/m$^2$ of SNS-595 and about 400 mg/m$^2$ of Ara-C. In some embodiments, the subject is dosed with about 100 mg/m$^2$ of SNS-595 and about 400 mg/m$^2$ of Ara-C. In some embodiments, the subject is dosed with about 110 mg/m$^2$ of SNS-595 and about 400 mg/m$^2$ of Ara-C. In some embodiments, the subject is dosed with about 120 mg/m$^2$ of SNS-595 and about 400 mg/m$^2$ of Ara-C. In some embodiments, the subject is dosed with about 130 mg/m$^2$ of SNS-595 and about 400 mg/m$^2$ of Ara-C. In some embodiments, the subject is dosed with about 140 mg/m$^2$ of SNS-595 and about 400 mg/m$^2$ of Ara-C. In some embodiments, the subject is dosed with about 150 mg/m$^2$ of SNS-595 and about 400 mg/m$^2$ of Ara-C.

In certain embodiments, the subject is dosed with 10 mg/m$^2$ SNS-595 and 200 mg/m2 of Ara-C; 18 mg/m$^2$ SNS-595 and 200 mg/m2 of Ara-C; 30 mg/m$^2$ SNS-595 and 200 mg/m2 of Ara-C; 45 mg/m$^2$ SNS-595 and 200 mg/m2 of Ara-C; 63 mg/m$^2$ SNS-595 and 200 mg/m2 of Ara-C; 70 mg/m$^2$ SNS-595 and 200 mg/m2 of Ara-C; 80 mg/m$^2$ SNS-595 and 200 mg/m2 of Ara-C; 90 mg/m$^2$ SNS-595 and 200 mg/m2 of Ara-C; 100 mg/m$^2$ SNS-595 and 200 mg/m2 of Ara-C; ; 100 mg/m$^2$ SNS-595 and 200 mg/m2 of Ara-C; 110 mg/m$^2$ SNS-595 and 200 mg/m2 of Ara-C; 120 mg/m$^2$ SNS-595 and 200 mg/m2 of Ara-C; 130 mg/m$^2$ SNS-595 and 200 mg/m2 of Ara-C; 140 mg/m$^2$ SNS-595 and 200 mg/m2 of Ara-C; or 150 mg/m$^2$ SNS-595 and 200 mg/m2 of Ara-C. In some embodiments, the subject is dosed with about 10 mg/m$^2$ of SNS-595 and about 200 mg/m2 of Ara-C. In some embodiments, the subject is dosed with about 20 mg/m$^2$ of SNS-595 and about 200 mg/m2 of Ara-C. In some embodiments, the subject is dosed with about 30 mg/m$^2$ of SNS-595 and about 200 mg/m2 of Ara-C. In some embodiments, the subject is dosed with about 45 mg/m$^2$ of SNS-595 and about 200 mg/m2 of Ara-C. In some embodiments, the subject is dosed with about 60 mg/m$^2$ of SNS-595 and about 200 mg/m2 of Ara-C. In some embodiments, the subject is dosed with about 70 mg/m$^2$ of SNS-595 and about 200 mg/m2 of Ara-C. In some embodiments, the subject is dosed with about 80 mg/m$^2$ of SNS-595 and about 200 mg/m2 of Ara-C. In some embodiments, the subject is dosed with about 90 mg/m$^2$ of SNS-595 and about 200 mg/m2 of Ara-C. In some embodiments, the subject is dosed with about 100 mg/m$^2$ of SNS-595 and about 200 mg/m2 of Ara-C. In some embodiments, the subject is dosed with about 110 mg/m$^2$ of SNS-595 and about 200 mg/m2 of Ara-C. In some embodiments, the subject is dosed with about 120 mg/m$^2$ of SNS-595 and about 200 mg/m2 of Ara-C. In some embodiments, the subject is dosed with about 130 mg/m$^2$ of SNS-595 and about 200 mg/m2 of Ara-C. In some embodiments, the subject is dosed with about 140 mg/m$^2$ of SNS-595 and about 200 mg/m2 of Ara-C. In some embodiments, the subject is dosed with about 150 mg/m$^2$ of SNS-595 and about 200 mg/m2 of Ara-C.

In other embodiments, the exemplary combination dosages of SNS-595 and Ara-C provided herein comprise the total weekly dosage of SNS-595, and the total daily dosage of Ara-C, respectively. For example, where a subject treated by the methods provided herein with a combination dose of about 70 mg/m$^2$ of SNS-595 and about 400 mg/m$^2$ of Ara-C, the subject is treated a total weekly dose of 70 mg/m$^2$ SNS-595, and a daily dose of 400 mg/m$^2$ of Ara-C over the course of seven days.

In certain embodiments, the methods of treating, preventing or managing a hematologic disorder in a subject in need thereof comprises administering a total dosage of 10 mg/m$^2$ -120 mg/m$^2$ SNS-595 preferably 10 - 40 mg/m$^2$ in combination with a continuous intravenous dose of about 50 mg/m$^2$ /day - 600 mg/m$^2$/day Ara-C preferably 200 to 400 over a 5 day period, wherein the 5-day period comprises a treatment cycle. In some embodiments, the method comprises administering a total dosage of 10 mg/m$^2$ -40 mg/m$^2$ SNS-595 in combination with a continuous intravenous dose of about 200 to 400 mg/m$^2$/day Ara-C over a 5-day period, wherein the 5-day period comprises a treatment cycle. In some embodiments, the method comprises administering a total dosage of 20 mg/m$^2$ -40 mg/m$^2$ SNS-595 in combination with a continuous intravenous dose of about 200 to 400 mg/m$^2$/day Ara-C over a 5-day period, wherein the 5-day period comprises a treatment cycle. In some embodiments, the method comprises administering a total dosage of 10, 20, or 30 mg/m$^2$ or 40 mg/m$^2$ SNS-595 in combination with a continuous intravenous dose of about 200, 300 or 400 mg/m$^2$/day Ara-C over a 5-day period, wherein the 5-day period comprises a treatment cycle. In some embodiments, the method

comprises administering a total dosage of 40 mg/m$^2$ -80 mg/m$^2$ SNS-595 in combination with a continuous intravenous dose of about 400 mg/m$^2$/day Ara-C over a 5-day period, wherein the 5-day period comprises a treatment cycle. In some embodiments, the method comprises administering a total dosage of 70 mg/m$^2$ SNS-595 in combination with a continuous intravenous dose of about 400 mg/m$^2$/day Ara-C over a 5-day period, wherein the 5-day period comprises a treatment cycle. In some embodiments, the treatment cycle is repeated at least once. In some embodiments, the treatment cycle is repeated at least twice. In some embodiments, the treatment cycle is repeated at least three times. In some embodiments, the treatment cycle is repeated at least four times.

In certain embodiments, the methods of treating, preventing, or managing a hematological disorder in a subject in need thereof comprises administering a total weekly amount of 10-120 mg/m$^2$ SNS-595 in combination with a total daily amount of 10-50 mg/m$^2$ Ara-C.

In one embodiment, the doses of Ara-C used are from 5-25 mg/m$^2$. Such doses are referred to herein as low dose ara-C for use in certain leukemias and in certain patient populations. In another embodiment the doses of Ara-C used are from 5-25 mg/m$^2$ twice a day. In another embodiment, the doses of Ara-C used are from 5-25 mg/m$^2$ twice a day for 10 days. In another embodiment the Ara-C is administered subcutaneously (SC). In another embodiment, the dose is from 10 mg/m$^2$ -20 mg/m$^2$ Ara-C twice a day. In another embodiment the Ara-C dose is 10 mg/m2 SC twice a day for 10 days. In another embodiment, the Ara-C dose is 15 mg/m$^2$ SC twice a day for 10 days. In another embodiment, the Ara-C dose is 20 mg/m$^2$ SC twice a day for 10 days.

In another embodiment, the dose of Ara-C is 10-40 mg/m$^2$ once a day. In another embodiment, the dose of Ara-C is 10-40 mg/m$^2$ once a day for 10 days. In another embodiment the dose is administered subcutaneously. In another embodiment, the dose is from 15-30 mg/m$^2$ Ara-C. In another embodiment the Ara-C dose is 20 mg/m$^2$ SC once a day. In another embodiment, the Ara-C dose is 20 mg/m$^2$ SC once a day for 10 days.

SNS-595 schedules that can be used in combination with Ara-C at a total daily Ara-C dose of 10 -50 mg/m$^2$ (administered asa continuous infusion, single bolus, or divided boluses), include, for example, SNS-595 administered once a week for three weeks (Day 1, 8, and 15) and SNS-595 administered twice a week for two weeks (Days 1, 4, 8, and 11). In certain embodiments, doses of SNS-595 are about 10-90 mg/m$^2$ for the once a week for three weeks schedule and about 10-50 mg/m$^2$ for the twice a week for two weeks schedule. In one embodiment, the daily Ara-C doses are administeed for 10 days starting on the same day as (i.e. within 24 hours of) the initiation of the SNS-595 dose.

Duration (interval) between repeated administrations of the schedules can range from about 1 week to 8 weeks after the end of the schedule (e.g., after Day 15 or Day 11 respectively). In another embodiment, the interval is from 3 weeks to 6 weeks. In another embodiment, the interval is from 4 weeks to 6 weeks. In another embodiment, the interval is measured from Day 21 or Day 14, for the once a week for three weeks and twice a week for two week schedules, respectively.

### 6.6.2 EXEMPLARY DOSING SCHEDULES OF SNS-595 AND ARA-C

In the embodiments of the present invention, SNS-595 and Ara-C can be administered according to any schedule deemed suitable by a practitioner of skill in the art. Provided in this section are exemplary dosing schedules of SNS-595 in combination with Ara-C that can be practiced within the present invention.

In certain embodiments, SNS-595 and Ara-C are administered in cycles. In certain embodiments, SNS-595 and Ara-C are administered in at least one cycle. In certain embodiments, SNS-595 and Ara-C are administered in at least two cycles. In certain embodiments, SNS-595 and Ara-C are administered in at least three cycles. In certain embodiments, SNS-595 and Ara-C are administered in at least four cycles. In certain embodiments each cycle is at least 28 days.

In a cycle, SNS-595 and Ara-C are administered in combination. In certain embodiments, SNS-595 is administered in two doses three days apart, *i.e.* on days 1 and 4 of a cycle. In certain embodiments, Ara-C is administered by continuous intravenous infusion for five days. In certain embodiments, Ara-C is administered by continuous IV infusion on days 1 through 5 of a cycle. In certain embodiments, SNS-595 is administered in two doses three days apart, *i.e.* on days 1 and 4 of a cycle, and Ara-C is administered by continuous intravenous infusion for five days.

In certain embodiments, as discussed above, the initial dose of SNS-595 is administered before the administration of Ara-C. In certain embodiments, the initial dose of SNS-595 is administered immediately before the administration of Ara-C. In certain embodiments, administration of Ara-C is initiated 1, 2, 3, 4, 8, 12, 16, 24, or 32 hours following administration of SNS-595, for instance, 1, 2, 3, 4, 8, 12, 16, 24, or 32 hours following completion of the administration of SNS-595. In certain embodiments, administration of Ara-C is initiated about 8 hours following administration of SNS-595, for instance, 8 hours following administration of SNS-595 .

In certain embodiments, provided is an assessment of the subject within a cycle. For instance, in certain embodiments, the subject is assessed for safety and/or efficacy of the therapy by any of the techniques described above. In certain embodiments, the subject is assessed 12-16 days following the initial administration of SNS-595 in the cycle *(i.e.* the subject is assessed on days 13, 14, 15, 16 or 17 of the cycle). In certain embodiments, the subject is assessed

14 days following initial administration of SNS-595 in the cycle *(i.e.* on day 15 of the cycle).

In certain embodiments, the subject is administered a following cycle of therapy based on an evaluation of the assessment by a practitioner of skill in the art. For instance, in certain embodiments, after a first cycle of therapy (Cycle 1; "Induction" in the examples below), a subject can be administered a second cycle of therapy (Cycle 2; "Reinduction" in the examples below) if bone marrow blasts are reduced with greater than 5% of blasts are observed in the marrow. In certain embodiments, therapy can be discontinued after the first cycle (Cycle 1; Induction) if the subject presents progressive disease.

In certain embodiments, after the second cycle of therapy (Cycle 2; Reinduction), a subject can be administered a third cycle of therapy (Cycle 3; "Consolidation 1" in the examples below) if the subject presents morphologic complete remission ("CR"; > 1000 neutrophils per microliter and > 100,000 platelets per microliter of serum, and < 5% bone marrow blasts). In certain embodiments, after a second cycle of therapy (Cycle 2; Reinduction), a subject can be administered a third cycle of therapy (Cycle 3; Consolidation 1) if the subject presents morphologic complete remission without platelet recovery ("CRp"; > 1000 neutrophils per microliter, ≤ 100,000 platelets per microliter of serum, and < 5% bone marrow blasts). In certain embodiments, after a second cycle of therapy (Cycle 2; Reinduction), a subject can be administered a third cycle of therapy (Cycle 3; Consolidation 1) if the subject presents morphologic complete remission without incomplete blood count recovery ("CRi"; ≤ 1000 neutrophils per microliter, ≤ 100,000 platelets per microliter of serum, and < 5% bone marrow blasts). In certain embodiments, therapy can be discontinued after the first cycle (Cycle 1; Induction) if the subject presents progressive disease. In certain embodiments, therapy can be discontinued after the second cycle (Cycle 2; Reinduction) if the subject presents > 5% bone marrow blasts.

In certain embodiments, after the third cycle of therapy (Cycle 3; Consolidation 1), a subject can be administered a fourth cycle of therapy (Cycle 4; "Consolidation 2" in the examples below) if the subject presents peripheral blood CR (*i.e.* bone marrow need not be assessed). In certain embodiments, after a third cycle of therapy (Cycle 3; Consolidation 1), a subject can be administered a fourth cycle of therapy (Cycle 4; Consolidation 2) if the subject presents peripheral blood CRp (*i.e.* bone marrow need not be assessed). In certain embodiments, after a third cycle of therapy (Cycle 3; Consolidation 1), a subject can be administered a fourth cycle of therapy (Cycle 4; Consolidation 2) if the subject presents peripheral blood Cri *(i.e.* bone marrow need not be assessed). In certain embodiments, therapy can be discontinued after the third cycle (Cycle 3; Consolidation 1) if the subject presents progressive disease.

In certain embodiments, a subject can be administered Cycle 3 (Consolidation 1) following Cycle 1 (Induction). For instance, a subject can proceed from Cycle 1 to Cycle 3 if the subject presents CR, CRp or Cri following Cycle 1.

In certain embodiments, Cycle 2 (Reinduction) is initiated no more than 14 days following the assessment of Cycle 1 (Induction).

In certain embodiments, Cycle 3 (Consolidation 1) is initiated 27 days to 83 days following the initiation of treatment in the previous Cycle (*i.e*. on day 28 to day 84 of the previous Cycle). As discussed above, in certain embodiments, Cycle 3 (Consolidation 1) follows Cycle 1 (Induction). In certain embodiments, Cycle 3 (Consolidation 1) follows Cycle 2 (Reinduction).

In certain embodiments, Cycle 4 (Consolidation 2) is initiated at least 27 days following the initiation of treatment Cycle 3 (Consolidation 1), *i.e.* on day 28 of Cycle 3 (Consolidation 1).

In certain embodiments, the dose of SNS-595 is constant in each cycle of the therapy (Induction, Reinduction, Consolidation 1, Consolidation 2). In certain embodiments, the dose of Ara-C is constant in each cycle of the therapy (Induction, Reinduction, Consolidation 1, Consolidation 2). In certain embodiments, the dose of Ara-C is can be reduced from one Cycle to a second Cycle. For instance, in certain embodiments, in Cycle 1 (Induction) Ara-C can be administered at 400 mg/$m^2$ while in Cycles 2-4 (Reinduction, Consolidation 1, Consolidation 2) Ara-C can be administered at 200 mg/$m^2$. In certain embodiments, in Cycles 1-2 (Induction, Reinduction) Ara-C can be administered at 400 mg/$m^2$ while in Cycles 2-4 (Consolidation 1, Consolidation 2) Ara-C can be administered at 200 mg/$m^2$. In certain embodiments, in Cycles 1-3 (Induction, Reinduction, Consolidation 1) Ara-C can be administered at 400 mg/$m^2$ while in Cycle 4 (Consolidation 2) Ara-C can be administered at 200 mg/$m^2$. In certain embodiments, in Cycles 1-4 (Induction, Reinduction, Consolidation 1, Consolidation 2) Ara-C can be administered at 400 mg/$m^2$. Such dose reductions are administered according to the judgment of a practitioner of skill in the art, for instance, if a subject presents one or more dose limiting toxicities described herein.

In certain embodiments, therapy can continue beyond Cycle 4 according to the assessment described above. Patients can continue to be monitored for remission according to the assessment following therapy according to the judgment of the practitioner of skill.

## 7. EXAMPLES

Certain embodiments of the invention are illustrated by the following nonlimiting example.

### 7.1 EXAMPLE 1: PHARMACEUTICAL COMPOSITION SUITABLE FOR INJECTION OR INTRAVENOUS INFUSION

Acidic compositions (<pH 4) provided the appropriate balance of increased solubility of SNS-595 and desirable pharmaceutical properties (*e.g.* increased patient comfort by causing less irritation at the delivery site). An illustrative example of a suitable composition comprises: 10 mg SNS-595 per mL of aqueous solution of 4.5% sorbitol that is adjusted to pH 2.5 with methanesulfonic acid. One protocol for making such a solution includes the following for making a 100 mg/10 mL presentation: 100 mg of SNS-595 and 450 mg D-sorbitol are added to distilled water; the volume is brought up to a volume of 10 mL; and the pH of the resulting solution is adjusted to 2.5 with methanesulfonic acid. The resulting composition is also suitable for lyophilization. The lyophilized form is then reconstituted with sterile water to the appropriate concentration prior to use.

### 7.2 EXAMPLE 2: MTT CELL VIABILITY ASSAY:

The following cell lines were used in this assay: HL-60 (promyelocytic leukemia); Jurkat (T cell leukemia); CCRF-CEM (lymphoblastic leukemia); CEM/C2 (camptothecan resistant derivative of CCRF-CEM).

Cells were seeded in 96 wells plates at 3000 cells per well and incubated for 16 hours. Compound dilutions were performed in DMSO from 10mM with 3 fold dilutions. Titrations were diluted 1:100 in media to achieve final compound concentrations. The 96 well plates were aspirated and compound dilutions in media were added (100ml/well). MTT analysis was carried out after 72 hours of incubation at 37° C. Briefly, 20ml of MTT solution was added to each well. Cells were incubated at 37° C for 1-2 hours. Cells were lysed with the addition of 100 ml/well cell lysis buffer and MTT was solubilized overnight at 37° C. Plates were read on a spectromax machine with an absorbance measurement at 570nM. $IC_{50}$'s were calculated (data provided in Table 1) using regression analysis within GraphPad Prism. As provided in Table 1, SNS-595 shows potent anti-proliferative activity against hematologic cell lines tested.

**Table 1:** $IC_{50}$ data for various cell lines

| | $IC_{50}$ ng/mL | | | |
|---|---|---|---|---|
| **Cell Line** | **SNS-595** | **Etoposide** | **Doxorubicin** | **Irinotecan** |
| HL-60 | 53 | 136 | 24 | 905 |
| Jurkat | 23 | nd | nd | Nd |
| CCRF-CEM | 18 | nd | 3 | 479 |
| CEM/C2 | 10 | nd | 17 | 44400 |

### 7.3 EXAMPLE 3: XENOGRAFT MODELS:

LM3-Jck human malignant lymphoma tumor lobes (2-3 mm square) were transplanted subcutaneously into nude mice. Tumors were allowed to grow to approximately 7-14 mm in diameter. Mice were pair-matched into no treatment, irinotecan (100 mg/kg, IV, q4d x 3), doxorubicin (12 mg/kg, IV, Single shot), etoposide (12 mg/kg, IV, qld x5), and SNS-595 (25 and 20 mg/kg, IV, q7d x 5) treatment groups. Acceptable toxicity was defined as a mean group weight loss of 30% or less and not more than one toxic death among 6 treated animals. Anti-tumor activities of the drugs were assessed 21 days after the start of administration.

CCRF-CEM acute lymphoblastic leukemia tumor lobes of 2-3 mm square were transplanted subcutaneously into nude mice. Tumors were allowed to grow to approximately 8-20 mm in diameter. Mice were pair-matched into no treatment, irinotecan (100 mg/kg, IV, q4d x 3), doxorubicin (12 mg/kg, IV, q7d x 3), etoposide (12 mg/kg, IV, qldx5), and SNS-595 (25 and 20 mg/kg, IV, q7d x 5) treatment groups. Acceptable toxicity was defined as a mean group weight loss of 30% or less and not more than one toxic death among 6 treated animals. Anti-tumor activities of the drugs were assessed 20 or 21 days after the start of administration. **FIGs. 1-2** and Table 2 provides data for tumor inhibition rate (IR) and survival ratio in the CCRF-CEM and LM3-Jck xenograft models.

**Table 2:** Comparative anti-tumor activity of SNS-595 and other anti-cancer drugs

| | | **CCRF-CEM** | | **LM3 - JcK** | |
|---|---|---|---|---|---|
| **Treatment** | **Dose (mg/kg)** | **IR (%)** | **Survival Ratio** | **IR (%)** | **Survival Ratio** |
| SNS-595 q7d X3,IV | 20 | -* | - | 98.9* | 6/6 |
| | 25 | 98.1* | 6/6* | 98.5* | 6/6 |

(continued)

| | | CCRF-CEM | | LM3 - JcK | |
|---|---|---|---|---|---|
| Treatment | Dose (mg/kg) | IR (%) | Survival Ratio | IR (%) | Survival Ratio |
| Irinotecan q4d x3, IV | 100 | 99.7* | 5/6 | 97.7* | 6/6 |
| Doxorubicin q7d X3, IV | 12 | 50.3* | 6/6 | 57.2* | 6/6 |
| Etoposide qd X 5, IV | 12 | 28.3 | 6/6 | 3.0 | 6/6 |

As summarized in Table 2 and demonstrated in **FIG. 2,** SNS-595 administered at 20 and 25 mg/kg shows strong antitumor activity with complete tumor regressions (CR) against LM-3 Jck malignant lymphoma. Tumor inhibition rate (IR) of SNS-595 was similar to that of irinotecan and superior to etoposide and doxorubicin in both the CCRF-CEM and LM3-Jck xenograft models.

### 7.4 EXAMPLE 4: BONE MARROW / CYTOLOGY ASSAY

Female CD-1 mice were administered 5, 10, 15, or 20 mg/kg SNS-595 intravenously on Day 0 and Day 4. Blood was drawn on days 6, 8, and 12 post initial injection for hematological analysis. Femurs were extracted on day 6 fixed in Streck and H&E stained prior to bone marrow cellularity analysis. Two days after the second administration of SNS-595, bone marrow isolated from femurs showed a dose-dependent reduction in cellularity. At 20 mg/kg, cellularity was reduced to 7.5%, while circulating neutrophils were reduced from a pre-dose level of 1244 ±55 cells/mL to a nadir of 51 ±24 cells/mL blood on day 8. Absolute neutrophil counts subsequently rebounded and soon returned to normal levels. Total WBCs also reached a nadir on day 8, but returned to normal levels. Dose dependent decrease in the hematopoietic bone marrow cellularity is shown in **FIG. 3.** The FIG. shows cellularity in bone marrow 6 days post initial injection of SNS-595 at various doses. **FIG. 9** shows bone marrow rebound at 12 days post initial injection of 20 mg/kg SNS-595.

**FIG. 4** shows neutrophil reponse from blood samples on days 0, 6, 8 and 12 post initial injection of SNS-595. **FIGs. 5** and **6** show neutrophil and total white blood cell counts from blood samples on day 8 post initial injection. All SNS-595 dose groups demonstrated a significant decrease in peripheral neutrophils by day 8. White blood cell count was significantly reduced at the 10, 15 and 20 mg/kg doses of SNS-595. Animals receiving 20 mg/kg injections of SNS-595 had less than 50 cells/ml on day 8. Meanwhile, a minor platelet response was observed at 8 days post initial injection of various doses of SNS-595, as shown in **FIG. 7. FIG. 8** shows body weight loss at 5, 7, 9, 13 and 16 days post initial injection of SNS-595 at 0, 5, 10, 15 and 20 mg/kg doses. Body weight loss was tolerable across all dose groups.

### 7.5 EXAMPLE 5: EFFECT OF COMBINATION DOSING OF SNS-595 AND CYTARABINE (ARA-C) ON THE CYTOLOGY OF THE BONE MARROW

The effect of combination dosing of cytarabine (Ara-C) and SNS-595 was studied in 10 groups of nu/nu mice. The study comprised administration of SNS-595 in two intravenous (IV) doses once every four days (q4d x2) and six subcutaneous (SC) doses of Cytarabine (Ara-C) thrice every four days (tid q4d x2). The effects of dosing were studied on changes in body weight, peripheral cytology, and femoral bone marrow cellularity over time as described below.

Ara-C used in this study was obtained from Henry Schein, Inc.; D-Sorbitol and Methanesulfonic acid from Sigma Aldrich and CD-1 female mice from Charles River Laboratories.

SNS-595 was formulated in 0.17% methanesulfonic acid in 5% sorbitol and Ara-C was formulated in sterile water. Table 3 summarizes the dosing concentrations and volumes for dosing formulations.

**Table 3: Formulations:**

| Group | Compound | Dose (mg/kg) | Schedule | Dosing Solution ConC (mg/mL) | Dosing Volume (mL/kg) | Volume Stock (mL) * | Volume Diluent (mL) |
|---|---|---|---|---|---|---|---|
| 2, 8 | SNS-595 | 5 | Q4d x 2 | 1 | 5 | 0.7 | 6.3 |
| 3, 9, 10 | SNS-595 | 10 | Q4d x 2 | 2 | 5 | 2.0 | 8.0 |
| 4 | SNS-595 | 15 | Q4d x 2 | 3 | 5 | 1.2 | 2.8 |
| 5,6,8,9,10 | Ara-C | 20,40 | Tid q4d x 2 | 4 | 5, 10 | 4.8 | 19.2 |

(continued)

| Group | Compound | Dose (mg/kg) | Schedule | Dosing Solution ConC (mg/mL) | Dosing Volume (mL/kg) | Volume Stock (mL) * | Volume Diluent (mL) |
|---|---|---|---|---|---|---|---|
| 7 | Ara-C | 60 | Tid q4d x 2 | 6 | 5 | 1.8 | 4.2 |
| *: SNS-595 stock: 10 mg/mL, Ara-C stock: 20 mg/mL | | | | | | | |

Before beginning the study, all animals were allowed to acclimatize for 3 days from shipping related stress and the health of the mice was assessed daily by observation. Purified water (reverse osmosis) and irradiated food (PicoLab Rodent Diet 20, #5053; Dean's Animal Feeds, San Carlos, CA) were provided ad libitum, and the animals were kept on a 12 hour light and dark cycle. All animals were weighed, randomized by body weight, and assigned to the study groups shown in Table 4 before initial dosing.

**Table 4: Group designations, doses and schedules**

| Gp | N | Compound | Dose (mg/kg) | Dose Route | Schedule | Dosing volume (ml/kg) | Compound | Dose (mg/kg) | Dose Route | Schedule | Dosing volume (ml/kg) |
|---|---|---|---|---|---|---|---|---|---|---|---|
| 1 | 20 | Vehicle | 0 | IV | q4d x 2 | 5 | Vehicle | 0 | SC | tid q4d x 2 | 10 |
| 2 | 20 | SNS-595 | 5 | IV | q4d x 2 | 5 | - | - | - | - | - |
| 3 | 20 | SNS-595 | 10 | IV | q4d x 2 | 5 | - | - | - | - | - |
| 4 | 20 | SNS-595 | 15 | IV | q4d x 2 | 5 | - | - | - | - | - |
| 5 | 20 | - | - | - | - | - | Ara-C | 20 | SC | tid q4d x 2 | 5 |
| 6 | 20 | - | - | - | - | - | Ara-C | 40 | SC | tid q4d x 2 | 10 |
| 7 | 20 | - | - | - | - | - | Ara-C | 60 | SC | tid q4d x 2 | 10 |
| 8 | 20 | SNS-595 | 5 | IV | Q4d x 2 | 5 | Ara-C | 40 | SC | tid q4d x 2 | 10 |
| 9 | 20 | SNS-595 | 10 | IV | Q4d x 2 | 5 | Ara-C | 20 | SC | tid q4d x 2 | 5 |
| 10 | 20 | SNS-595 | 10 | IV | Q4d x 2 | 5 | Ara-C | 40 | SC | tid q4d x 2 | 10 |

The schedule for dosing and tissue collection is summarized in Table 5.

**Table 5: Schedule for dosing and tissue collection**

| Group | Day 0 | Day 4 | Day 6 | Day 8 | Day 12 | Day 18 |
|---|---|---|---|---|---|---|
| Day | Thur | Mon | Wed | Fri | Tues | Mon |
| 1-10 | dose | Dose | Tissue collect ion | Tissue collection | Tissue collection | Tissue collection |

At various time points described in Table 5, mice were euthanized by $CO_2$ asphyxiation in accordance to Institutional Animal Care and Use Committee (IACUC) guidelines. Blood was collected through cardiac tap and transferred to EDTA tubes. The analysis of murine blood CBC and differential counts for samples in these tubes was conducted (at Quality Clinical Labs, Moutainview).

Femurs were harvested for histological evaluation. The femur was placed in Streck fixative, paraffin embedded, sectioned, transferred to slides, and H&E stained (at BioPathology Sciences). Immunohistochemical slides were processed and analyzed for percent cellularity of intact bone marrow.

Statistical significance was determined by one-tailed student t-test, using Welch's correction for unequal variances.

## Results

Body Weight changes observed in the study are summarized in Table 6.

**Table 6: Summary for body weight changes.**

| Group | N | Compound | Dose (mg/kg) | Route | Schedule | % of Body Wt Nadir | Day of body Wt Nadir |
|---|---|---|---|---|---|---|---|
| 1 | 20 | Vehicle | 0 | IV | q4d x 2 | 0% | 0 |
| 1 | 20 | Vehicle | 0 | SC | tid q4d x 2 | 0% | 0 |
| 2 | 20 | SNS-595 | 5 | IV | q4d x 2 | 0% | 0 |
| 3 | 20 | SNS-595 | 10 | IV | q4d x 2 | 0% | 0 |
| 4 | 20 | SNS-595 | 15 | IV | q4d x 2 | -2.8% | 7 |
| 5 | 20 | Ara-C | 20 | SC | tid q4d x 2 | -0.3% | 7 |
| 6 | 20 | Ara-C | 40 | SC | tid q4d x 2 | 0% | 0 |
| 7 | 20 | Ara-C | 60 | SC | tid q4d x 2 | -1.7% | 7 |
| 8 | 20 | SNS-595 | 5 | IV | q4d x 2 | -8.0% | 7 |
| 8 | 20 | Ara-C | 40 | SC | tid q4d x 2 | -8.0% | 7 |
| 9 | 20 | SNS-595 | 10 | IV | q4d x 2 | -17.2% | 9 |
| 9 | 20 | Ara-C | 20 | SC | tid q4d x 2 | -17.2% | 9 |
| 10 | 20 | SNS-595 | 10 | IV | q4d x 2 | -29.7% | 9 |
| 10 | 20 | Ara-C | 40 | SC | tid q4d x 2 | -29.7% | 9 |

**FIG. 10** shows the % body weight loss plotted over time after q4d x 2 IV administrations of 5, 10, and 15 mg/kg of SNS-595. The maximum body weight loss was observed on day 7. The maximum body weight loss observed did not exceed 3%.

**FIG. 11** shows the % body weight loss plotted over time after tid q4d x2 IP administrations of 20, 40, and 60 mg/kg of Ara-C. The maximum body weight loss was observed on day 7. The maximum body weight loss observed did not exceed 2%.

**FIG. 12** shows the % body weight loss plotted over time after combination administration of SNS-595 q4d x2 IV at 5 and 10 mg/kg and Ara-C tid q4d x2 IP at 20 and 40 mg/kg. Maximum body weight losses were observed on day 9. At 10 mg/kg of SNS-595 and 40 mg/kg of Ara-C, average body weight loss exceeded 20% and the dose was considered toxic. The results from this group were not analyzed further. The other two combination groups did not lose more than 20% of their original body weight and were allowed to continue and have their results analyzed.

### 7.5.1 CYTOLOGY OF NEUTROPHILS

**FIG. 13** shows the number of neutrophils/μl plotted over time after q4d x2 IV administrations of 5, 10, and 15 mg/kg of SNS-595. The nadir of the number of neutrophils was observed on day 8. At all dose levels, the number of neutrophils was significantly lower than the vehicle group on day 8 ($p<0.01$). For all dose levels, the number of neutrophils returned to normal levels by day 12.

**FIG. 14** shows the number of neutrophils/μl plotted over time after tid q4d x2 IP administrations of 20, 40, and 60 mg/kg of Ara-C. The nadir of the number of neutrophils for the two highest dose levels (40 and 60 mg/kg) was observed on day 8 and for the lowest level (20 mg/kg) was observed on day 6. At all dose levels, the number of neutrophils was significantly lower than the vehicle group on day 8 ($p<0.01$). For all dose levels, the number of neutrophils returned to normal levels by day 12.

**FIG. 15** shows the number of neutrophils/μl plotted over time after q4d x2 IV administrations of 5 mg/kg of SNS-595, tid q4d x2 IP administrations of 40 mg/kg of Ara-C, and the combination of the two doses. The nadir of the number of neutrophils for the combination dose group was observed on day 8. At all dose levels, the number of neutrophils was significantly lower than the vehicle group on day 8 ($p<0.01$). Additionally, the combination dose was significantly lower than the single agent SNS-595 group and the single agent Ara-C on day 8 ($p<0.05$). For all dose levels, the number of neutrophils returned to normal levels by day 12.

**FIG. 16** shows the number of neutrophils/μl plotted over time after q4d x2 IV administrations of 10 mg/kg of SNS-595, tid q4d x2 IP administrations of 20 mg/kg of Ara-C, and the combination of the two doses. The nadir of the number of neutrophils for the combination dose group was observed on day 8. At all dose levels, the number of neutrophils was significantly lower than the vehicle group on day 8 ($p<0.01$). Additionally, the combination dose was significantly lower than the single agent SNS-595 group and the single agent Ara-C on day 8 ($p<0.01$). For all dose levels, the number of neutrophils returned to normal levels by day 12, with the combination group producing levels of neutrophils that were elevated above normal.

### 7.5.2 CYTOLOGY OF TOTAL WHITE BLOOD CELLS

**FIG. 17** shows the number of white blood cells/μl plotted over time after q4d x2 IV administrations of 5, 10, and 15 mg/kg of SNS-595. A dose-dependent reduction in circulating white blood cells was observed. At the highest dose level (15 mg/kg), the number of white blood cells was significantly lower than the vehicle group on day 8 ($p<0.01$). For all dose levels, the number of white blood cells failed to return to normal levels by day 18.

**FIG. 18** shows the number of white blood cells/μl plotted over time after tid q4d x2 IP administrations of 20, 40, and 60 mg/kg of Ara-C. There was a dose-dependent reduction in circulating white blood cells. At the highest dose level (60 mg/kg), the number of white blood cells was significantly lower than the vehicle group on day 8 ($p<0.01$). For all dose levels, the number of white blood cells failed to return to normal levels by day 18.

**FIG. 19** shows the number of white blood cells/μl plotted over time after q4d x2 IV administrations of 5 mg/kg of SNS-595, tid q4d x2 IP administrations of 40 mg/kg of Ara-C, and the combination of the two doses. The nadir of the number of white blood cells for the combination dose group was observed on day 6. For the combination dose group, the number of white blood cells was significantly lower than the vehicle group on day 8 ($p<0.01$). For the combination dose group, the number of white blood cells returned to levels expressed by the vehicle group by day 18.

**FIG. 20** shows the number of white blood cells/μl plotted over time after q4d x2 IV administrations of 10 mg/kg of SNS-595, tid q4d x2 IP administrations of 20 mg/kg of Ara-C, and the combination of the two doses. The nadir of the number of white blood cells for the combination dose group was observed on day 8. For the single agent SNS-595 group and the combination group, the number of circulating white blood cells was significantly lower than the vehicle group on day 8 ($p<0.01$). Additionally, the combination dose produced a significantly lower number of white blood cells than the single agent SNS-595 group ($p<0.05$) and the single agent Ara-C on day 8 ($p<0.01$). For all dose groups, the number of white blood cells returned to levels expressed by the vehicle group by day 18.

### 7.5.3 CYTOLOGY OF PLATELETS

**FIG. 21** shows the number of platelets/μl plotted over time after q4d x2 IV administrations of 5,10, and 15 mg/kg of SNS-595. The nadir of the number of platelets for the combination dose group was observed on day 6. At the two highest dose levels (10 and 15 mg/kg), the number of platelets decreased significantly lower than the vehicle group on day 6 ($p<0.01$). Only at the lowest dose level (5 mg/kg) did the number of platelets not significantly decrease lower than the vehicle group on day 6 ($p>0.05$). For all dose levels, the number of platelets returned to normal levels by day 12, with the highest dose of SNS-595 rebounding by displaying a small increase over the vehicle group.

**FIG. 22** shows the number of platelets/μl plotted over time after tid q4d x2 IP administrations of 20, 40, and 60 mg/kg of Ara-C. The nadir of the platelets for the 20 and 60 mg/kg dose levels was observed on day 6 and for the 40

mg/kg dose level was observed on day 8. At all dose levels, the number of platelets was significantly lower than the vehicle group on day 6 and day 8 ($p<0.01$). For all dose levels, the number of platelets returned to normal levels by day 12, with the two highest dose levels of Ara-C rebounding by displaying an increase over the vehicle group.

**FIG. 23** shows the number of platelets/μl plotted over time after q4d x2 IV administrations of 5 mg/kg of SNS-595, tid q4d x2 IP administrations of 40 mg/kg of Ara-C, and the combination of the two doses. The nadir of the platelets for the combination dose group was observed on day 8. For the combination dose group, the number of platelets was significantly lower than the vehicle group on day 6 and day 8 ($p<0.01$). For the combination dose group, the number of platelets rebounded above normal levels by day 12.

**FIG. 24** shows the number of platelets/μl plotted over time after q4d x2 IV administrations of 10 mg/kg of SNS-595, tid q4d x2 IP administrations of 20 mg/kg of Ara-C, and the combination of the two doses. The nadir of the number of platelets for the combination dose group was observed on day 6. For the combination dose group, the number of platelets was significantly lower than the vehicle group on day 6 and day 8 ($p<0.01$). Additionally, the combination dose was significantly lower than the single agent SNS-595 group ($p<0.01$) and the single agent Ara-C ($p<0.05$) on day 6. For the combination dose group, the number of platelets rebounded above normal levels by day 12.

### 7.5.4 FEMORAL BONE MARROW CELLULARITY

**FIG. 25** shows a panel of representative cross sections of femurs from across the groups after q4d x2 IV administrations of 5, 10, and 15 mg/kg of SNS-595 or tid q4d x 2 SC administration of 20, 40 and 60 mg/kg of Ara-C or co-administration of 5,10 mg/kg q4d x 2 IV SNS-595 and 40, 20 mg/kg tid q4d x 2 SC Ara-C respectively. The groups dosed with SNS-595 as a single agent demonstrated a dose-dependent reduction in bone marrow cellularity. No dose dependent reduction was observed for single agent administration of Ara-C. The co-administration of 10 mg/kg of SNS-595 and 20 mg/kg of Ara-C resulted in the greatest reduction in cellularity compared to all other groups, including the highest dose of single agent SNS-595 (15 mg/kg) or Ara-C (60 mg/kg). SNS-595 dosed once a day at 10 mg/kg IV as a single agent and Ara-C dosed three times a day as a single agent at 20 mg/kg SC resulted in 44% and 12% reductions in cellularity, respectively. In contrast, combination dosing of SNS-595 once a day at 10 mg/kg IV with Ara-C dosed three times a day at 20 mg/kg SC resulted in a 93% reduction in cellularity. Normal cellularity recovered on Day 12 (8 days post last dose).

**FIG. 26** shows a panel of representative cross sections of femurs from across the groups after q4d x2 IV administrations of 15 mg/kg of SNS-595 or tid q4d x2 SC administration of 60 mg/kg of Ara-C or co-administration of 5, 10 mg/kg q4dx2 IV SNS-595 and 40, 20 mg/kg tid q4d x2 SC Ara-C, respectively. Normal cellularity recovered on Day 12 (8 days post last dose) across the four groups.

The study indicated that the combination administration of SNS-595 IV at 10 mg/kg and Ara-C SC at 40 mg/kg was toxic and the results were not analyzed. The other groups did not lose more than 18% body weight during the course of the study. At their highest dosing levels, SNS-595 and Ara-C significantly reduced peripheral neutrophils, total white blood cells, and platelets. A dose-dependent decrease in bone marrow cellularity was observed for administration SNS-595, but this was not observed for administration of Ara-C. The combination administration of SNS-595 IV at 5 mg/kg and Ara-C SC at 40 mg/kg significantly reduced peripheral neutrophils to lower levels than the single agent administrations. The combination administration of SNS-595 IV at 10 mg/kg and Ara-C SC at 20 mg/kg significantly reduced peripheral neutrophils, total white blood cells, and platelets to lower levels than the single agent administrations. Average total bone marrow cellularity across treatment groups is summarized in Table 7.

**Table 7. Average total bone marrow cellularity**

| Group | Compound | Dose (mg/kg) | Route | Schedule | Average Total Cellularity Day 6 | Average Total Cellularity Day 12 |
|---|---|---|---|---|---|---|
| 1 | Vehicle | 0 | IV | q4d x 2 | 98% | No data |
| | Vehicle | 0 | SC | tid q4d x 2 | | |
| 2 | SNS-595 | 5 | IV | q4dx2 | 70% | No data |
| 3 | SNS-595 | 10 | IV | q4dx2 | 56% | No data |
| 4 | SNS-595 | 15 | IV | q4d x 2 | 26% | 100% |
| 5 | Ara-C | 20 | SC | tid q4d x 2 | 88% | No data |
| 6 | Ara-C | 40 | SC | tid q4d x 2 | 90% | No data |

| Group | Compound | Dose (mg/kg) | Route | Schedule | Average Total Cellularity Day 6 | Average Total Cellularity Day 12 |
|---|---|---|---|---|---|---|
| 7 | Ara-C | 60 | SC | tid q4d x 2 | 58% | 99% |
| 8 | SNS-595 | 5 | IV | q4d x 2 | 24% | 99% |
| | Ara-C | 40 | SC | tid q4d x 2 | | |
| 9 | SNS-595 | 10 | IV | q4d x 2 | 7% | 100% |
| | Ara-C | 20 | SC | tid q4d x 2 | | |

The combination administration of SNS-595 IV at 10 mg/kg and Ara-C SC at 20 mg/kg resulted in lower total cellularity on Day 6 compared to the highest dose levels of SNS-595 administered at 15 mg/kg and Ara-C administered at 60 mg/kg.

## 7.6 EXAMPLE 6: EVALUATION OF SNS-595 MONOTHERAPY AND COMBINATION THERAPY WITH CYTARABINE (ARA-C)

The study comprised administration of single agent SNS-595, Ara-C or the two combined to Female CD-1 mice (7-8 weeks old) in intravenous (IV) and subcutaneous (SC) doses according to the following doses and schedules:

1. SNS-595: 10 and 20 mg/kg IV q4d x 2
(total drug administered = 20 and 40mg/kg)
2. Ara-C: 20 and 60 mg/kg SC tid q4d x 2
(total drug administered = 120 and 360 mg/kg)
3. Combination: SNS-595 at 10 mg/kg IV q4d x 2 (total drug administered = 20mg/kg) and
4. Ara-C at 20 mg/kg SC tid q4d x 2 (total drug administered = 120 mg/kg).

The effects of dosing were studied on changes in body weight, peripheral cytology, and femoral bone marrow cellularity over time as described herein.

Ara-C used in this study was obtained from Henry Schein, Inc.; D-Sorbitol and Methanesulfonic acid from Sigma Aldrich and CD-1 female mice from Charles River Laboratories. SNS-595 was formulated in 0.17% methanesulfonic acid in 5% sorbitol and Ara-C was formulated in sterile water.

Blood was drawn for hematologic analysis on Days 6, 8, 12 and 18 post initiation of treatment. Blood was analyzed at Quality Control Labs (Mountain View, CA) for complete blood count and differential count. Bone marrow smears from femurs on Day 6 and Day 12 were prepared and geimsa stained for differential counts. Femurs were also placed in Streck fixative, processed, sectioned and H&E stained. Femur and bone marrow smears were analyzed at BioPathology Sciences (South San Francisco, CA) for total cellularity and differential count.

**Table 8:** below summarizes the dosing concentrations and schedules for dosing formulations along with the effect of treatment on average cellularity in bone marrow volumes.

**Table 8:** dosing concentrations and schedules for dosing formulations

| Treatment | Total drug administered | Total Cellularity (Avg) Exp #1 | Total Cellularity (Avg) Exp #2 |
|---|---|---|---|
| Vehicle | N/A | 98% | 96% |
| SNS-595 10 mg/kg q4d x 2 | 20 mg/kg | 56% | 54% |
| SNS-595 20 mg/kg q4d x 2* | 40 mg/kg | ND | 18% |
| Ara-C 20 mg/kg tid q4d x 2 | 120 mg/kg | 88% | 92% |
| Ara-C 60 mg/kg tid q4d x 2* | 360 mg/kg | 58% | 54% |
| SNS-595 10 mg/kg q4d x 2 + Ara-C 20 mg/kg tid q4d x 2 | 20 and 120 mg/kg | 7% | 26% |
| *maximum tolerated dose (MTD) in CD-1 mouse model<br>ND: not determined | | | |

Average cellularity in bone marrow was observed two days after completion of treatment with either single agent SNS-595 or Ara-C or a combination of SNS-595 and Ara-C. The compounds were administered on Day 0 and Day 4. As noted in Table 8, in animals treated with 60 mg/kg Ara-C (maximum tolerated dose), bone marrow cellularity was reduced to 58%. In animals treated with 10 mg/kg SNS-595 (50% maximum tolerated dose), the cellularity was reduced to 56%. The combination of 10 mg/kg SNS-595 (50% maximum tolerated dose) co-dosed with 20 mg/kg Ara-C (33% maximum tolerated dose) reduced bone marrow cellularity to 7%.

The changes in bone marrow cellularity following treatment with SNS-595, Ara-C or the combination were reflected in peripheral blood. **FIG. 27** demonstrates a decrease in white blood cells (panel A), neutrophils (panel B) and platelets (panel C) following combination and single agent treatments.

The changes in myeloid cell lineages following treatment with SNS-595, Ara-C or SNS-595 in combination with Ara-C were observed in bone marrow smears and are depicted in **FIG. 28.** As seen in panel A, a numeric decrease in mature neutrophils was noted two days after completion of the SNS-595/Ara-C combination treatment with recovery occurring six days later. Panel B demonstrates a significant increase in immature neutrophils noted on Day 6 in the SNS-595 treated animals which returned to control levels by Day 12. Two days after treatment, blast counts in SNS-595/Ara-C treated animals were slightly elevated relative to the vehicle control, as seen in panel C. These changes in neutrophil lineage are indicative of bone marrow recovery following chemotherapeutic treatment. (Cell counts are an average of three separate fields).

The cellularity of the bone marrow returned to normal levels eight days after treatment with SNS-595/Ara-C combination. The recovery of the marrow was reflected in peripheral blood one week later, *i.e.,* two weeks post treatment, as demonstrated in **FIG. 29.**

This data demonstrates synergistic effect of SNS-595/Ara-C combination in reducing bone marrow cellularity with a subsequent timely recovery using doses below single agent MTDs.

## 7.7 EXAMPLE 7: EFFECT OF ORDER OF ADMINISTRATION OF COMBINED DOSE OF SNS-595 & ARA-C ON TOLERABILITY

The effect of timing of SNS-595 administration when combined with a dose of cytarabine (Ara-C) has on tolerability was studied in 5 groups of CD-1 mice.

The study comprised administration of SNS-595 in one intravenous (IV) dose given every four days, approximately 96 hours apart (q4d x2) and three subcutaneous (SC) doses of Cytarabine (Ara-C) given every four days (tid q4d x2). The effect of administering SNS-595 in combination with either the first, second or third daily dose of Ara-C was studied on tolerability as described below.

**Table 9: Group Designations, doses and schedules**

| Group | N | Compound | Dose (mg/kg) | Schedule | Compound | Dose (mg/kg) | Schedule |
|---|---|---|---|---|---|---|---|
| 1 | 4 | SNS-595 | 10 | q4d x 2 IV | | | |
| 2 | 4 | Ara-C | 20 | tid q4d x 2 SC | | | |
| 3* | 4 | SNS-595 | 10 | q4d x 2 IV | Ara-C | 20 | tid q4d x 2 SC |
| 4** | 4 | SNS-595 | 10 | q4d x 2 IV | Ara-C | 20 | tid q4d x 2 SC |
| 5*** | 4 | SNS-595 | 10 | q4d x 2 IV | Ara-C | 20 | tid q4d x 2 SC |

* On day 0 and 4, SNS-595 and Ara-C are dosed together first, then two additional doses of Ara-C are given alone
** On day 0 and 4, two doses of Ara-C are given first, followed by the final dose of Ara-C combined with SNS-595
*** On day 0 and 4, one dose of Ara-C is administered, then SNS-595 and Ara-C are dosed together, then the final dose of Ara-C is given

**FIG. 30** provides tolerability data, expressed as % body weight change, at 0, 3, 7, 9, 11, 15, and 18 days post initial injection for the administration of SNS-595 alone (group 1), Ara-C alone (group 2), and SNS-595 combined with Ara-C at the first (group 3), second (group 5) or third (group 4) daily dose of Ara-C. The group 3 combination was well tolerated, with mean body weight loss less than 10%. For this combination, on day 0 and 4, SNS-595 and Ara-C were dosed together first, then two additional doses of Ara-C were given alone.

### 7.8 EXAMPLE 8: COMBINATION DOSING OF SNS-595 AND CYTARABINE: EFFECT OF DELAYED DOSING OF SNS-595 ON BONE MARROW CYTOLOGY

This example provides an exemplary study on the effect of combination dosing of cytarabine (Ara-C) and SNS-595, wherein SNS-595 is administered with cytarabine on either day 0 or day 4, on normal bone marrow cellularity and peripheral blood counts. (Note: day four is approximately 96 hours post the first injection of cytarabine.) The study comprises administration of SNS-595 in a single intravenous dose on day 0 or day 4, and three subcutaneous doses of cytarabine given on both day 0 and 4 (tid q4d x2). Exemplary group designations, doses and schedules are provided in Table 10 below.

The study comprised administration of SNS-595 in one intravenous (IV) dose given every four days, approximately 96 hours apart (q4d x2) and three subcutaneous (SC) doses of Cytarabine (Ara-C) given every four days (tid q4d x2). The effect of administering SNS-595 in combination with either the first, second or third daily dose of Ara-C was studied on tolerability as described below.

**Table 10: Group Designations, doses and schedules**

| Group | Compound | Dose (mg/kg) | Schedule | Compound | Dose (mg/kg) | Schedule |
|---|---|---|---|---|---|---|
| 1 | Vehicle | 0 | qd IV | Vehicle | 0 | tid q4d x2 SC |
| 2 | SNS-595* | 10 | qd IV | | | |
| 3 | SNS-595* | 15 | qd IV | | | |
| 4 | SNS-595* | 20 | qd IV | | | |
| 5 | Cytarabine | 20 | tid q4d x2 SC | | | |
| 6 | SNS-595* | 10 | qd IV | Cytarabine | 20 | tid q4d x2 SC |
| 7 | SNS-595* | 15 | qd IV | Cytarabine | 20 | tid q4d x2 SC |
| 8 | SNS-595* | 20 | qd IV | Cytarabine | 20 | tid q4d x2 SC |
| 9 | Vehicle | 0 | qd IV | Vehicle | 0 | tid q4d x2 SC |
| 10 | SNS-595** | 10 | qd IV | | | |
| 11 | SNS-595** | 15 | qd IV | | | |
| 12 | SNS-595** | 20 | qd IV | | | |
| 13 | Cytarabine | 20 | tid q4d x2 SC | | | |
| 14 | SNS-595** | 10 | qd IV | Cytarabine | 20 | tid q4d x2 SC |
| 15 | SNS-595** | 15 | qd IV | Cytarabine | 20 | tid q4d x2 SC |
| 16 | SNS-595** | 20 | qd IV | Cytarabine | 20 | tid q4d x2 SC |

* SNS-595 is administered intravenously on day 0

** SNS-595 is administered intravenously on day 4 (day 4 is approximately 96 hours post first cytarabine injection)

Vehicle treated animals of group 1 are euthanized for sample isolation on days 2 and 6. Groups 2-8 (SNS-595 alone, cytarabine alone, cytarabine plus SNS-595, day 0) are euthanized and samples taken on days 2, 6, 8 and 12. Vehicle treated animals of group 9 are euthanized for sample isolation on days 4 and 6. Groups 10-16 ( SNS-595 alone, cytarabine alone, cytarabine plus SNS-595, day 4) are euthanized and samples taken on days 4, 6, 8 and 12. Normal bone marrow cellularity and peripheral blood neutrophil counts are determined as described in Example 5. The combinations are well tolerated when SNS-595 is administered in combination with cytarabine on day 0 or approximately 96 hours later on day 4 (see **FIG. 31).** The data indicate that a single dose of SNS-595 can be administered in combination with cytarabine on either day 0 or day 4.

When SNS-595 is administered on day 0 in combination with cytarabine, a dose-dependent decrease in bone marrow cellularity in the combination treatment groups occurs by day 2 (48 hours post injection) with a complete recovery of bone marrow cellularity by day 12 (see **FIG. 32).** The peripheral blood neutrophil counts in the animals treated with these combinations reflected the changes in bone marrow cellularity with the nadir of the counts occurring on day 6 (2 days after the second cytarabine injection) and recovery by day 12 (see **FIG. 33).**

When SNS-595 is administered on day 4 (approximately 96 hours post first cytarabine injection) in combination

with cytarabine, mean bone marrow cellularity is approximately 33% by day 6 in the SNS-595/cytarabine treated animals with complete recovery by day 12 (see **FIG. 34).** The peripheral blood neutrophil counts in the animals treated with these combinations reflected the changes in bone marrow cellularity with the nadir of the counts occurring on day 8 (4 days after combination treatment) and recovery by day 12 (see **FIG. 35).**

### 7.9 EXAMPLE 9: COMBINATION DOSING OF SNS-595 AND CYTARABINE: EFFECT OF DELAYED DOSING OF SNS-595 ON BONE MARROW CYTOLOGY

This example provides an exemplary study on the effect of combination dosing of cytarabine (Ara-C) and SNS-595, wherein SNS-595 is administered on either day 1 or day 4 of cytarabine administration, on normal bone marrow cellularity and peripheral blood counts. The study comprises administration of SNS-595 in a single intravenous dose on day 1 or day 4, and six subcutaneous doses of cytarabine thrice every four days. Exemplary group designations, doses and schedules are provided in Table 11 below.

**Table 11: Group Designations, doses and schedules**

| Group | Compound | Dose (mg/kg) | Schedule | Compound | Dose (mg/kg) | Schedule |
|---|---|---|---|---|---|---|
| 1 | Vehicle | 0 | qd IV | Vehicle | 0 | tid q4d x2 SC |
| 2 | SNS-595* | 10 | qd IV | | | |
| 3 | SNS-595* | 15 | qd IV | | | |
| 4 | SNS-595* | 20 | qd IV | | | |
| 5 | Cytarabine | 20 | tid q4d x2 SC | | | |
| 6 | SNS-595* | 10 | qd IV | Cytarabine | 20 | tid q4d x2 SC |
| 7 | SNS-595* | 15 | qd IV | Cytarabine | 20 | tid q4d x2 SC |
| 8 | SNS-595* | 20 | qd IV | Cytarabine | 20 | tid q4d x2 SC |
| 9 | Vehicle | 0 | qd IV | Vehicle | 0 | tid q4d x2 SC |
| 10 | SNS-595** | 10 | qd IV | | | |
| 11 | SNS-595** | 15 | qd IV | | | |
| 12 | SNS-595** | 20 | qd IV | | | |
| 13 | Cytarabine | 20 | tid q4d x2 SC | | | |
| 14 | SNS-595** | 10 | qd IV | Cytarabine | 20 | tid q4d x2 SC |
| 15 | SNS-595** | 15 | qd IV | Cytarabine | 20 | tid q4d x2 SC |
| 16 | SNS-595** | 20 | qd IV | Cytarabine | 20 | tid q4d x2 SC |
| *SNS-595 is administered intravenously on day 0<br>** SNS-595 is administered intravenously on day 4 | | | | | | |

Vehicle treated animals of group 1 are euthanized for sample isolation on days 2 and 6. Groups 5-8 (Cytarabine alone, cytarabine plus SNS-595, day 0) are euthanized and and samples taken on days 2, 6, 8 and 12. Vehicle treated animals of group 9 are euthanized for sample isolation on days 4 and 6. Groups 13-16 (Cytarabine alone, cytarabine plus SNS-595, day 4) are euthanized and and samples taken on days 4, 6, 8 and 12. Normal bone marrow cellularity and peripheral blood cell counts are determined as described in Example 5.

### 7.10 EXAMPLE 10: TREATMENT OF HEMATOLOGIC MALIGNANCIES WITH SNS-595 IN COMBINATION WITH CYTARABINE (ARA-C)

Pharmaceutical compositions comprising a combination of SNS-595 and cytarabine (Ara-C) as described above are used to treat acute myeloid leukemia (AML) in a subject in need thereof. Patients with refractory or relapsed AML may receive up to 4 cycles (see Treatment Schema, below), consisting of 1 or 2 induction-therapy cycles (Induction, Reinduction) and 1 or 2 consolidation-therapy cycles (Consolidation 1, Consolidation 2). A complete therapeutic regimen is defined as a minimum 28-day period (or a minimum of 22 days during Induction if a patient will receive Reinduction

therapy), during which patients receive SNS-595 Injection on Days 1 and 4 in combination with a 5-day continuous IV infusion of cytarabine, followed by weekly observations until hematologic recovery.

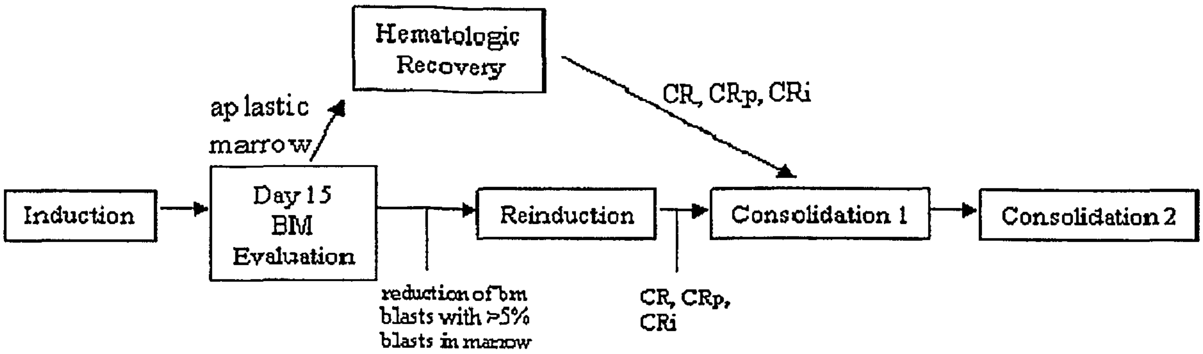


**Treatment Schema**

Abbreviations:

BM = bone marrow
CR = complete remission
CRi = morphologic CR with incomplete blood count recovery
CRp = morphologic CR with incomplete platelet recovery

Subjects undergo safety assessments including physical examination, vital signs, hematology, serum chemistry, urinalysis, assessment of adverse events (AEs), and leukemia-associated symptoms (LAS). Evaluation of disease response is according to the International Working Group (IWG) criteria (Cheson et al., J Clin Oncol. 21(24); 4642-49 (2003); *see also* Table 11). A bone marrow biopsy/aspirate is obtained at Screening, on Day 15 (window of Day 13-17) during Induction and Reinduction to assess persistent leukemia, and at the time of hematologic recovery during Induction and Reinduction to document clinical response. Bone marrow aspirate and blood samples are collected for exploratory biomarker analysis, and blood samples for PK analysis. After completion of treatment, patients are followed monthly for survival. Patients can be categorized as having a complete remission (CR), CR with incomplete platelet recovery (CRp), or CR with incomplete hematologic recovery (CRi; see Table 12 for treatment outcome definitions).

**Table 12: International Working Group (IWG) Response Criteria and Treatment Outcomes**

| Categories of Response | Neutrophils (microL) | Platelets (microL) | BM blasts (%) | Other |
|---|---|---|---|---|
| **Morphologic CR** | > 1000 | > 100,000 | <5 | |
| **Morphologic CR without platelet recovery (CRp)** | > 1000 | ≤100,000 | <5 | |
| **Morphologic CR with incomplete blood count recovery (CRi)** | ≤1000 | ≤100,000 | <5 | not defined |
| **Partial remission** | > 1000 | > 100,000 | > 50 or decrease to 5-25 | blasts less than 5% if Auer rod positive |
| **Recurrence** | not defined | not defined | ≤5 | relapse after CR; reappearance or development of EMD, reappearance of molecular or cytogenetic abnormality |
| Abbreviations: AML = acute myeloid leukemia; BM = bone marrow; CR = complete remission; CRi = morphologic CR with incomplete blood count recovery CRp = morphologic CR with incomplete platelet recovery; PB = peripheral blood; PR = partial remission. Recurrence indicates a prior CR. | | | | |

A baseline bone marrow (BM) biopsy / aspirate is collected from the patient 14 days before Induction Day 1 to assess the patient's disease. A portion of this BM biopsy/aspirate sample may be utilized for biomarker (pharmacodynamic) analysis. If this sample is not utilized, a second BM biopsy or aspirate sample is obtained prior to Induction therapy for biomarker analysis. Potential pharmacodynamic biomarkers include mRNA, proteins, and phosphoproteins involved in DNA repair, such as histone yH2AX, and apoptosis, such as caspase 3. These markers are measured before and after treatment to assess the effect of SNS-595 Injection plus cytarabine on blood cells, and to provide potential predictive indicators of clinical outcome.

### 7.10.1 PHARMACEUTICAL COMPOSITIONS COMPRISING SNS-595 & ARA-C

SNS-595 Injection is a clear, pale-yellow liquid formulated for IV administration in 10-mL Type 1 glass vials. Each vial contains 100 mg SNS-595 at a concentration of 10 mg/mL and a pH of 2.5. Each mL contains 45 mg of D-sorbitol to maintain isotonicity, and methanesulfonic acid for pH control. This sterile, nonpyrogenic solution is manufactured under Good Manufacturing Practices (GMP) and is formulated without preservatives.

Cytarabine is commercially available and should be reconstituted in 5% dextrose and water (D5W).

### 7.10.2 ADMINISTRATION OF SNS-595 INJECTION

Undiluted SNS-595 Injection is administered as a slow IV push or via a syringe pump, over approximately 10 minutes, at a volume calculated to supply the prescribed dose. To calculate the dose for all treatments, the patient's body surface area (BSA) obtained on Induction Day 1 is used. If body weight has changed by 10% or more (evaluated on Day 1 of each cycle), BSA is recalculated for subsequent doses. A dose calculation example is presented below. The Mosteller formula is recommended, but not required:

$$\text{Mosteller formula:} \quad \text{BSA}\ (m^2) = \sqrt{\ }([\text{height (cm)} \times \text{weight (kg)}] \div 3600)$$

The following is an example of a Mosteller formula dose calculation based on a patient with a height of 183 cm and a weight of 82 kg, which gives the patient the following BSA:

$$\text{BSA}\ (m^2) = \sqrt{\ }([183\ \text{cm} \times 82\ \text{kg}] \div 3600)$$

$$\text{BSA}\ (m^2) = 2.0 \quad (\text{round to nearest } 10^{th})$$

*See* Mosteller RD, Simplified calculation of body surface area. N Engl J Med. 317(17):1098 (1987).

On the day of treatment, an IV infusion of D5W is started at a keep vein open (KVO) rate until the start of the IV push; at which time, the D5W infusion rate is increased to 100 mL/hour. Using aseptic technique, SNS-595 Injection is drawn into a syringe, and slowly injected into the IV port closest to the catheter. No other medication is administered in the same IV line (port or catheter) while SNS-595 Injection is being administered.

### 7.10.3 ADMINISTRATION OF CYTARABINE (ARA-C)

Cytarabine is reconstituted in D5W and administered according to the commercial package insert. Treatment with cytarabine is not in the same IV line (port or catheter) while SNS-595 Injection is being administered.

### 7.10.4 TREATMENT SCHEDULE

Patients receive SNS-595 Injection on Days 1 and 4 in combination with a 5-day continuous IV infusion of cytarabine in the infusion regimens outlined below.

#### 7.10.4.1 Induction Treatment

The dose of SNS-595 Injection is delivered as a 10-minute IV push. Cytarabine is administered on days 1 through 5 at 400 $mg/m^2$/day as a continuous IV infusion. Cytarabine administration is initiated immediately following

treatment with SNS-595 Injection on Day 1, in a line separate from that used to administer SNS-595 Injection.

#### 7.10.4.2 Reinduction Treatment

A patient may be deemed eligible for reinduction treatment based upon a bone marrow (BM) biopsy or aspirate performed on Day 15 (with a window of Days 13-17). Reinduction is initiated no later than 2 weeks after the Day 15 BM biopsy or aspirate. For Reinduction Treatment, SNS-595 Injection is administered at the same dosage as was used in Induction treatment. Similarly, cytarabine is administered at the same dosage as was used in Induction treatment.

#### 7.10.4.3 Consolidation Treatment

A patient may be deemed eligible for Consolidation Treatment if they have a CR, $CR_p$, or $CR_i$ following Induction or Reinduction. For Consolidation Treatment, SNS-595 Injection is administered at the same dosage as was used in Induction treatment. Similarly, cytarabine is administered at the same dosage as was used in Induction treatment. Consolidation 1 is initiated no earlier than 28 days (Study Day 28) after the first treatment of Induction (or Reinduction) and no later than 84 days after the first administration of SNS-595 and/or Ara-C. A patient may be deemed eligible for Consolidation 2 treatment if they have maintained peripheral blood evidence of a CR, $CR_p$, or $CR_i$ following Consolidation 1. A recovery bone marrow assessment is not necessary for Consolidation 2 treatment. Consolidation 2 is initiated no earlier than 28 days after the first treatment of Consolidation 1.

### 7.10.5 <u>ANTILEUKEMIC ACTIVITY ASSESSMENTS</u>

Antileukemic activity assessment is based on the IWG response criteria (see Table 11). BM biopsies or aspirate are performed for determination of response at Screening, on Day 15 (window of Days 13 - 20) of Induction and Reinduction, and at the time of hematologic recovery following Induction/Reinduction.

The embodiments described above are intended to be merely exemplary, and those skilled in the art will recognize, or will be able to ascertain using no more than routine experimentation, numerous equivalents of specific compounds, materials, and procedures. All such equivalents are considered to be within the scope of the invention and are encompassed by the appended claims.

### <u>Certain Items:</u>

**1.** A method of treating leukemia comprising administering to a mammal having a leukemia a therapeutically effective combination of an enantiomerically pure (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid and Ara-C, wherein the Ara-C is administered in a total amount of about 5-1500 mg/m2 per day for at least one day.

**2.** The method of item 1, wherein the leukemia is chronic lymphocytic leukemia, chronic myelocytic leukemia, acute lymphoblastic leukemia or acute myelogenous leukemia.

**3.** The method of item 2, wherein the acute lymphoblastic leukemia originates in the blast cells of the bone marrow, thymus or lymph nodes.

**4.** The method of item 3, wherein the acute lymphoblastic leukemia is a T-cell leukemia.

**5.** The method of item 4, wherein the T-cell leukemia is a peripheral T-cell leukemia, T-cell lymphoblastic leukemia, cutaneous T-cell leukemia or adult T-cell leukemia.

**6.** The method of item 1, wherein the leukemia is an acute myelogenous leukemia.

**7.** The method of item 6, wherein the acute myelogenous leukemia is a myeloblastic leukemia or promyelocytic leukemia.

**8.** The method of item 1, wherein the leukemia is relapsed, refractory or resistant to conventional therapy.

**9.** The method of item 9, wherein the relapsed or refractory leukemia is de novo acute myeloid leukemia or secondary

acute myeloid leukemia.

**10.** The method of item 10, wherein the secondary acute myeloid leukemia is a therapy related acute myeloid leukemia.

**11.** The method of any one of items 1-10, further comprising administering a therapeutically effective amount of another second active agent or a support care therapy.

**12.** The method of item 11, wherein the other second active agent is a therapeutic antibody that specifically binds to a cancer antigen, hematopoietic growth factor, cytokine, anti-cancer agent, antibiotic, cox-2 inhibitor, immunomodulatory agent, immunosuppressive agent, corticosteroid or a pharmacologically active mutant or derivative thereof.

**13.** The method of item **11,** wherein the second active agent is an alkylating agent, an anti-neoplastic antibiotic, an anti-metabolite, a platinum coordination complex, a topoisomerase II inhibitor or radiation.

**14.** The method of item **11,** wherein the second active agent is etoposide, daunomycin, actinomycin D, mitomycin C, cisplatin, carboplatin, premetrexed, methotrexate, 5-Fu, wortmannin, geldanamycin, gemcitabin or a combination thereof.

**15.** The method of item **1** or 11 wherein the amount of Ara-C is about 200-400 mg/m$^2$/day.

**16.** The method of item **1** or 11 wherein the amount of Ara-C is about 400 mg/m$^2$/day.

**17.** The method of item 1 or 11 wherein the amount of Ara-C is about 10-50 mg/m$^2$/day.

**18.** The method of item 1 or 11 wherein the amount of Ara-C is about 20 mg/m$^2$/day.

**19.** The method of item 18 wherein the amount of Ara-C is administered as 10 mg/m$^2$ twice a day.

**20.** The method of item 18 wherein the Ara-C is administered subcutaneously for 10 days.

**21.** The method of item 20 wherein the enantiomerically pure (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid is administered twice a week.

**22.** The method of item 21 wherein the enantiomerically pure (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid administered twice a week is at a dose of about 10-40 mg/m$^2$.

**23.** The method of item 21 wherein the enantiomerically pure (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid is administered twice a week for two weeks.

**24.** The method of item 20 wherein the enantiomerically pure (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid is administered once a week.

**25.** The method of item 24 wherein the enantiomerically pure (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid is administered at a dose of 10mg/m$^2$-90 mg/m$^2$.

**26.** The method of item 24 wherein the enantiomerically pure (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid is administered once a week for three weeks.

**27.** The method of item 1 or 11, wherein the amount of enantiomerically pure (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid administered is from about 1 to about 150 mg/m$^2$.

**28.** The method of item 1 or 11, wherein the amount of enantiomerically pure (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid administered is from about 10 to about 120 $mg/m^2$.

**29.** The method of item 1 or 11, wherein the amount of enantiomerically pure (+)-1,4-dihydro-7-[(3 S,4S)-3-methoxy-4-(methylamino)-1-pyrrolidinyl]-4-oxo-1-(2-thiazolyl)-1,8-naphthyridine-3-carboxylic acid administered is from about 10 to about 90 $mg/m^2$ per week.

**30.** The method of item 1 or 11, wherein the amount of enantiomerically pure (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid administered is about 40 to about 80 $mg/m^2$ per week.

**31.** The method of item 1 or 11 wherein the enantiomerically pure (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid is administered as an IV injection.

**32.** The method of item 1 or 11, wherein the enantiomerically pure (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid is administered twice per week.

**33.** The method of item 15, wherein the enantiomerically pure (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid is administered intravenously twice per week and Ara-C is intraveneously administered continuously over a treatment cycle of 5 days..

**34.** The method of item 33, wherein Ara-C is administered at 400 $mg/m^2$/day.

**35.** The method of item 33, wherein the enantiomerically pure (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid is administered on day 1 and day 4 of said treatment cycle of 5 days.

**36.** The method of item 33, wherein the treatment cycle is repeated at least once.

**37.** The method of item 33, wherein the treatment cycle is repeated at least two times.

**38.** The method of item 33, wherein the treatment cycle is repeated at least three times.

**39.** The method of item 1 or 11, wherein the enantiomerically pure (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid is administered 8 to 16 hours after the start of at least one Ara-C administration.

**40.** The method of item 1 or 11 wherein the enantiomerically pure (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid is administered within 24 hours before or after the start of at least one Ara-C administration.

**41.** The method of item 1 or 11 wherein Ara-C is administered immediately after administration of the enantiomerically pure (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid.

**42.** The method of item 1 or 11, wherein the mammal is a human.

**43.** A combination comprising an enantiomerically pure (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid and Ara-C.

## Claims

1. A compound for use in a method for treating leukemia in a mammal, wherein the compound is (+)-1,4- dihydro-7-[(3S,4S)-3-methoxy-4-(methylamino)-1-pyrrolidinyl]-4-oxo-1-(2-thiazolyl)-1,8-naphthyridine-3-carboxylic acid and the method comprises administering the compound in combination with Ara-C at a dose of about 5-1500 $mg/m^2$, wherein the dose of Ara-C is administered per day for at least one day

2. The compound for use of claim 1, wherein the leukemia is chronic lymphocytic leukemia, chronic myelocytic leukemia or acute lymphoblastic leukemia.

3. The compound for use of claim 2, wherein the acute lymphoblastic leukemia originates in the blast cells of the bone marrow, thymus or lymph nodes.

4. The compound for use of claim 3, wherein the acute lymphoblastic leukemia is a T-cell leukemia.

5. The compound for use of claim 4, wherein the T-cell leukemia is a peripheral T-cell leukemia, T-cell lymphoblastic leukemia, cutaneous T-cell leukemia or adult T-cell leukemia.

6. The compound for use of claim 1, wherein the leukemia is relapsed, refractory or resistant to conventional therapy.

7. The compound for use of any one of claims 1-6, wherein the compound is administered with a therapeutically effective amount of another second active agent or a support care therapy.

8. The compound for use of claim 7, wherein the other second active agent is a therapeutic antibody that specifically binds to a cancer antigen, hematopoietic growth factor, cytokine, anti-cancer agent, antibiotic, cox-2 inhibitor, immunomodulatory agent, immunosuppressive agent, corticosteroid or a pharmacologically active mutant or derivative thereof.

9. The compound for use of claim 8, wherein the second active agent is an alkylating agent, an anti-neoplastic antibiotic, an anti-metabolite, a platinum coordination complex, a topoisomerase II inhibitor or radiation.

10. The compound for use of claim 8, wherein the second active agent is etoposide, daunomycin, actinomycin D, mitomycin C, cisplatin, carboplatin, premetrexed, methotrexate, 5-Fu, wortmannin, geldanamycin, gemcitabin or a combination thereof.

11. The compound for use of claim 1 or 8, wherein the (+)-1,4-dihydro-7-[(3 S,4S)-3-methoxy-4-(methylamino)-1-pyrrolidinyl]-4-oxo-1-(2-thiazolyl)-1, 8-naphthyridine-3-carboxylic acid is administered as an IV injection.

12. The compound for use of claim 11, wherein the (+)-1,4-dihydro-7-[(3S,4S)-3-methoxy-4-(methylamino)-1-pyrrolidinyl]-4-oxo-1-(2-thiazolyl)-1,8-naphthyridine-3-carboxylic acid is administered intravenously twice per week and Ara-C is intravenously administered continuously over a treatment cycle of 5 days.

*FIG. 1*

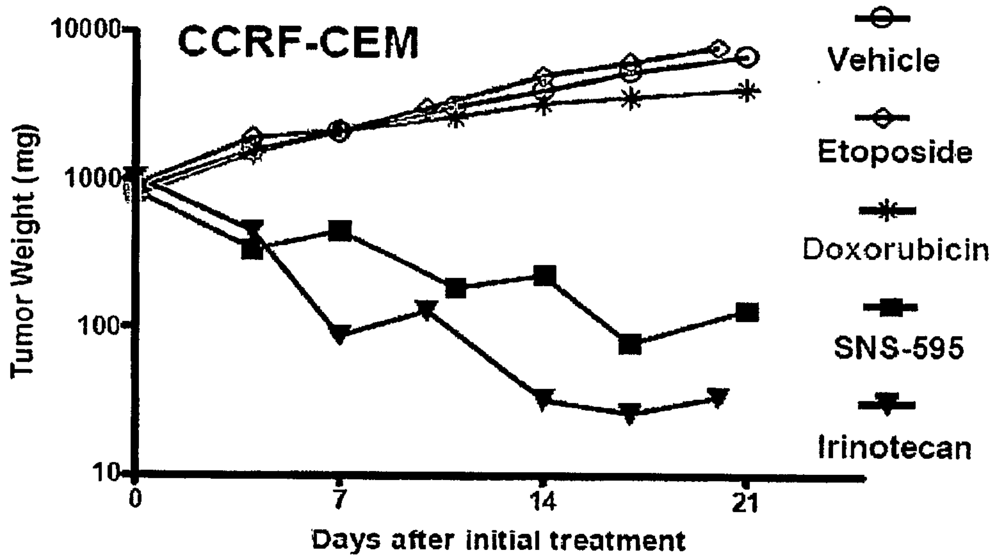
CCRF-CEM
Tumor Weight (mg)
10000
1000
100
10
0
7
14
21
Days after initial treatment
Vehicle
Etoposide
Doxorubicin
SNS-595
Irinotecan

*FIG. 2*

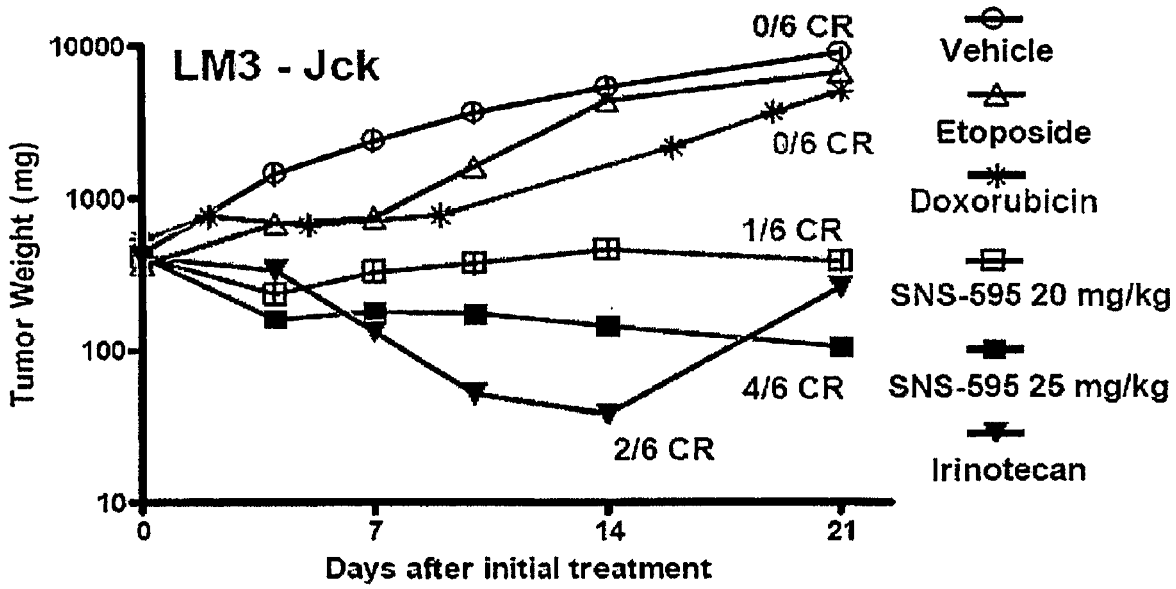
LM3 - Jck
Tumor Weight (mg)
10000
1000
100
10
0
7
14
21
Days after initial treatment
0/6 CR
0/6 CR
1/6 CR
4/6 CR
2/6 CR
Vehicle
Etoposide
Doxorubicin
SNS-595 20 mg/kg
SNS-595 25 mg/kg
Irinotecan

*FIG. 3*

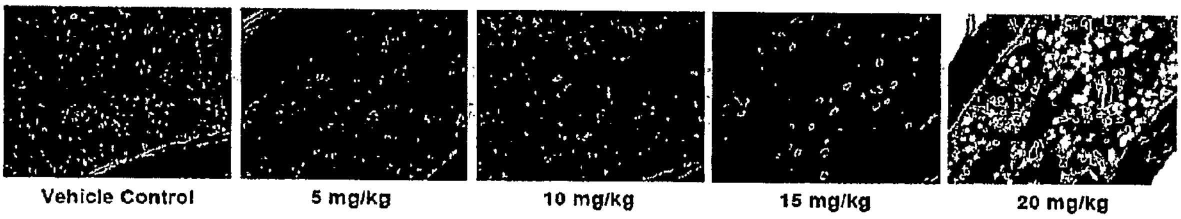

*FIG. 4*

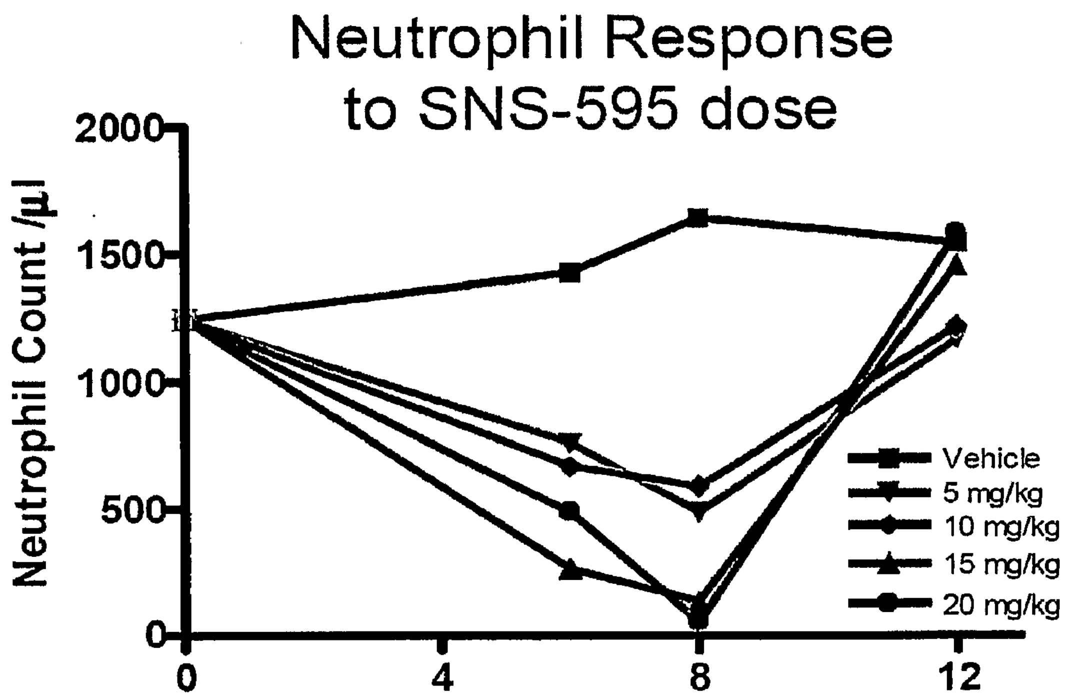
Neutrophil Response
to SNS-595 dose
Neutrophil Count /µl
2000
1500
1000
500
0
0
4
8
12
Vehicle
5 mg/kg
10 mg/kg
15 mg/kg
20 mg/kg

*FIG. 5*

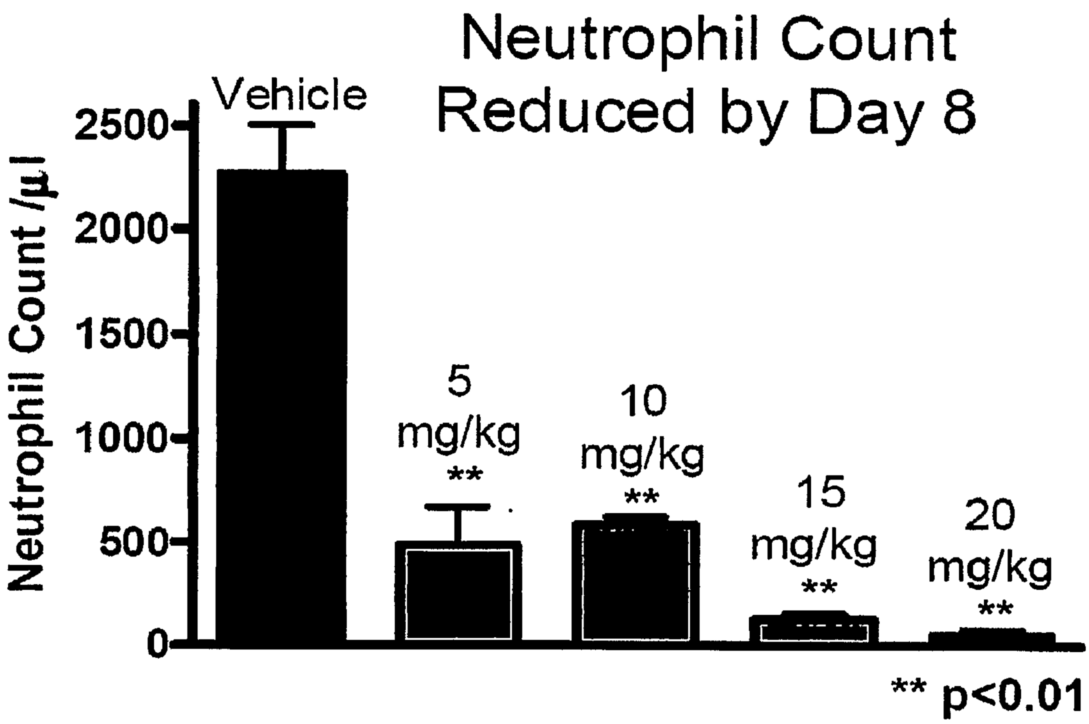
Neutrophil Count
Reduced by Day 8
Neutrophil Count /µl
2500
2000
1500
1000
500
0
Vehicle
5
mg/kg
**
10
mg/kg
**
15
mg/kg
**
20
mg/kg
**
** p<0.01

*FIG. 6*

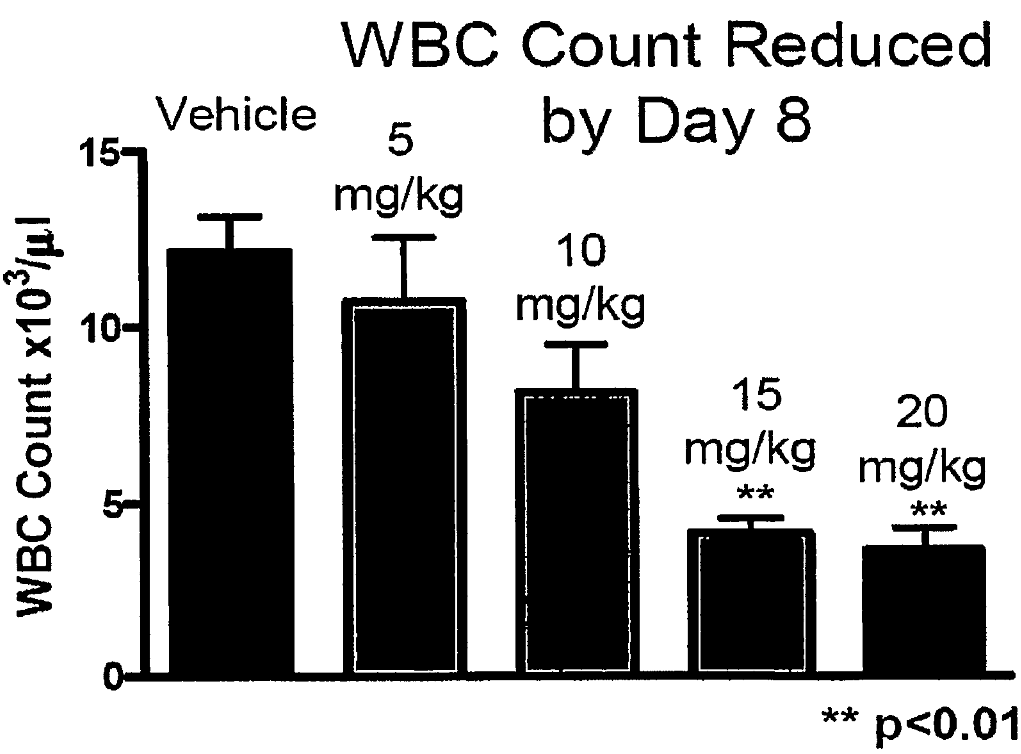
WBC Count Reduced by Day 8
Vehicle
5 mg/kg
10 mg/kg
15 mg/kg
**
20 mg/kg
**
WBC Count x10³/µl
15
10
5
0
** p<0.01

*FIG. 7*

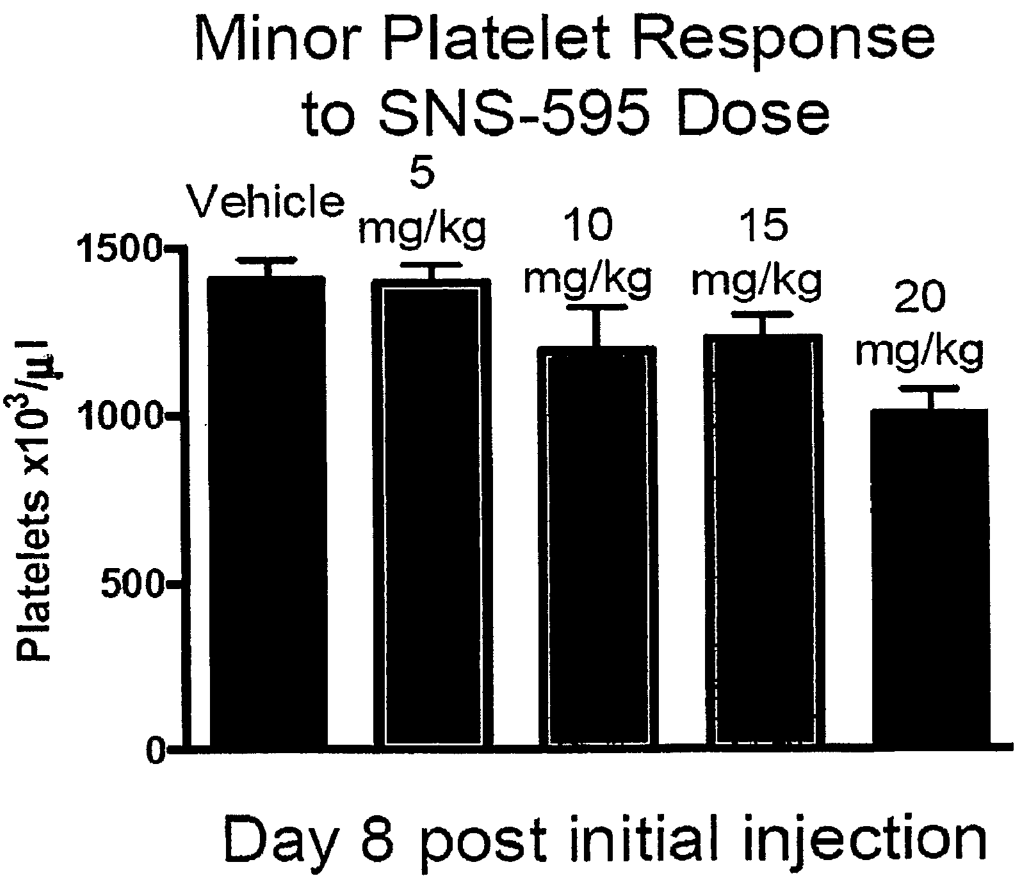
Minor Platelet Response
to SNS-595 Dose
Vehicle
5
mg/kg
10
mg/kg
15
mg/kg
20
mg/kg
1500
1000
500
0
Platelets x10$^3$/µl
Day 8 post initial injection

*FIG. 8*

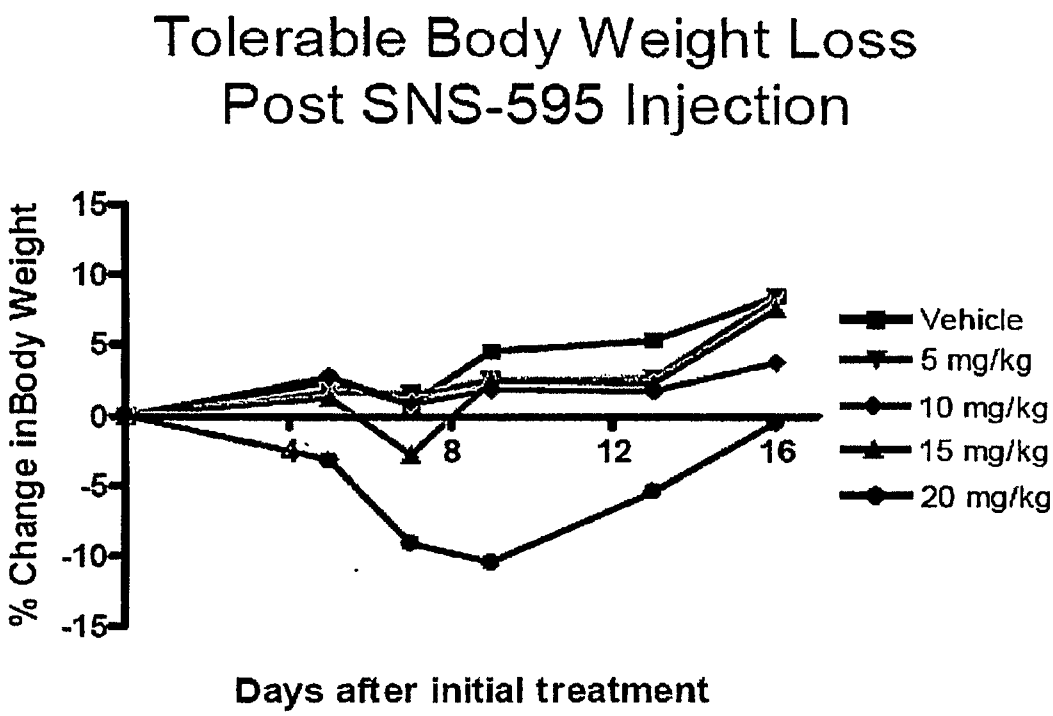
Tolerable Body Weight Loss
Post SNS-595 Injection
% Change inBody Weight
15
10
5
0
-5
-10
-15
4
8
12
16
Days after initial treatment
Vehicle
5 mg/kg
10 mg/kg
15 mg/kg
20 mg/kg

*FIG. 9*

Bone Marrow Rebounds

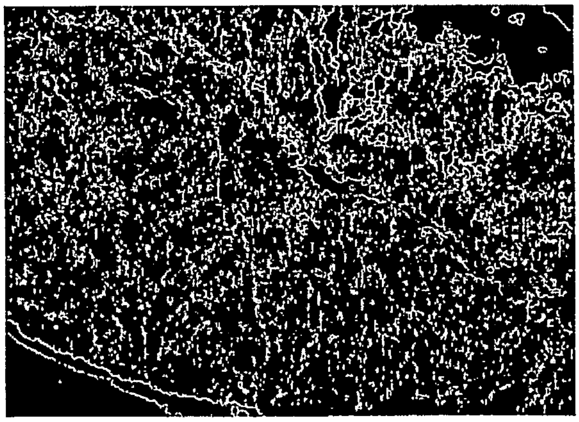

20 mg/kg SNS-595
Day 12

FIG. 10

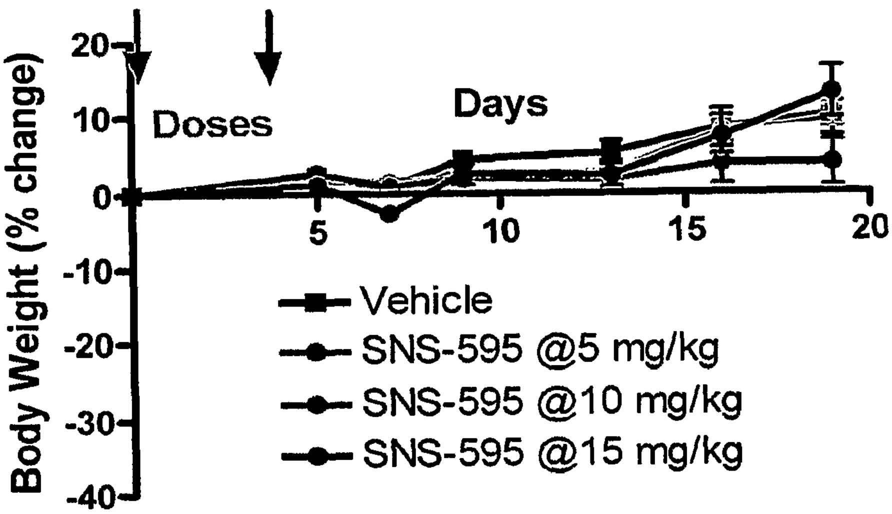
Body Weight (% change)
20
10
0
-10
-20
-30
-40
Doses
Days
5
10
15
20
Vehicle
SNS-595 @5 mg/kg
SNS-595 @10 mg/kg
SNS-595 @15 mg/kg

*FIG. 11*

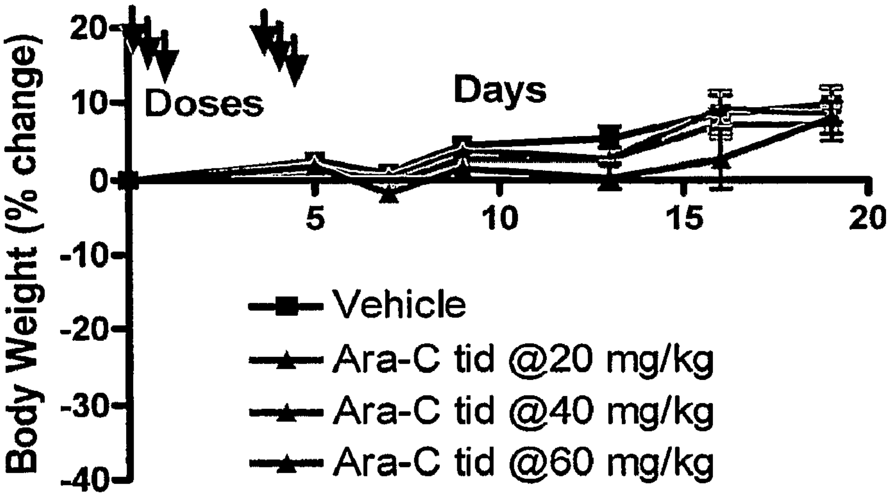
Body Weight (% change)
20
10
0
-10
-20
-30
-40
Doses
Days
5
10
15
20
Vehicle
Ara-C tid @20 mg/kg
Ara-C tid @40 mg/kg
Ara-C tid @60 mg/kg

*FIG. 12*

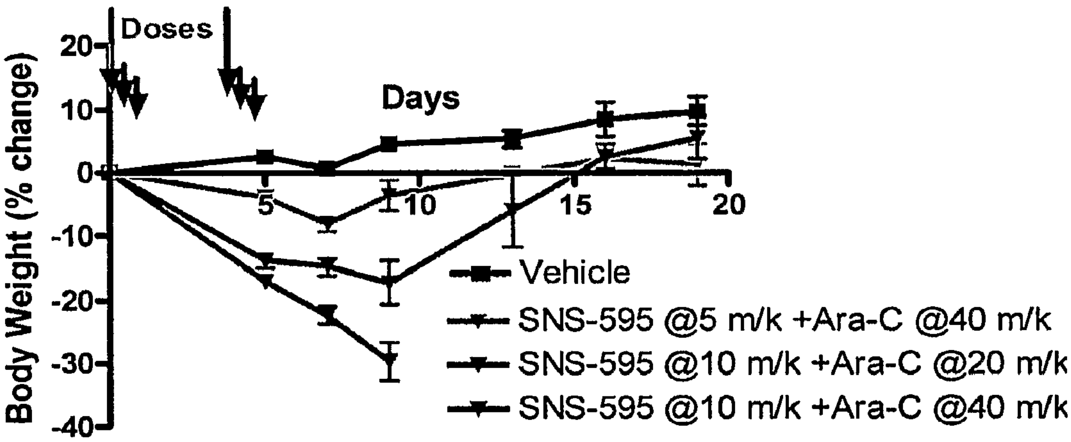
Doses
Days
Body Weight (% change)
20
10
0
-10
-20
-30
-40
5
10
15
20
Vehicle
SNS-595 @5 m/k +Ara-C @40 m/k
SNS-595 @10 m/k +Ara-C @20 m/k
SNS-595 @10 m/k +Ara-C @40 m/k

*FIG. 13*

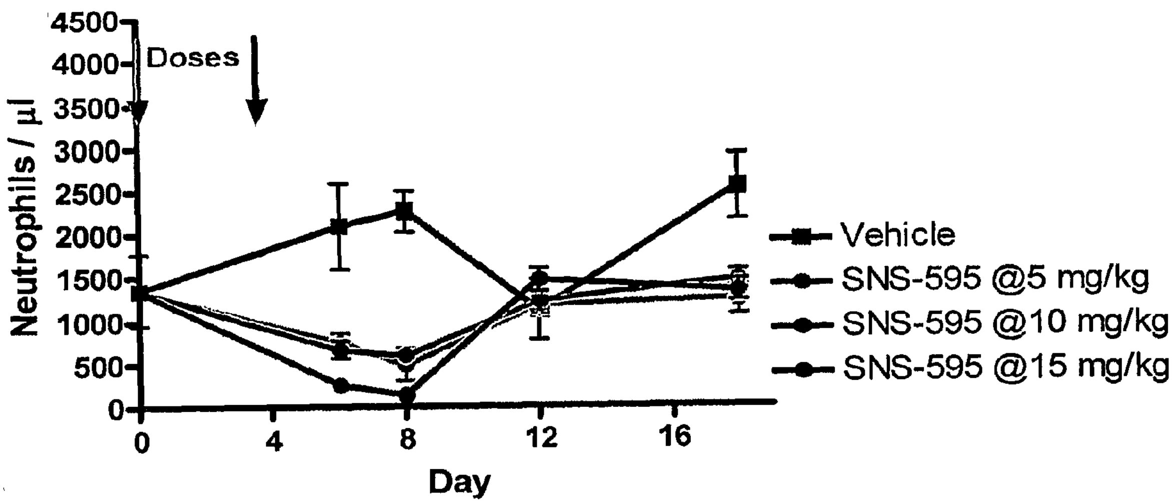
Neutrophils / µl
4500
4000
3500
3000
2500
2000
1500
1000
500
0
Doses
0
4
8
12
16
Day
Vehicle
SNS-595 @5 mg/kg
SNS-595 @10 mg/kg
SNS-595 @15 mg/kg

*FIG. 14*

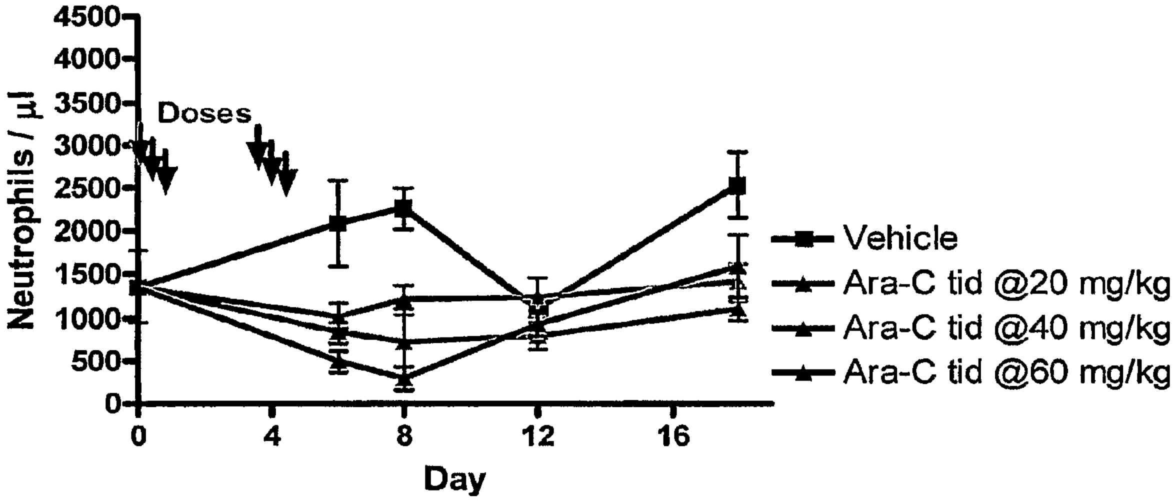
Neutrophils / µl
4500
4000
3500
3000
2500
2000
1500
1000
500
0
Doses
0
4
8
12
16
Day
Vehicle
Ara-C tid @20 mg/kg
Ara-C tid @40 mg/kg
Ara-C tid @60 mg/kg

*FIG. 15*

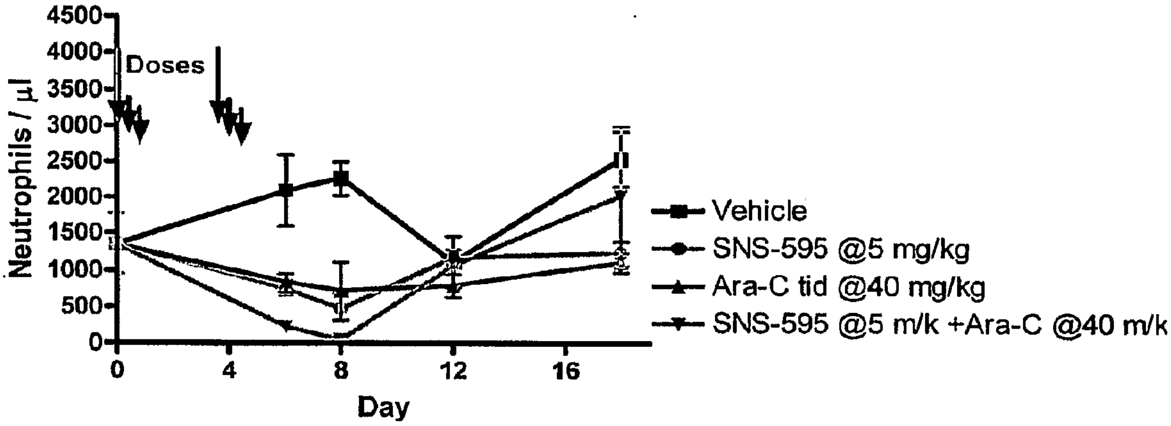
4500
4000
3500
3000
2500
2000
1500
1000
500
0
Neutrophils / µl
Doses
0
4
8
12
16
Day
Vehicle
SNS-595 @5 mg/kg
Ara-C tid @40 mg/kg
SNS-595 @5 m/k +Ara-C @40 m/k

*FIG. 16*

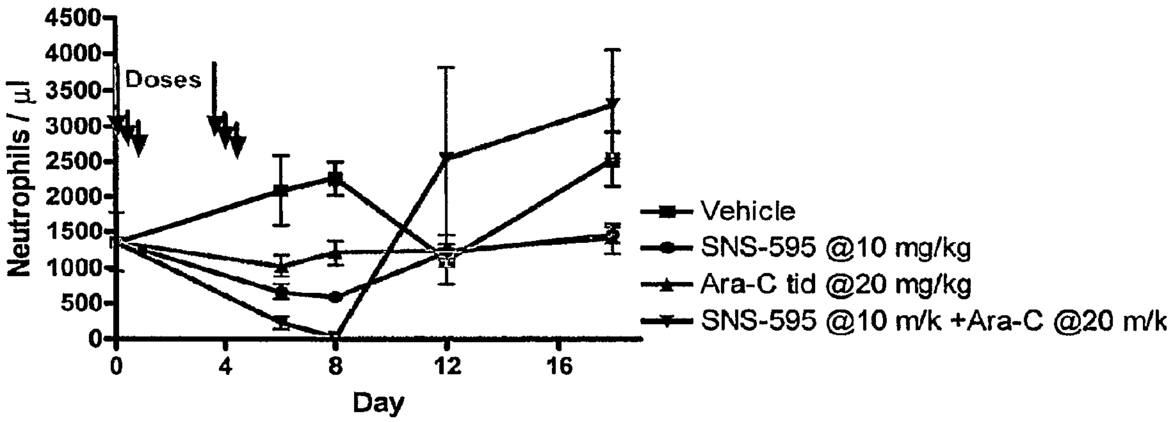
4500
4000
3500
3000
2500
2000
1500
1000
500
0
Neutrophils / µl
Doses
0
4
8
12
16
Day
Vehicle
SNS-595 @10 mg/kg
Ara-C tid @20 mg/kg
SNS-595 @10 m/k +Ara-C @20 m/k

*FIG. 17*

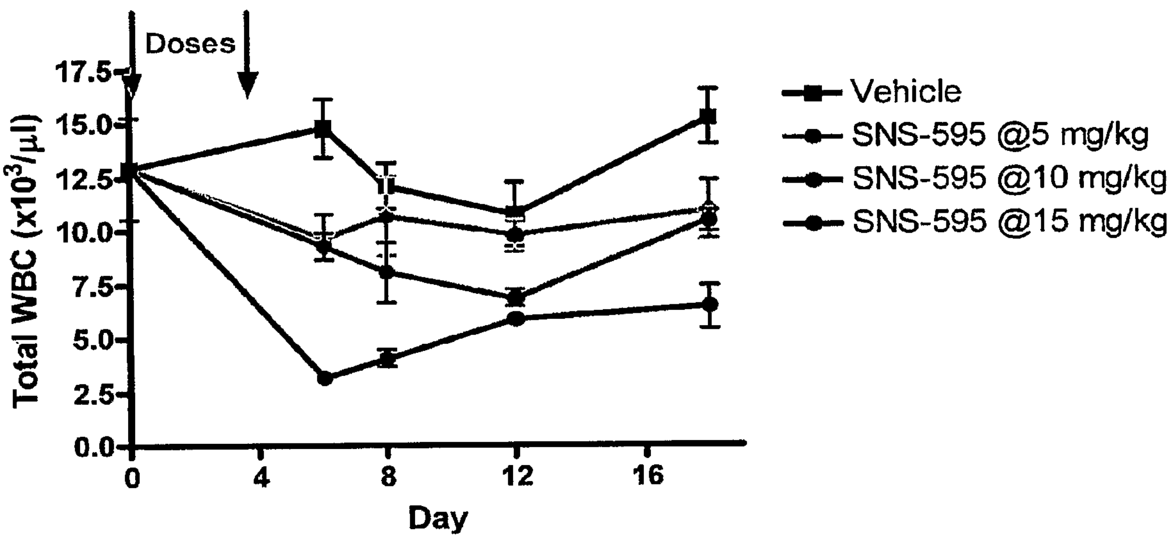
Doses
17.5
15.0
12.5
10.0
7.5
5.0
2.5
0.0
Total WBC (x10³/µl)
0
4
8
12
16
Day
Vehicle
SNS-595 @5 mg/kg
SNS-595 @10 mg/kg
SNS-595 @15 mg/kg

*FIG. 18*

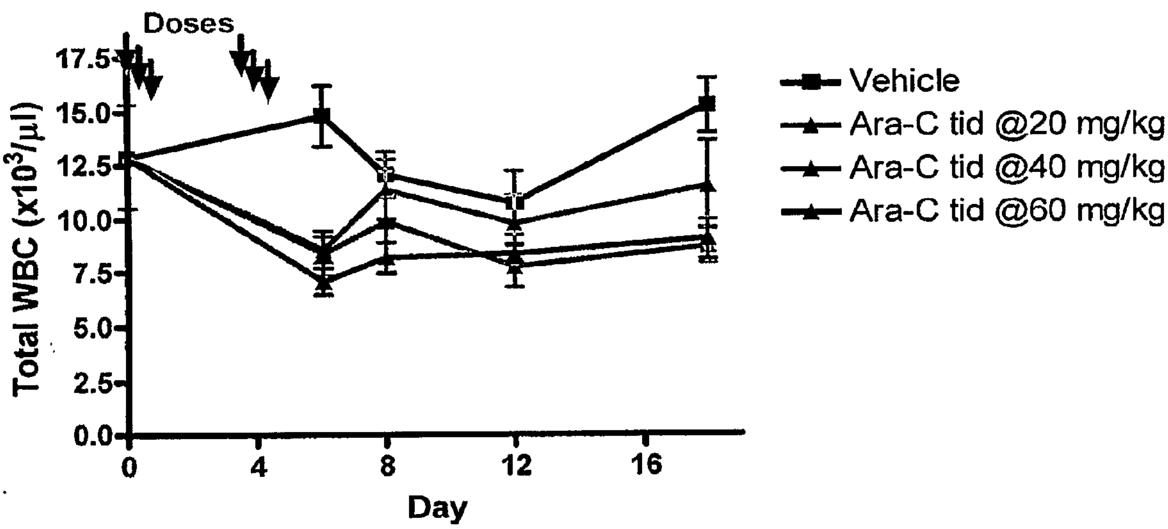
Doses
Total WBC (x10³/μl)
17.5
15.0
12.5
10.0
7.5
5.0
2.5
0.0
0
4
8
12
16
Day
Vehicle
Ara-C tid @20 mg/kg
Ara-C tid @40 mg/kg
Ara-C tid @60 mg/kg

*FIG. 19*

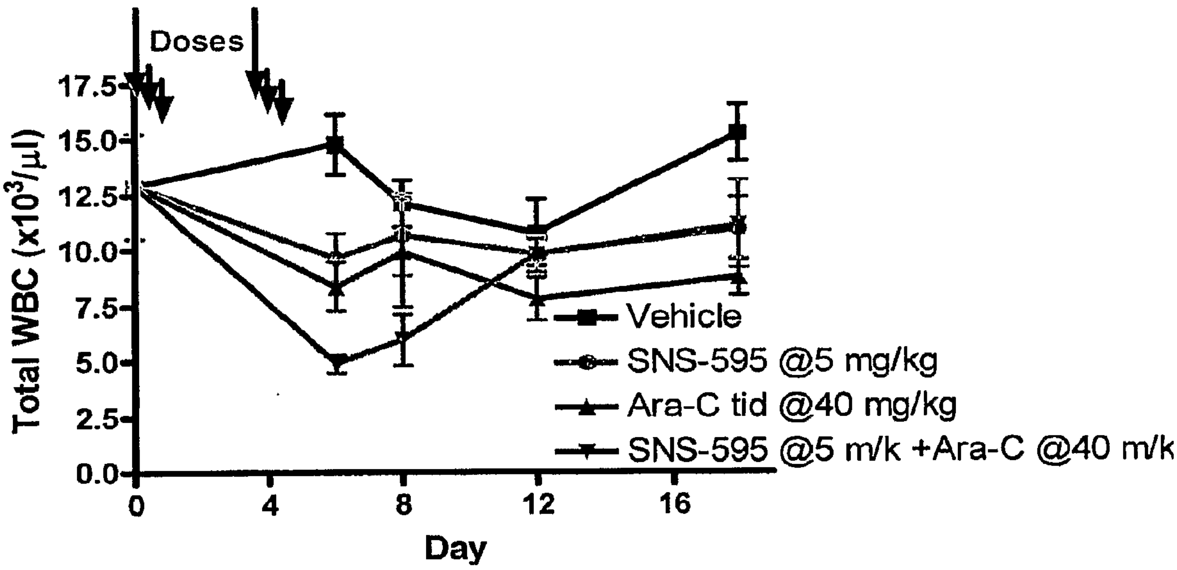
Doses
Total WBC (x10³/μl)
17.5
15.0
12.5
10.0
7.5
5.0
2.5
0.0
0
4
8
12
16
Day
Vehicle
SNS-595 @5 mg/kg
Ara-C tid @40 mg/kg
SNS-595 @5 m/k +Ara-C @40 m/k

*FIG. 20*

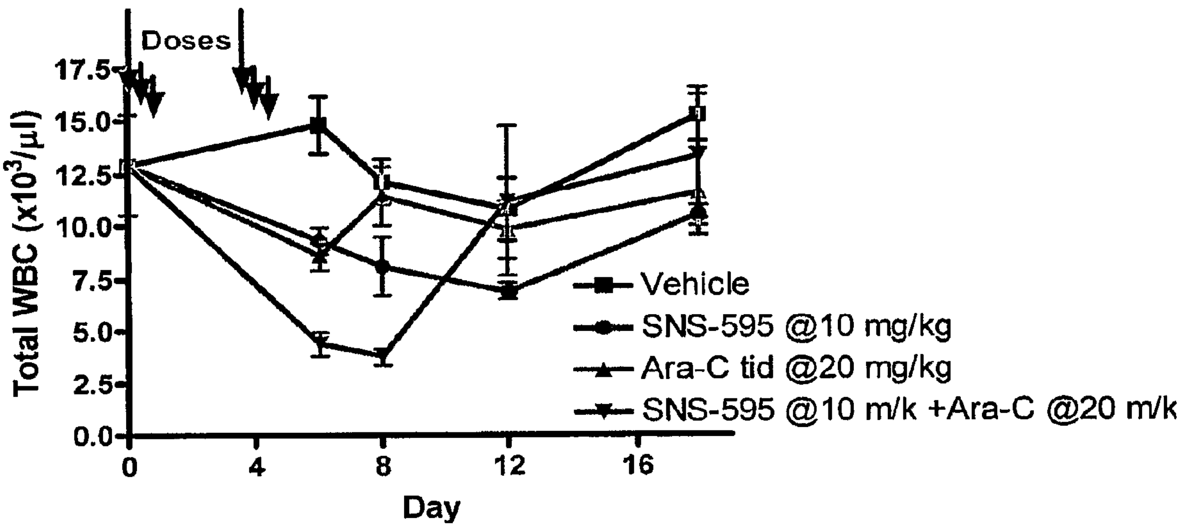
Doses
17.5
15.0
12.5
10.0
7.5
5.0
2.5
0.0
Total WBC (x10³/μl)
0
4
8
12
16
Day
Vehicle
SNS-595 @10 mg/kg
Ara-C tid @20 mg/kg
SNS-595 @10 m/k +Ara-C @20 m/k

*FIG. 21*

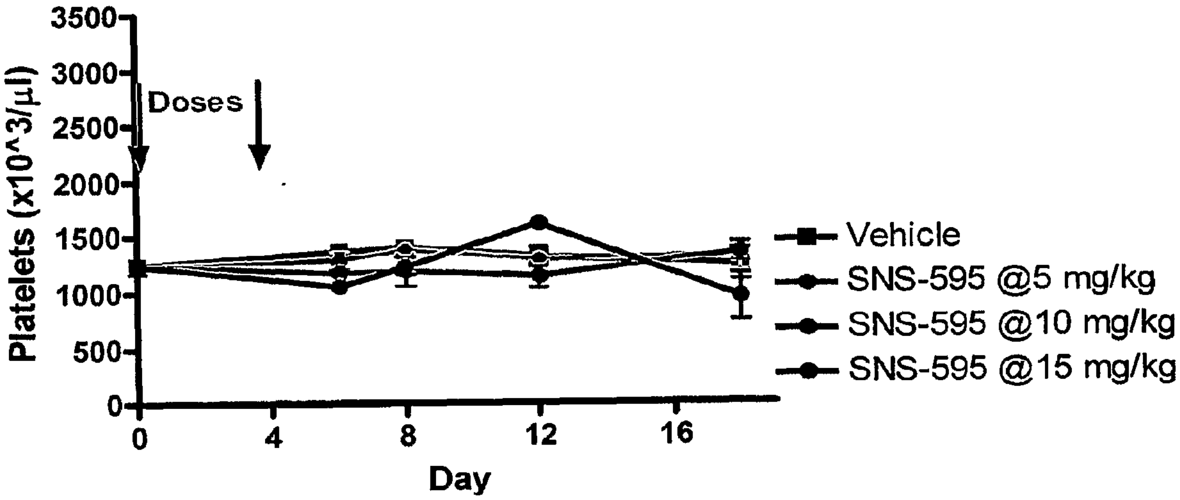
3500
3000
2500
2000
1500
1000
500
0
Platelets (x10^3/μl)
Doses
0
4
8
12
16
Day
Vehicle
SNS-595 @5 mg/kg
SNS-595 @10 mg/kg
SNS-595 @15 mg/kg

*FIG. 22*

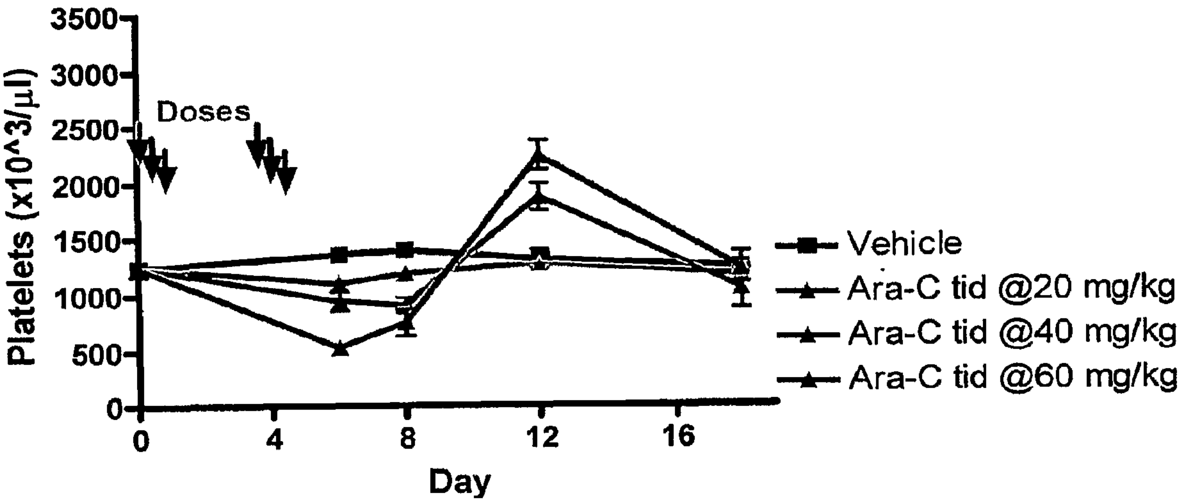
3500
3000
2500
2000
1500
1000
500
0
Platelets (x10^3/μl)
Doses
0
4
8
12
16
Day
Vehicle
Ara-C tid @20 mg/kg
Ara-C tid @40 mg/kg
Ara-C tid @60 mg/kg

*FIG. 23*

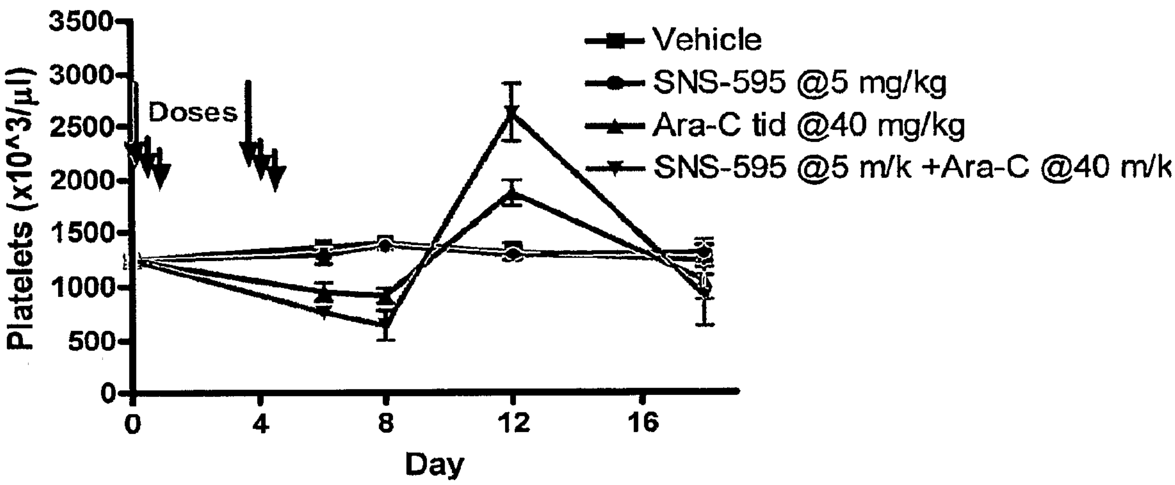
3500
3000
2500
2000
1500
1000
500
0
Platelets (x10^3/µl)
Doses
0
4
8
12
16
Day
Vehicle
SNS-595 @5 mg/kg
Ara-C tid @40 mg/kg
SNS-595 @5 m/k +Ara-C @40 m/k

*FIG. 24*

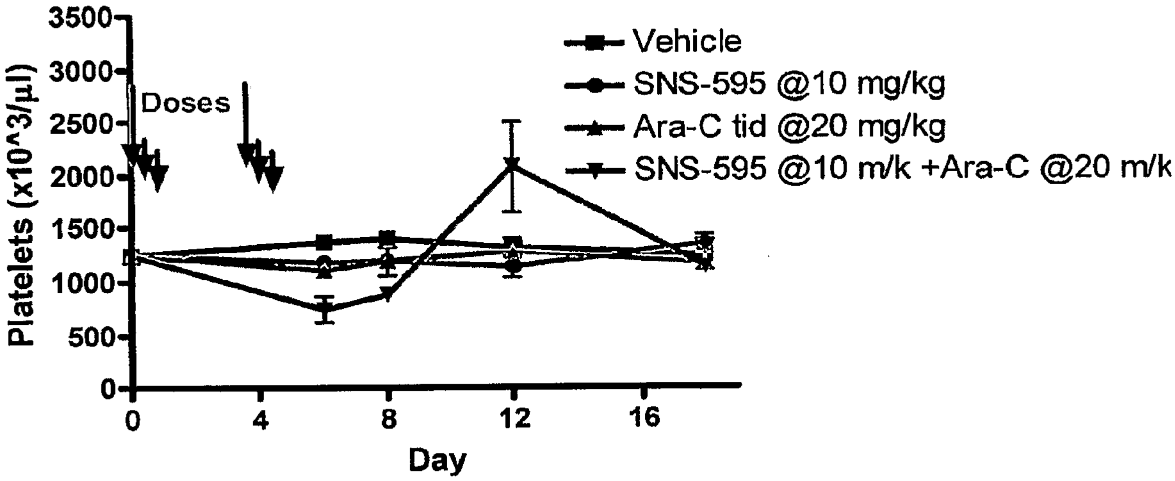
Platelets (x10^3/µl)
3500
3000
2500
2000
1500
1000
500
0
Doses
0
4
8
12
16
Day
Vehicle
SNS-595 @10 mg/kg
Ara-C tid @20 mg/kg
SNS-595 @10 m/k +Ara-C @20 m/k

FIG. 25

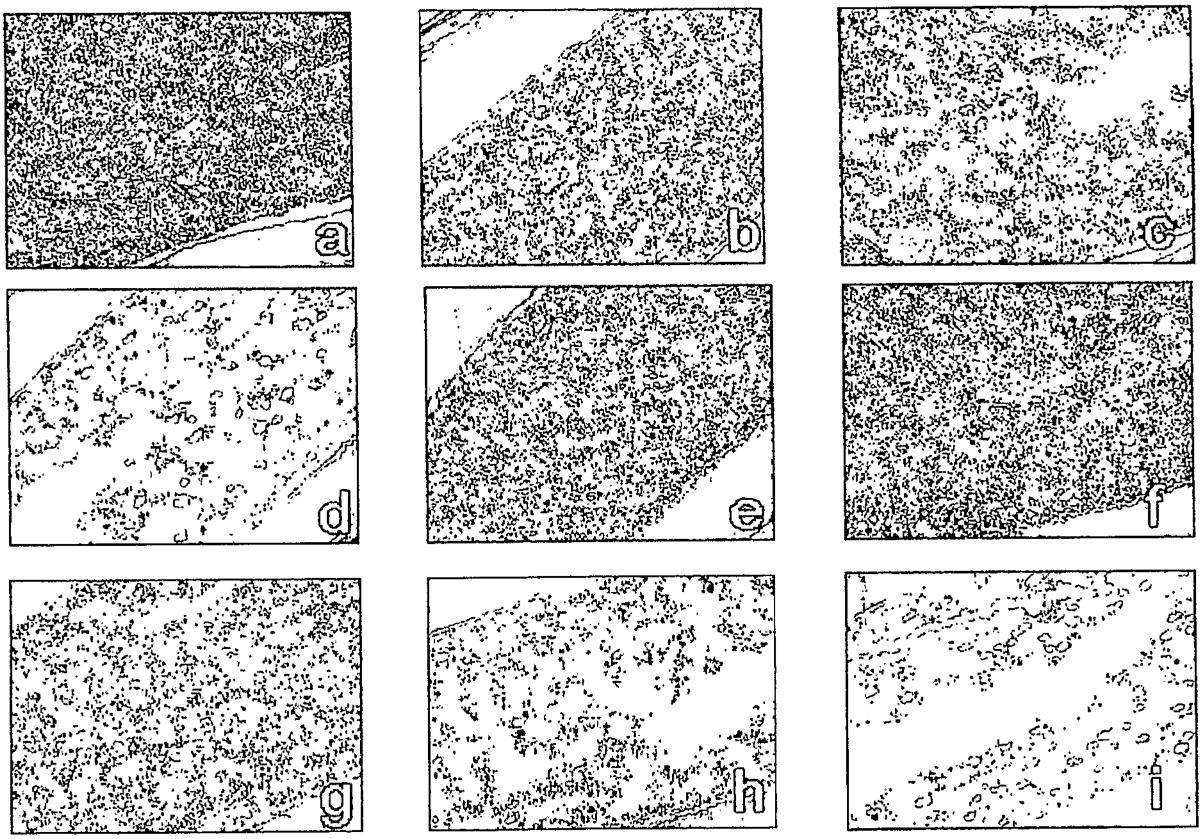

*FIG. 26*

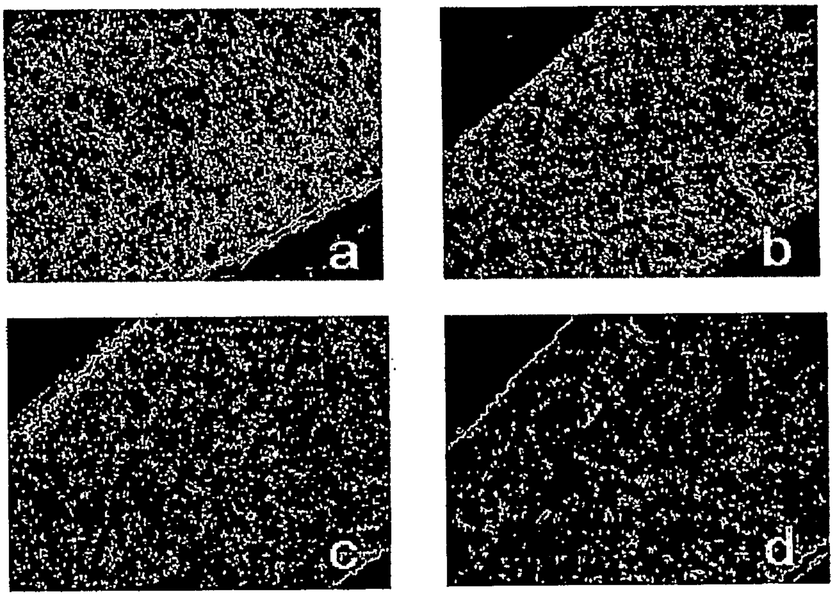

*FIG. 27*

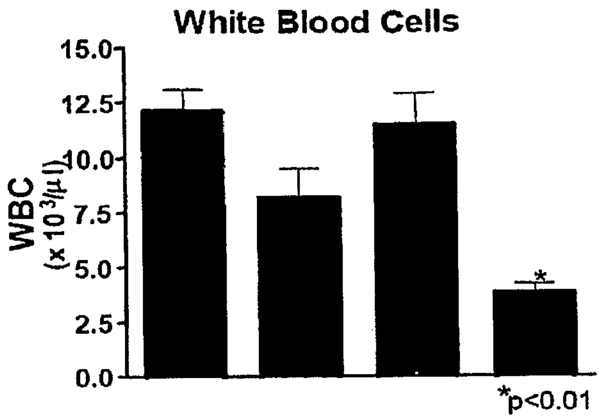


Panel A

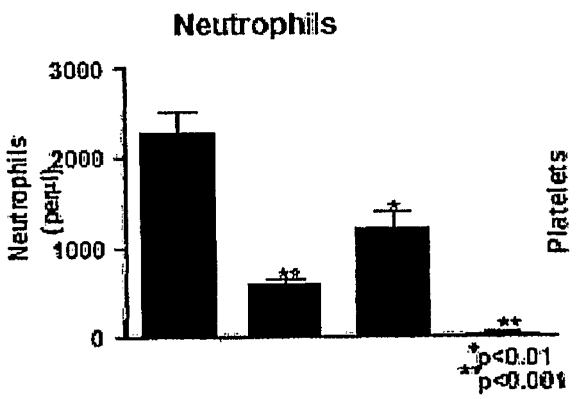


Panel B

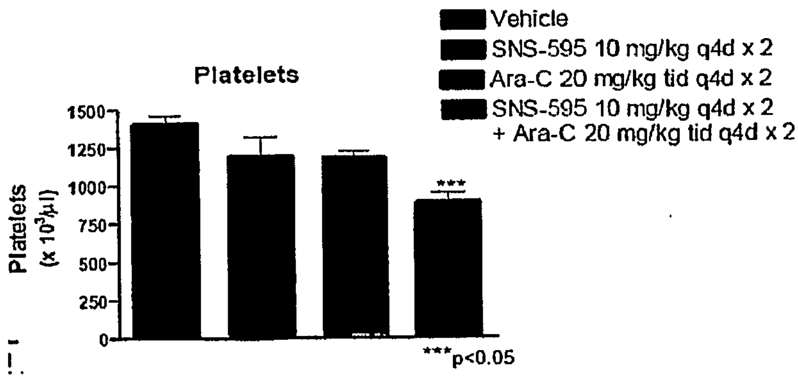


Panel C

*FIG. 28*

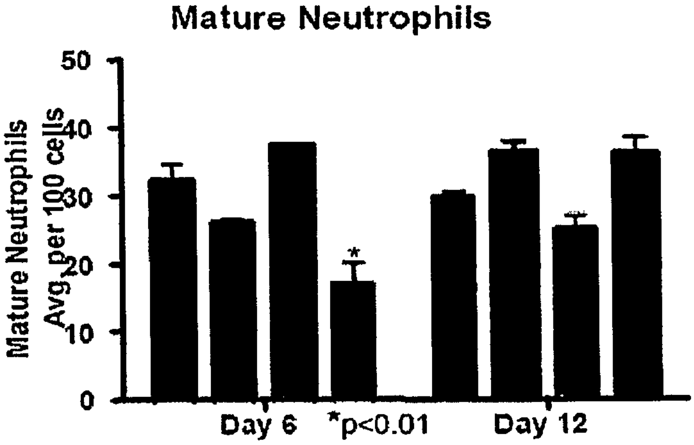


**Panel A**

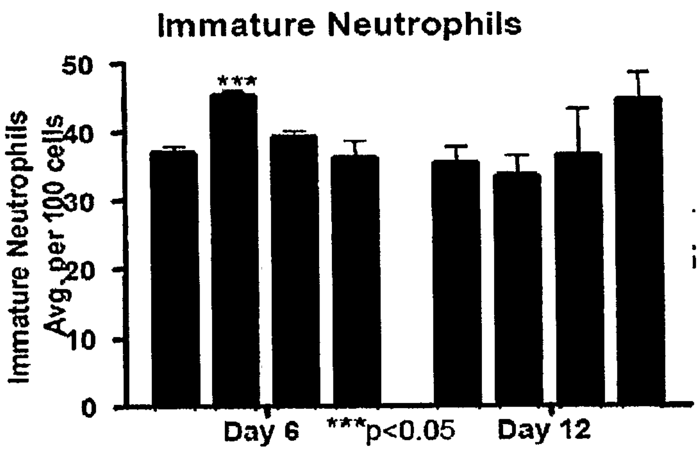


**Panel B**

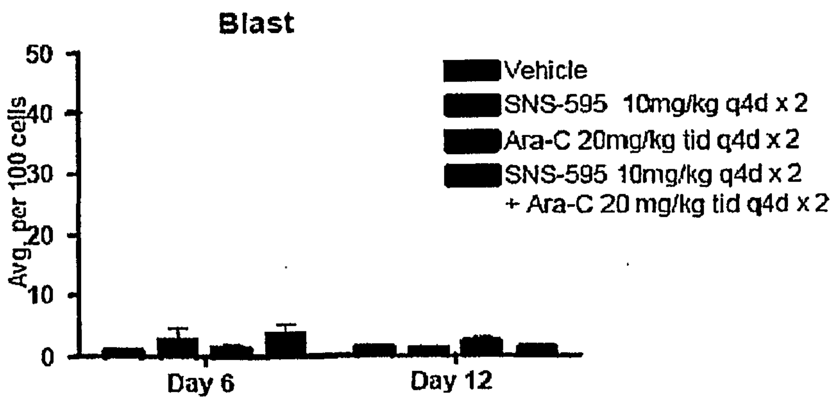


**Panel C**

*FIG. 29*

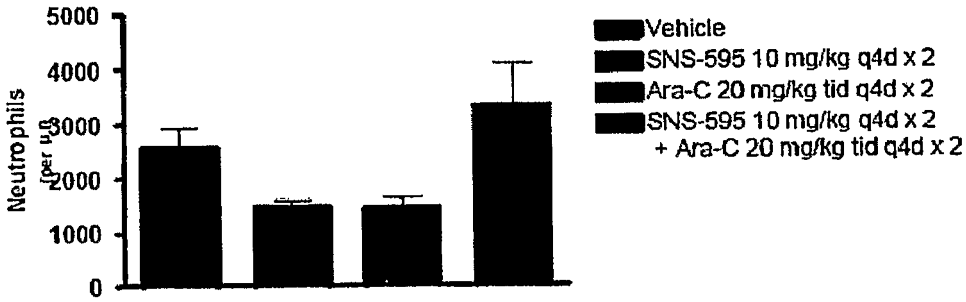
5000
4000
3000
2000
1000
0
Neutrophils (per µl)
Vehicle
SNS-595 10 mg/kg q4d x 2
Ara-C 20 mg/kg tid q4d x 2
SNS-595 10 mg/kg q4d x 2
+ Ara-C 20 mg/kg tid q4d x 2

*FIG. 30*

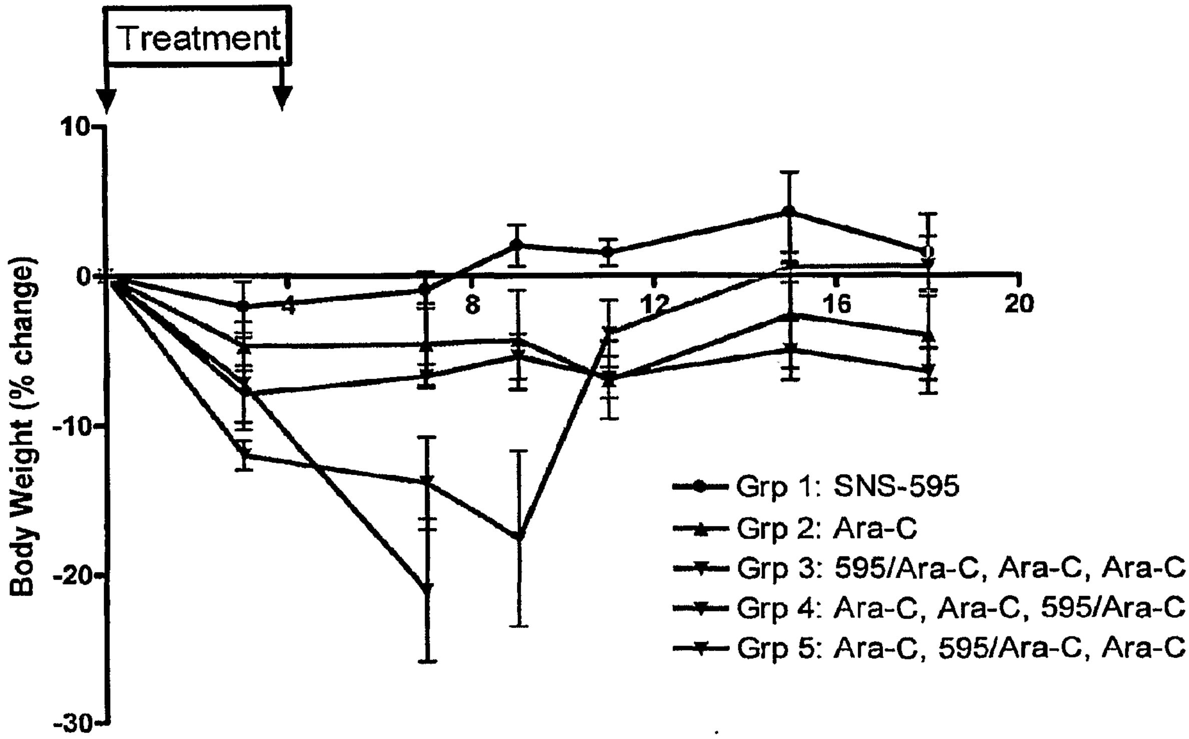
Treatment
Body Weight (% change)
10
0
-10
-20
-30
4
8
12
16
20
Grp 1: SNS-595
Grp 2: Ara-C
Grp 3: 595/Ara-C, Ara-C, Ara-C
Grp 4: Ara-C, Ara-C, 595/Ara-C
Grp 5: Ara-C, 595/Ara-C, Ara-C

*FIG. 31*

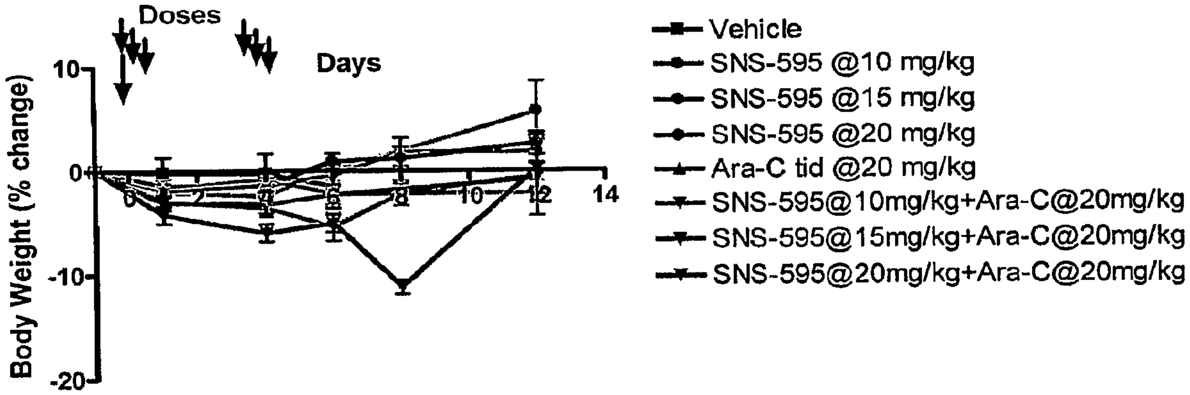
Doses
Days
Body Weight (% change)
10
0
-10
-20
0
2
4
6
8
10
12
14
Vehicle
SNS-595 @10 mg/kg
SNS-595 @15 mg/kg
SNS-595 @20 mg/kg
Ara-C tid @20 mg/kg
SNS-595@10mg/kg+Ara-C@20mg/kg
SNS-595@15mg/kg+Ara-C@20mg/kg
SNS-595@20mg/kg+Ara-C@20mg/kg

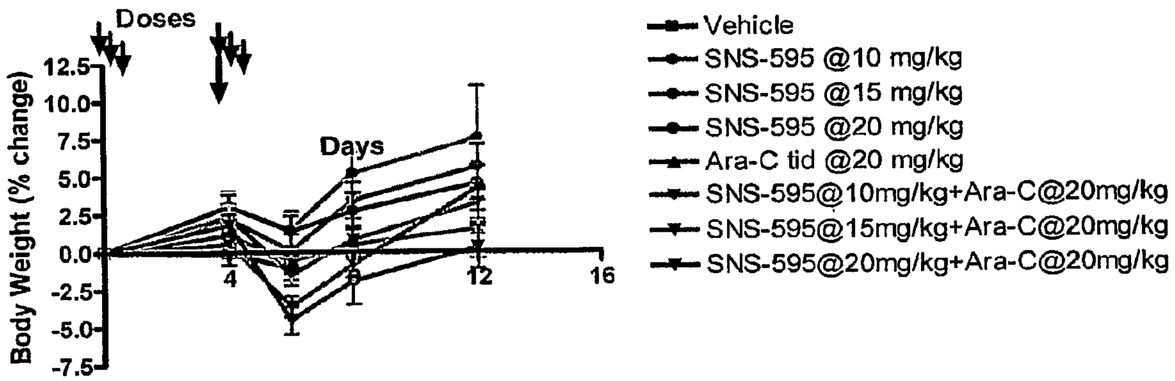
Doses
Days
Body Weight (% change)
12.5
10.0
7.5
5.0
2.5
0.0
-2.5
-5.0
-7.5
4
8
12
16
Vehicle
SNS-595 @10 mg/kg
SNS-595 @15 mg/kg
SNS-595 @20 mg/kg
Ara-C tid @20 mg/kg
SNS-595@10mg/kg+Ara-C@20mg/kg
SNS-595@15mg/kg+Ara-C@20mg/kg
SNS-595@20mg/kg+Ara-C@20mg/kg

*FIG. 32*

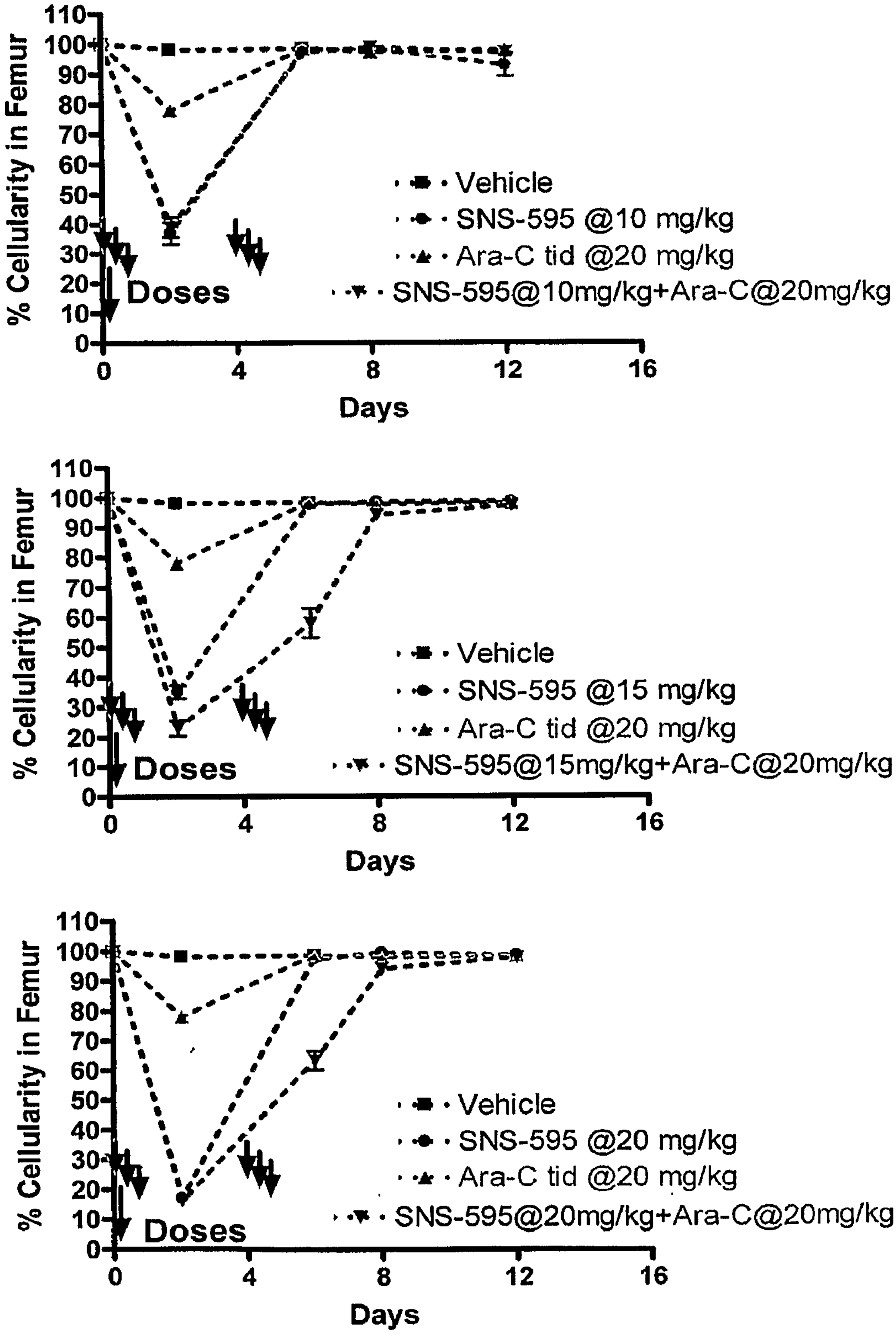
% Cellularity in Femur
110
100
90
80
70
60
50
40
30
20
10
0
Doses
0
4
8
12
16
Days
Vehicle
SNS-595 @10 mg/kg
Ara-C tid @20 mg/kg
SNS-595@10mg/kg+Ara-C@20mg/kg
% Cellularity in Femur
Doses
Days
Vehicle
SNS-595 @15 mg/kg
Ara-C tid @20 mg/kg
SNS-595@15mg/kg+Ara-C@20mg/kg
% Cellularity in Femur
Doses
Days
Vehicle
SNS-595 @20 mg/kg
Ara-C tid @20 mg/kg
SNS-595@20mg/kg+Ara-C@20mg/kg

FIG. 33

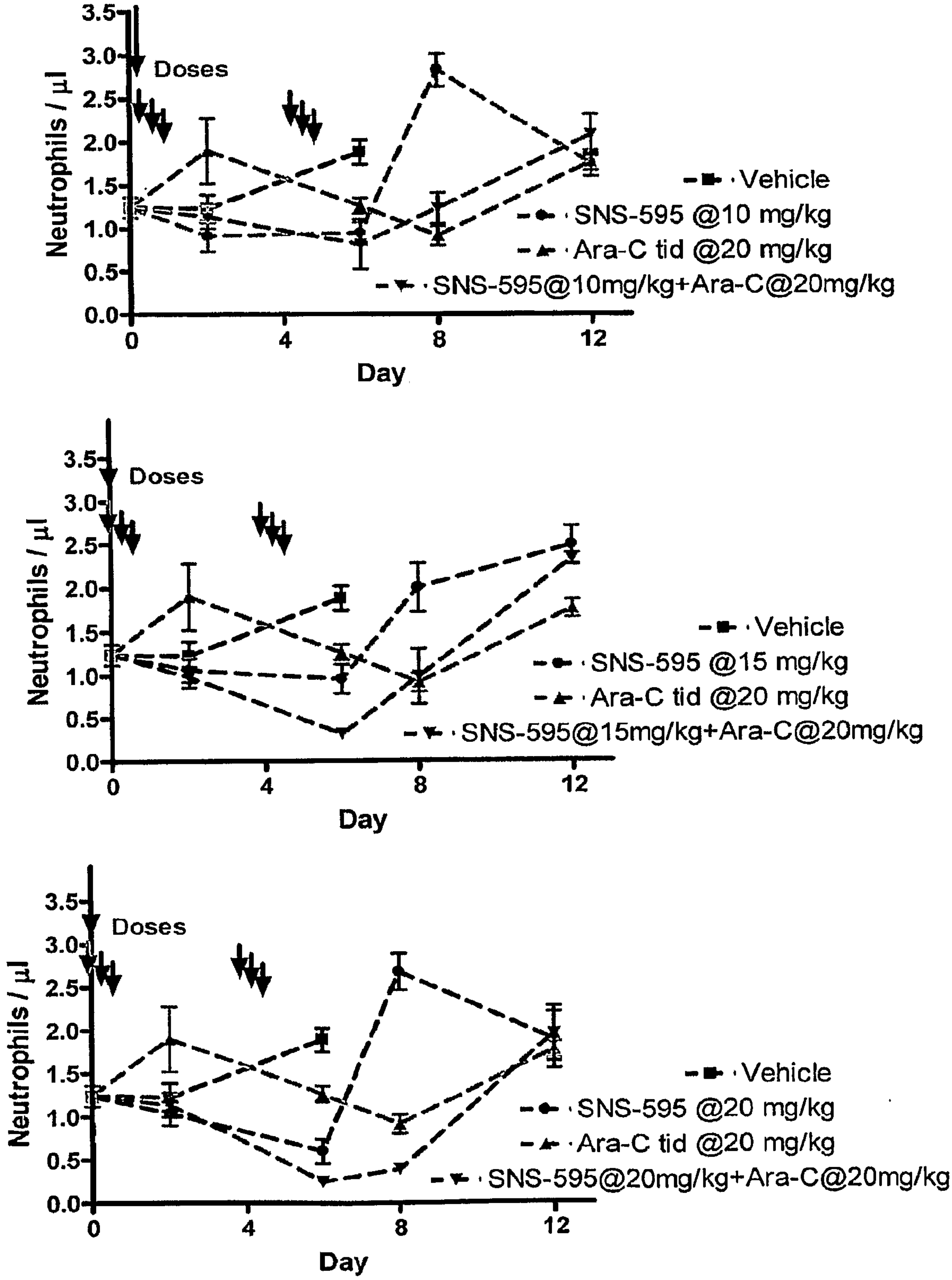

Doses
Neutrophils / µl
Day
Vehicle
SNS-595 @10 mg/kg
Ara-C tid @20 mg/kg
SNS-595@10mg/kg+Ara-C@20mg/kg
Doses
Neutrophils / µl
Day
Vehicle
SNS-595 @15 mg/kg
Ara-C tid @20 mg/kg
SNS-595@15mg/kg+Ara-C@20mg/kg
Doses
Neutrophils / µl
Day
Vehicle
SNS-595 @20 mg/kg
Ara-C tid @20 mg/kg
SNS-595@20mg/kg+Ara-C@20mg/kg

*FIG. 34*

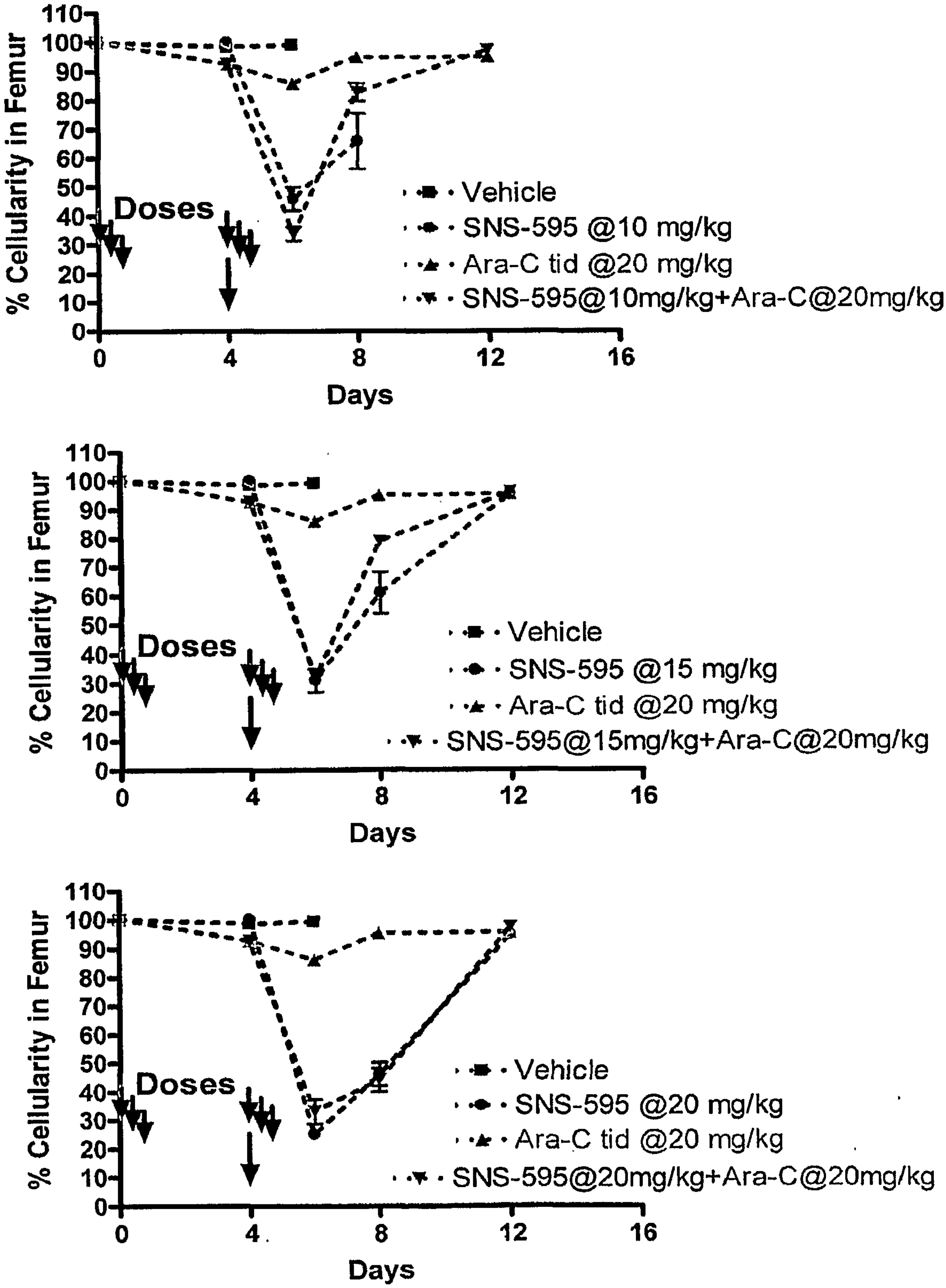
% Cellularity in Femur
Days
Doses
Vehicle
SNS-595 @10 mg/kg
Ara-C tid @20 mg/kg
SNS-595@10mg/kg+Ara-C@20mg/kg
% Cellularity in Femur
Days
Doses
Vehicle
SNS-595 @15 mg/kg
Ara-C tid @20 mg/kg
SNS-595@15mg/kg+Ara-C@20mg/kg
% Cellularity in Femur
Days
Doses
Vehicle
SNS-595 @20 mg/kg
Ara-C tid @20 mg/kg
SNS-595@20mg/kg+Ara-C@20mg/kg

FIG. 35

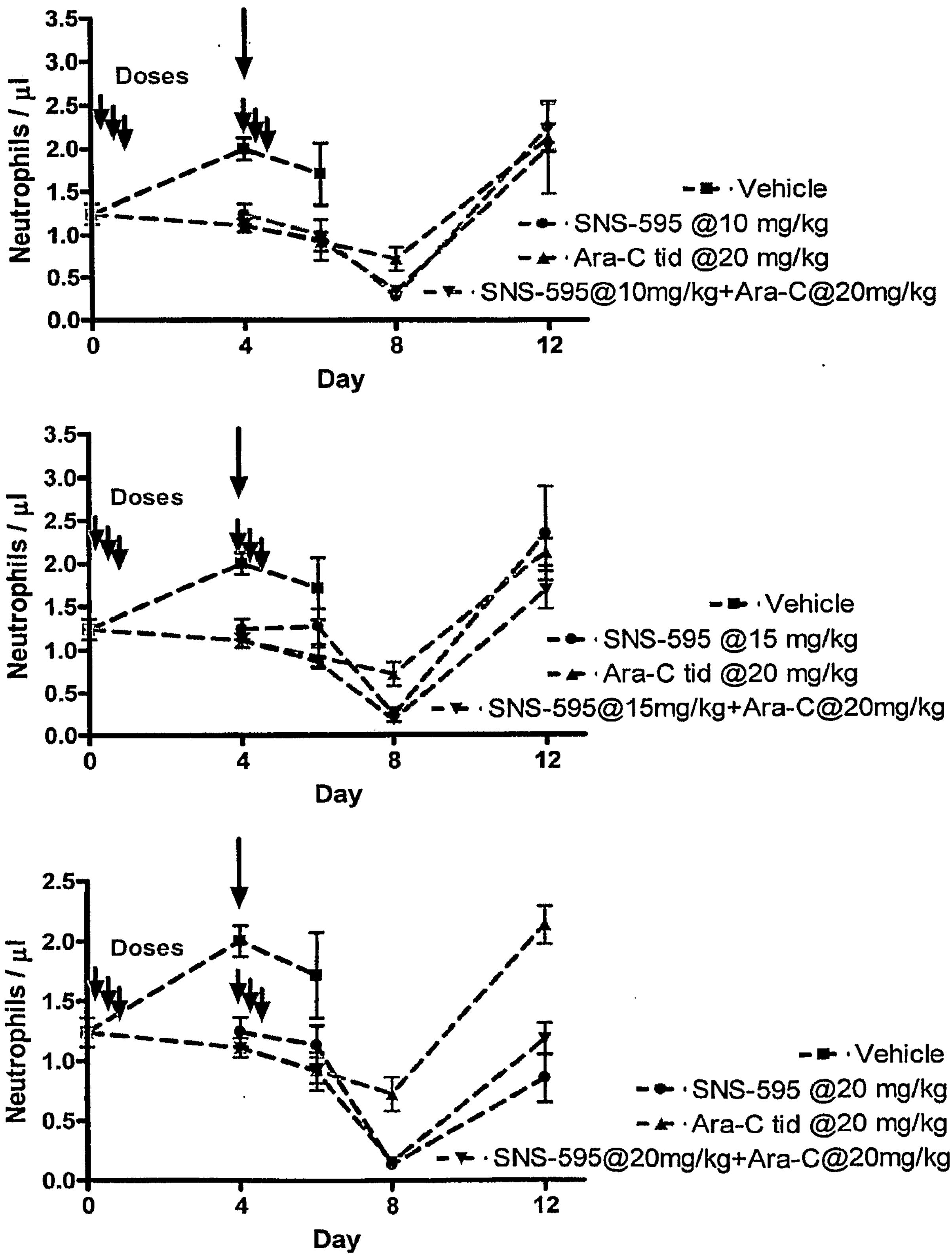

Neutrophils / µl
Doses
Day
Vehicle
SNS-595 @10 mg/kg
Ara-C tid @20 mg/kg
SNS-595@10mg/kg+Ara-C@20mg/kg
SNS-595 @15 mg/kg
SNS-595@15mg/kg+Ara-C@20mg/kg
SNS-595 @20 mg/kg
SNS-595@20mg/kg+Ara-C@20mg/kg

Europäisches Patentamt
European Patent Office
Office européen des brevets

# EUROPEAN SEARCH REPORT

Application Number
EP 15 18 9348

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | WO 2007/028171 A (SUNESIS PHARMACEUTICALS INC [US]; ADELMAN DANIEL C [US]; SILVERMAN JEF) 8 March 2007 (2007-03-08)<br>* claims 1-8,26, 43,48 *<br>* paragraph [0177] *<br>----- | 1-12 | INV.<br>A61K31/4375<br>A61K31/513<br>A61P35/02 |
| X,P | DATABASE BIOSIS [Online]<br>BIOSCIENCES INFORMATION SERVICE, PHILADELPHIA, PA, US;<br>November 2006 (2006-11),<br>ARBITRARIO JENNIFER ET AL: "SNS-595 a novel S phase active cytotoxic acts synergistically with cytarabine to reduce bone marrow cellularity and circulating neutrophils.",<br>XP002463634,<br>Database accession no. PREV200700259072<br>* abstract *<br>& BLOOD,<br>vol. 108, no. 11, Part 1,<br>November 2006 (2006-11), pages 657A-658A,<br>48TH ANNUAL MEETING OF THE AMERICAN-SOCIETY-OF-HEMATOLOGY; ORLANDO, FL, USA; DECEMBER 09 -12, 2006<br>ISSN: 0006-4971<br>-----<br>-/-- | 1-12 | TECHNICAL FIELDS SEARCHED (IPC)<br>A61K |

The present search report has been drawn up for all claims

| Place of search | Date of completion of the search | Examiner |
|---|---|---|
| Munich | 10 March 2016 | Peris Antoli, Berta |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 15 18 9348

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | Arbitrario Jennifer P ET AL: "SNS-595 A Novel S-Phase Active Cytotoxic Acts Synergistically With Cytarabine To Reduce Bone Marrow Cellularity And Circulating Neutrophils", Blood {ASH Annual Meeting Abstract} 108, 16 November 2006 (2006-11-16), page 1, XP055256297, Retrieved from the Internet: URL:http://www.sunesis.com/data-pdf/595/ASH_2006_BM_poster_final.pdf [retrieved on 2016-03-08] * the whole document * ----- | 1-12 | |
| Y,D | US 2006/063795 A1 (ARKIN MICHELLE [US] ET AL) 23 March 2006 (2006-03-23) * claims 18, 22 * * paragraph [0052] * * example 4 * ----- | 1-12 | |
| Y | LAWRENCE CHRIS E ET AL: "SNS-595, A NOVEL S-PHASE ACTIVE CYTOTOXIC, EXHIBITS POTENT IN VITRO AND IN VIVO ACTIVITIES, AND HAS THE POTENTIAL FOR TREATING ADVANCED HEMATOLOGIC MALIGNANCIES", PROCEEDINGS OF THE ANNUAL MEETING OF THE AMERICAN ASSOCIATION FOR CANCER RESEARCH, NEW YORK, NY, US, vol. 47, April 2006 (2006-04), page 1110, XP001199684, ISSN: 0197-016X * abstract * ----- | 1-12 | TECHNICAL FIELDS SEARCHED (IPC) |

-/--

The present search report has been drawn up for all claims

2

| Place of search | Date of completion of the search | Examiner |
|---|---|---|
| Munich | 10 March 2016 | Peris Antoli, Berta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 15 18 9348

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,P | DATABASE BIOSIS [Online] BIOSCIENCES INFORMATION SERVICE, PHILADELPHIA, PA, US; 15 November 2006 (2006-11-15), LANCET JEFFREY E ET AL: "A phase I dose-escalation study of the novel cell cycle active agent SNS-595 in advanced leukemias.", XP002463635, Database accession no. PREV200700258710 * abstract * & BLOOD, vol. 108, no. 11, Part 1, 16 November 2006 (2006-11-16), page 555A, 48TH ANNUAL MEETING OF THE AMERICAN-SOCIETY-OF-HEMATOLOGY; ORLANDO, FL, USA; DECEMBER 09 -12, 2006 ----- | 1-12 | |
| | | | TECHNICAL FIELDS SEARCHED (IPC) |

The present search report has been drawn up for all claims

2

| Place of search | Date of completion of the search | Examiner |
|---|---|---|
| Munich | 10 March 2016 | Peris Antoli, Berta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 15 18 9348

This annex lists the patent family members relating to the patent documents cited in the above-mentioned European search report. The members are as contained in the European Patent Office EDP file on
The European Patent Office is in no way liable for these particulars which are merely given for the purpose of information.

10-03-2016

| Patent document cited in search report | Publication date | Patent family member(s) | Publication date |
|---|---|---|---|
| WO 2007028171 A | 08-03-2007 | AU 2006287149 A1 | 08-03-2007 |
| | | EP 1931339 A1 | 18-06-2008 |
| | | WO 2007028171 A1 | 08-03-2007 |
| US 2006063795 A1 | 23-03-2006 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 83523906 P
- US 60873760 B
- US 5817669 A
- JP HEI10173986 B, Chikugi
- US 20050203120 A
- JP 2005215583 A
- US 20060025437 A
- US 20060063795 A
- US 20060247267 A
- US 5391485 A
- US 5393870 A
- US 5229496 A
- US 4810643 A
- US 4999291 A
- US 5528823 A
- US 5580755 A
- US 5134127 A


### Non-patent literature cited in the description


- **PENICHET, M.L. ; MORRISON, S.L.** *J. Immunol. Methods,* 2001, vol. 248, 91-101
- **EMENS, L.A. et al.** *Curr. Opinion Mol. Ther.,* 2001, vol. 3 (1), 77-84
- Physicians' Desk Reference,. 2002, 1755-1760
- **REMINGTON.** The Science and Practice of Pharmacy. Lippincott, Williams and Wilkins, 2005
- **CHESON et al.** *J Clin Oncol.,* 2003, vol. 21 (24), 4642-49
- **MOSTELLER RD.** Simplified calculation of body surface area. *N Engl J Med.,* 1987, vol. 317 (17), 1098